# EUROPEAN PATENT SPECIFICATION

(11) **EP 3 645 501 B1**
(45) Date of publication and mention of the grant of the patent: **23.08.2023**
(21) Application number: 18732793.7
(22) Date of filing: 25.06.2018
(51) Int. Cl.: C07C 211/54, C07C 211/56, C07C 211/59, C07C 211/61, C07C 321/28, C07D 209/86, C07D 307/91, C01B 33/00, C07D 219/02

(54) **MATERIALS FOR ELECTRONIC DEVICES**
MATERIALIEN FÜR ELEKTRONISCHE VORRICHTUNGEN
MATÉRIAUX POUR DISPOSITIFS ÉLECTRONIQUES

(30) Priority: 28.06.2017 EP 17178441
(43) Date of publication of application: 06.05.2020
(73) Proprietor: Merck Patent GmbH, 64293 Darmstadt (DE)
(72) Inventor: MONTENEGRO, Elvira, 69469 Weinheim (DE); MUJICA-FERNAUD, Teresa, 64283 Darmstadt (DE); MAIER-FLAIG, Florian, 69469 Weinheim (DE); VOGES, Frank, 67098 Bad Duerkheim (DE)
(86) International application number: PCT/EP2018/066926
(87) International publication number: WO 2019/002190

(56) References cited:
- WO-A1-2017/012687
- WO-A1-2017/012687
- WO-A1-2017/016632
- WO-A1-2017/016632
- WO-A1-2017/102063
- WO-A1-2017/102063
- US-A1- 2012 126 179

## Description

The present application relates to a spirobifluorene derivative of a formula (IA) or (IB) defined hereinafter which is suitable for use in electronic devices, especially organic electroluminescent devices (OLEDs).

Electronic devices in the context of this application are understood to mean what are called organic electronic devices, which contain organic semiconductor materials as functional materials. More particularly, these are understood to mean OLEDs.

The construction of OLEDs in which organic compounds are used as functional materials is common knowledge in the prior art. In general, the term OLEDs is understood to mean electronic devices which have one or more layers comprising organic compounds and emit light on application of electrical voltage.

In electronic devices, especially OLEDs, there is great interest in improving the performance data, especially lifetime, efficiency and operating voltage. In these aspects, it has not yet been possible to find any entirely satisfactory solution. Furthermore, for use in electronic devices, there is interest in finding functional materials which have excellent material properties, in particular a low sublimation temperature, because this facilitates the preparation of the devices by vapour deposition techniques.

A great influence on the performance data of electronic devices is possessed by layers having a hole-transporting function, for example hole-injecting layers, hole transport layers, electron blocking layers and also emitting layers. For use in these layers, there is a continuous search for new materials having hole-transporting properties.

In the context of studies of novel materials for use in OLEDs, it is found that spirobifluorene compounds which are substituted with an amino group in the 1-position, and which have in addition at least two further substituent groups on the spirobifluorene, are excellent functional materials for electronic devices. They are particularly useful as materials with a hole transporting function, for example for use in hole transporting layers, electron blocking layers and emitting layers. Spirobifluorene amines for use in electronic devices are disclosed in WO2017/102063, WO2017/16632, WO2017012687 and US2012126179.

When used in electronic devices, in particular in OLEDs, they lead to excellent results in terms of lifetime, operating voltage and quantum efficiency of the devices. The compounds also have one or more properties selected from very good hole-conducting properties, very good electron-blocking properties, high glass transition temperature, high oxidation stability, good solubility, high thermal stability, and low sublimation temperature.

The present application thus provides a compound of formula (IA) or (IB) 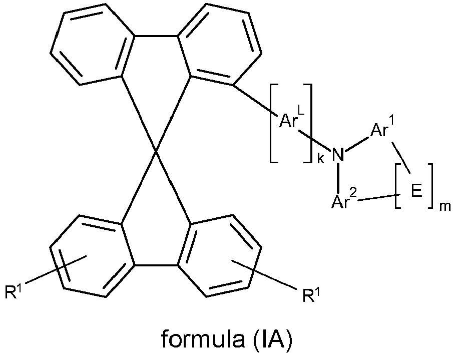 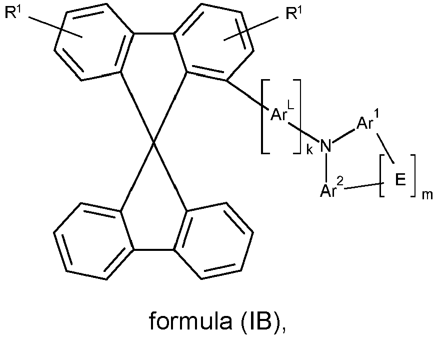 where the following applies to the variables:
Ar^{L} is selected from aromatic ring systems having 6 to 30 aromatic ring atoms, which may be substituted by one or more radicals R³, and
heteroaromatic ring systems having 5 to 30 aromatic ring atoms, which may be substituted by one or more radicals R³;
Ar¹ and Ar² are, identically or differently, selected from aromatic ring systems having 6 to 30 aromatic ring atoms, which may be substituted by one or more radicals R³, and heteroaromatic ring systems having 5 to 30 aromatic ring atoms, which may be substituted by one or more radicals R³;
E is a single bond or is a divalent group selected from C(R³)₂, N(R³), O, and S;
R¹ is, identically or differently on each occurrence, selected from straight-chain alkyl, alkoxy or thioalkyl groups having 1 to 20 C atoms, which may optionally be substituted by one or more groups F, and from branched or cyclic alkyl, alkoxy or thioalkyl groups having 3 to 20 C atoms, which may optionally be substituted by one or more groups F;
R³ is, identically or differently at each occurrence, selected from H, D, F, C(=O)R⁴, CN, Si(R⁴)₃, N(R⁴)₂, P(=O)(R⁴)₂, OR⁴, S(=O)R⁴, S(=O)₂R⁴, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R³ may be connected to each other to form a ring; where the said alkyl, alkoxy, alkenyl and alkynyl groups and the said aromatic and heteroaromatic ring systems may in each case be substituted by one or more radicals R⁴, and where one or more CH₂ groups in the said alkyl, alkoxy, alkenyl and alkynyl groups may in each case be replaced by -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO or SO₂;
R⁴ is, identically or differently at each occurrence, selected from H, D, F, C(=O)R⁵, CN, Si(R⁵)₃, N(R⁵)₂, P(=O)(R⁵)₂, OR⁵, S(=O)R⁵, S(=O)₂R⁵, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R⁴ may be connected to each other to form a ring; where the said alkyl, alkoxy, alkenyl and alkynyl groups and the said aromatic and heteroaromatic ring systems may in each case be substituted by one or more radicals R⁵, and where one or more CH₂ groups in the said alkyl, alkoxy, alkenyl and alkynyl groups may in each case be replaced by -R⁵C=CR⁵-, -C=C-, Si(R⁵)₂, C=O, C=NR⁵, -C(=O)O-, -C(=O)NR⁵-, NR⁵, P(=O)(R⁵), -O-, -S-, SO or SO₂;
R⁵ is selected, identically or differently at each occurrence, from H, D, F, CN, alkyl groups having 1 to 20 C atoms, aromatic ring systems having 6 to 40 C atoms, or heteroaromatic ring systems having 5 to 40 aromatic ring atoms; where two or more radicals R⁵ may be connected to each other to form a ring; and where the said alkyl groups, aromatic ring systems and heteroaromatic ring systems may be substituted by F and CN;
k is 0 or 1; where in the case of k=0, the group Ar^{L} is not present and the nitrogen atom and the spirobifluorene group are directly connected;
m is 0 or 1, where in the case of m=0, the group E is not present and the groups Ar¹ and Ar² are not connected;

The following definitions apply to the chemical groups used as general definitions. They only apply insofar as no more specific definitions are given.

An aryl group in the sense of this invention contains 6 to 40 aromatic ring atoms, of which none is a heteroatom. An aryl group here is taken to mean either a simple aromatic ring, for example benzene, or a condensed aromatic polycycle, for example naphthalene, phenanthrene, or anthracene. A condensed aromatic polycycle in the sense of the present application consists of two or more simple aromatic rings condensed with one another.

A heteroaryl group in the sense of this invention contains 5 to 40 aromatic ring atoms, at least one of which is a heteroatom. The heteroatoms are preferably selected from N, O and S. A heteroaryl group here is taken to mean either a simple heteroaromatic ring, such as pyridine, pyrimidine or thiophene, or a condensed heteroaromatic polycycle, such as quinoline or carbazole. A condensed heteroaromatic polycycle in the sense of the present application consists of two or more simple heteroaromatic rings condensed with one another.

An aryl or heteroaryl group, which may in each case be substituted by the above-mentioned radicals and which may be linked to the aromatic or heteroaromatic ring system via any desired positions, is taken to mean, in particular, groups derived from benzene, naphthalene, anthracene, phenanthrene, pyrene, dihydropyrene, chrysene, perylene, fluoranthene, benz-anthracene, benzophenanthrene, tetracene, pentacene, benzopyrene, furan, benzofuran, isobenzofuran, dibenzofuran, thiophene, benzothiophene, isobenzothiophene, dibenzothiophene, pyrrole, indole, isoindole, carbazole, pyridine, quinoline, isoquinoline, acridine, phenanthridine, benzo-5,6-quinoline, benzo-6,7-quinoline, benzo-7,8-quinoline, pheno-thiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzopyrimidine, quinoxaline, pyrazine, phenazine, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole.

An aromatic ring system in the sense of this invention contains 6 to 40 C atoms in the ring system and does not comprise any heteroatoms as aromatic ring atoms. An aromatic ring system in the sense of this application therefore does not comprise any heteroaryl groups. An aromatic ring system in the sense of this invention is intended to be taken to mean a system which does not necessarily contain only aryl groups, but instead in which, in addition, a plurality of aryl groups may be connected by a non-aromatic unit such as one or more optionally substituted C, Si, N, O or S atoms. The non-aromatic unit in such case comprises preferably less than 10% of the atoms other than H, relative to the total number of atoms other than H of the whole aromatic ring system. Thus, for example, systems such as 9,9'-spirobifluorene, 9,9'-diarylfluorene, triarylamine, diaryl ether, and stilbene are also intended to be taken to be aromatic ring systems in the sense of this invention, as are systems in which two or more aryl groups are connected, for example, by a linear or cyclic alkyl, alkenyl or alkynyl group or by a silyl group. Furthermore, systems in which two or more aryl groups are linked to one another via single bonds are also taken to be aromatic ring systems in the sense of this invention, such as, for example, systems such as biphenyl and terphenyl.

Preferably, an aromatic ring system is understood to be a chemical group, in which the aryl groups which constitute the chemical group are conjugated with each other. This means that the aryl groups are connected with each other via single bonds or via connecting units which have a free pi electron pair which can take part in the conjugation. The connecting units are preferably selected from nitrogen atoms, single C=C units, single C=C units, multiple C=C units and/or C=C units which are conjugated with each other, -O-, and -S-.

A heteroaromatic ring system in the sense of this invention contains 5 to 40 aromatic ring atoms, at least one of which is a heteroatom. The heteroatoms are preferably selected from N, O or S. A heteroaromatic ring system is defined as an aromatic ring system above, with the difference that it must obtain at least one heteroatom as one of the aromatic ring atoms. It thereby differs from an aromatic ring system according to the definition of the present application, which cannot comprise any heteroatom as aromatic ring atom.

An aromatic ring system having 6 to 40 aromatic ring atoms or a heteroaromatic ring system having 5 to 40 aromatic ring atoms is in particular a group which is derived from the above mentioned aryl or heteroaryl groups, or from biphenyl, terphenyl, quaterphenyl, fluorene, spirobifluorene, dihydrophenanthrene, dihydropyrene, tetrahydropyrene, indenofluorene, truxene, isotruxene, spirotruxene, spiroisotruxene, and indenocarbazole.

For the purposes of the present invention, a straight-chain alkyl group having 1 to 20 C atoms or a branched or cyclic alkyl group having 3 to 20 C atoms or an alkenyl or alkynyl group having 2 to 20 C atoms, in which, in addition, individual H atoms or CH₂ groups may be substituted by the groups mentioned above under the definition of the radicals, is preferably taken to mean the radicals methyl, ethyl, n-propyl, i-propyl, n-butyl, i-butyl, s-butyl, t-butyl, 2-methylbutyl, n-pentyl, s-pentyl, cyclopentyl, neopentyl, n-hexyl, cyclohexyl, neohexyl, n-heptyl, cycloheptyl, n-octyl, cyclooctyl, 2-ethylhexyl, trifluoromethyl, pentafluoroethyl, 2,2,2-trifluoroethyl, ethenyl, propenyl, butenyl, pentenyl, cyclopentenyl, hexenyl, cyclohexenyl, heptenyl, cycloheptenyl, octenyl, cyclooctenyl, ethynyl, propynyl, butynyl, pentynyl, hexynyl or octynyl.

An alkoxy or thioalkyl group having 1 to 20 C atoms is preferably taken to mean methoxy, trifluoromethoxy, ethoxy, n-propoxy, i-propoxy, n-butoxy, i-butoxy, s-butoxy, t-butoxy, n-pentoxy, s-pentoxy, 2-methylbutoxy, n-hexoxy, cyclohexyloxy, n-heptoxy, cycloheptyloxy, n-octyloxy, cyclooctyloxy, 2-ethylhexyloxy, pentafluoroethoxy, 2,2,2-trifluoroethoxy, methylthio, ethylthio, n-propylthio, i-propylthio, n-butylthio, i-butylthio, s-butylthio, t-butylthio, n-pentylthio, s-pentylthio, n-hexylthio, cyclohexylthio, n-heptylthio, cycloheptylthio, n-octylthio, cyclooctylthio, 2-ethylhexylthio, trifluoromethylthio, pentafluoroethylthio, 2,2,2-trifluoroethylthio, ethenylthio, propenylthio, butenylthio, pentenylthio, cyclopentenylthio, hexenylthio, cyclohexenylthio, heptenylthio, cycloheptenylthio, octenylthio, cyclooctenylthio, ethynylthio, propynylthio, butynylthio, pentynylthio, hexynylthio, heptynylthio or octynylthio.

Preferably, group Ar^{L} is selected from aromatic ring systems having 6 to 30 aromatic ring atoms, which may be substituted by one or more radicals R³.

It is particularly preferred if Ar^{L} is selected from divalent groups derived from benzene, biphenyl, terphenyl, naphthyl, fluorenyl, indenofluorenyl, spirobifluorenyl, dibenzofuranyl, dibenzothiophenyl, and carbazolyl, which may each be substituted by one or more radicals R³.

Preferred groups Ar^{L} conform to the following formulae

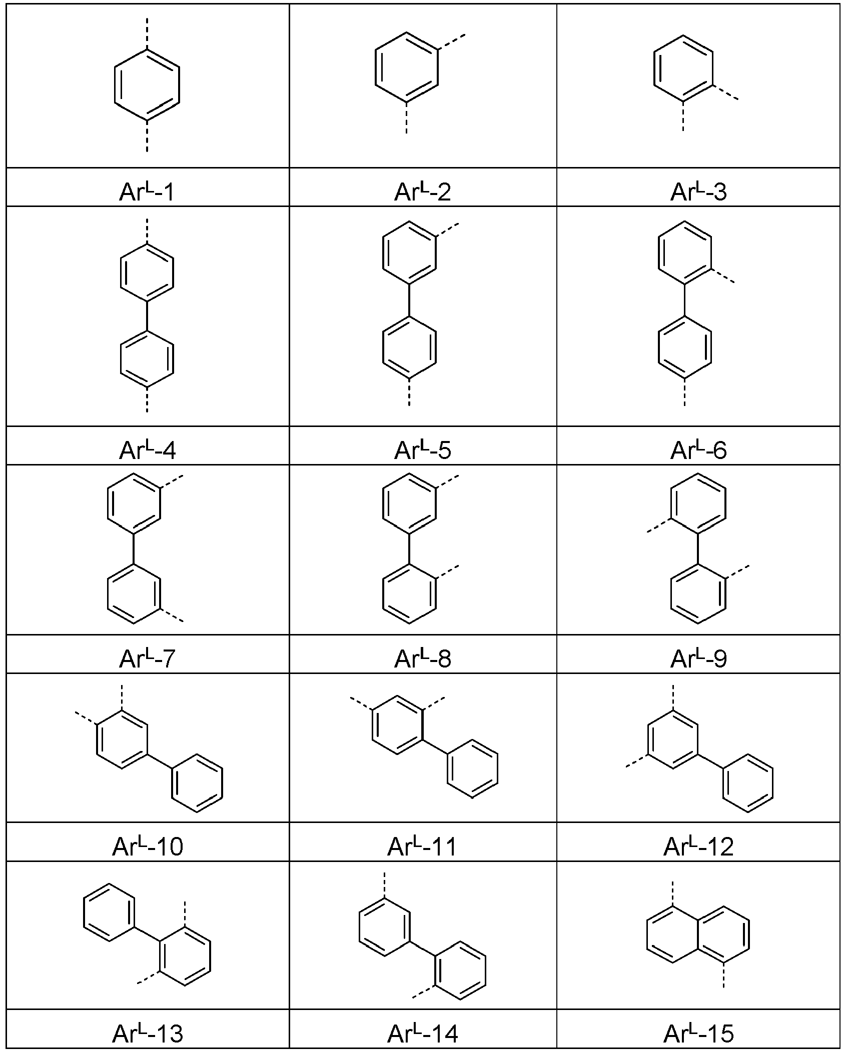

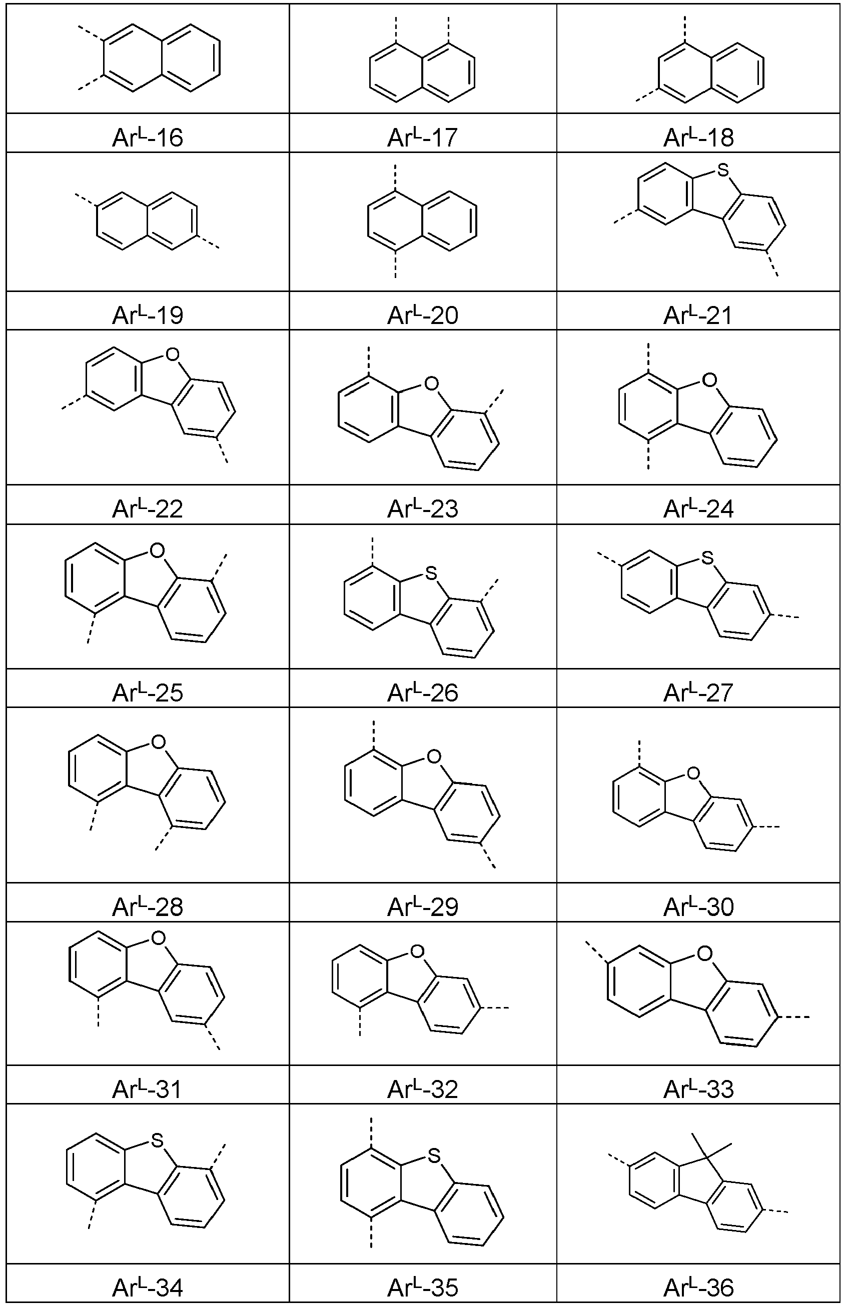

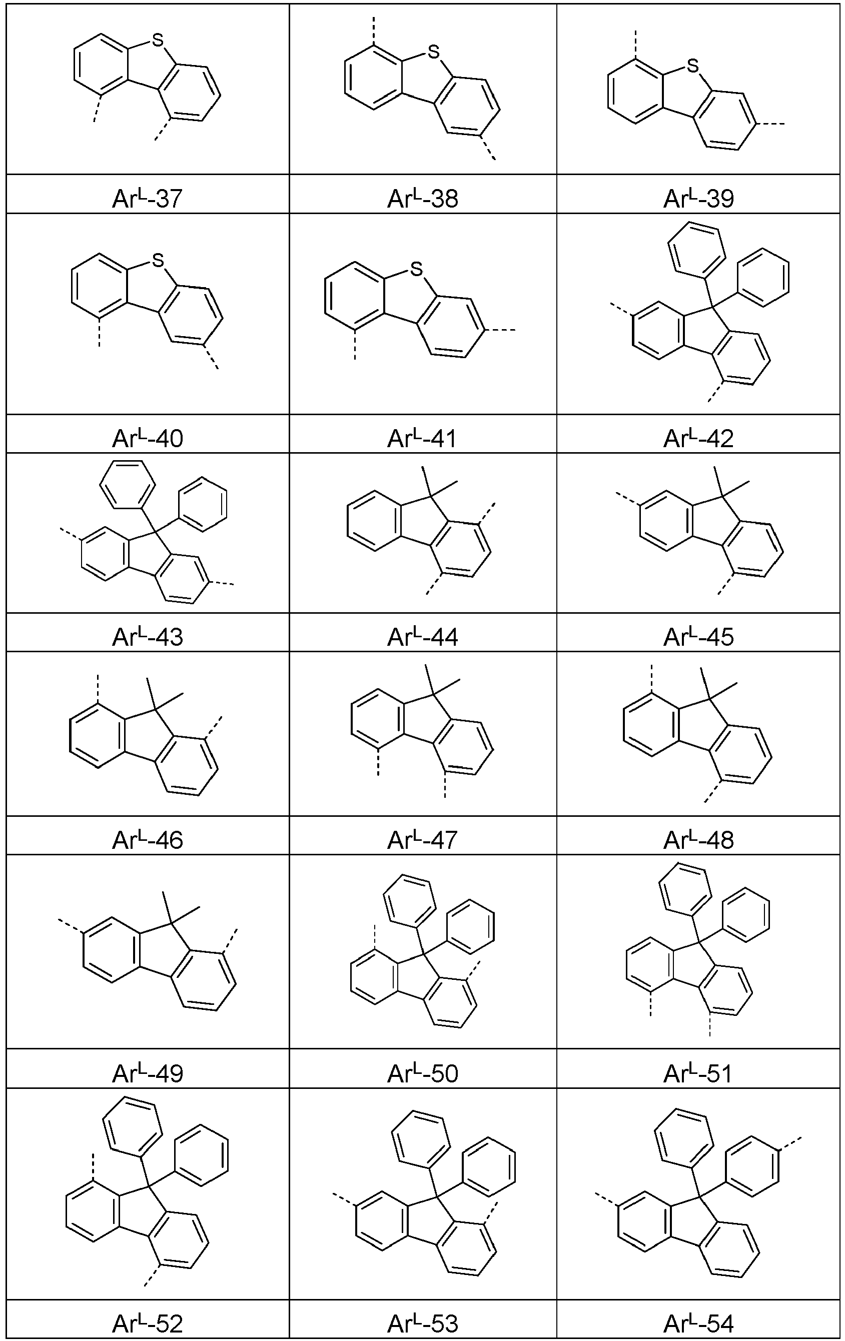

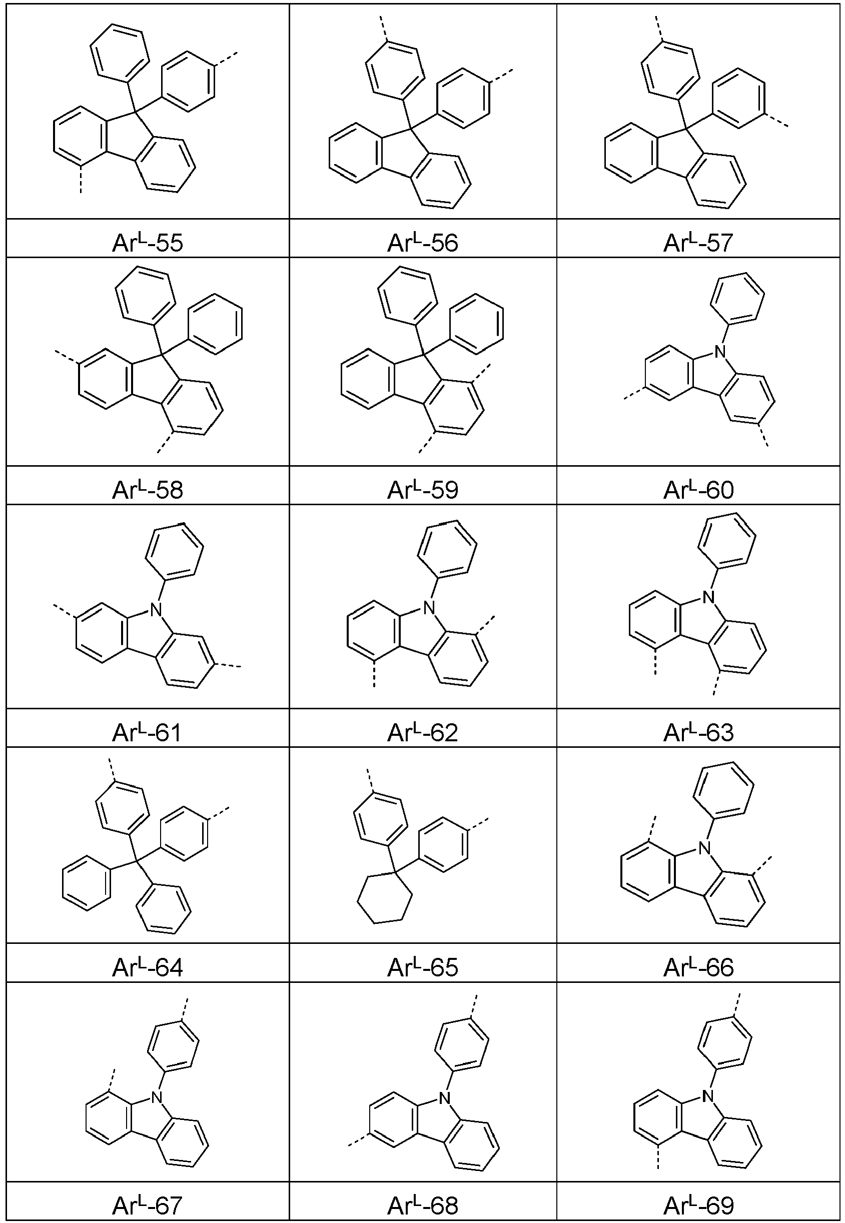

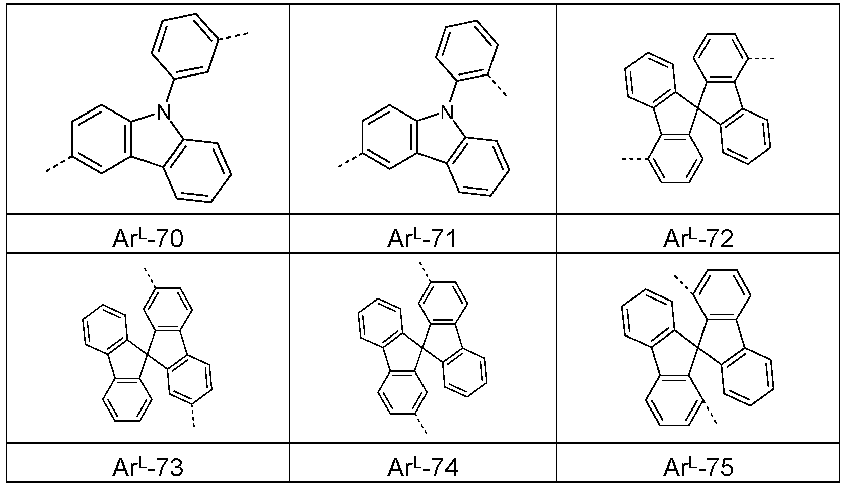

where the dotted lines represent the bonds of the divalent group to the rest of the formula (IA) or (IB).

Particularly preferred among the groups above are the groups according to one of formulae Ar^{L}-1, Ar^{L}-2, Ar^{L}-3, Ar^{L}-9, Ar^{L}-12, Ar^{L}-16, Ar^{L}-17, Ar^{L}-36, Ar^{L}-64, and Ar^{L}-73.

It is preferred that index k is 0, meaning that the group Ar^{L} is not present, so that the spirobifluorene and the nitrogen atom of the amine are directly connected with each other.

Preferably, groups Ar¹ and Ar² are, identically or differently, selected from radicals derived from the following groups, which are each optionally substituted by one or more radicals R³, or from combinations of 2 or 3 radicals derived from the following groups, which are each optionally substituted by one or more radicals R³: phenyl, biphenyl, terphenyl, quaterphenyl, naphthyl, fluorenyl, especially 9,9'-dimethylfluorenyl and 9,9'-diphenylfluorenyl, benzofluorenyl, spirobifluorenyl, indenofluorenyl, dibenzofuranyl, dibenzothiophenyl, carbazolyl, benzofuranyl, benzothiophenyl, indolyl, quinolinyl, pyridyl, pyrimidyl, pyrazinyl, pyridazinyl and triazinyl.

Particularly preferred groups Ar¹ and Ar² are, identically or differently, selected from phenyl, biphenyl, terphenyl, quaterphenyl, naphthyl, fluorenyl, especially 9,9'-dimethylfluorenyl and 9,9'-diphenylfluorenyl, benzofluorenyl, spirobifluorenyl, indenofluorenyl, dibenzofuranyl, dibenzothiophenyl, carbazolyl, benzofuranyl, benzothiophenyl, benzofused dibenzofuranyl, benzofused dibenzothiophenyl, naphthyl-substituted phenyl, fluorenyl-substituted phenyl, spirobifluorenyl-substituted phenyl, dibenzofuranyl-substituted phenyl, dibenzothiophenyl-substituted phenyl, carbazolyl-substituted phenyl, pyridyl-substituted phenyl, pyrimidyl-substituted phenyl, and triazinyl-substituted phenyl, each of which may optionally be substituted by one or more radicals R³.

Preferably, Ar¹ and Ar² are selected differently.

Preferred groups Ar¹ and Ar² are, identically or differently, selected from groups of the following formulae

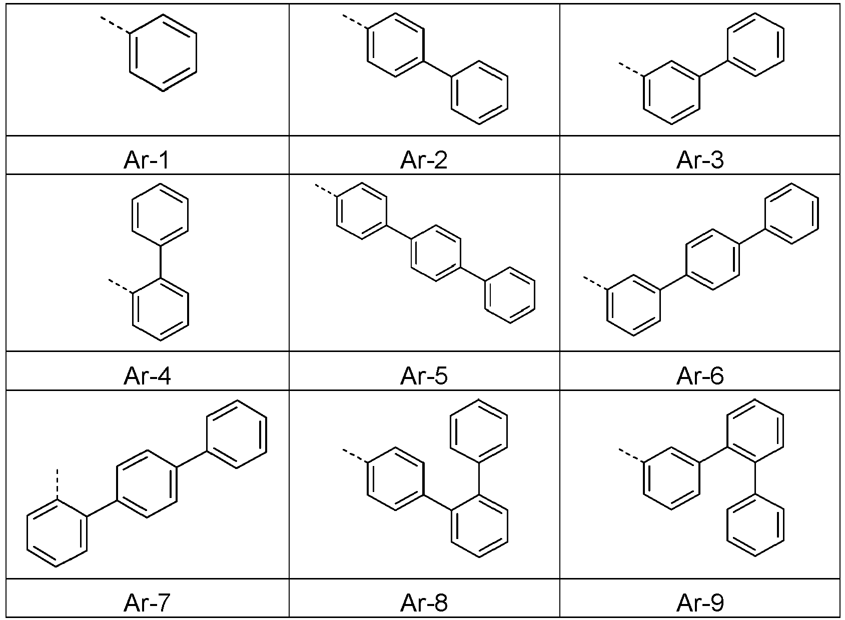

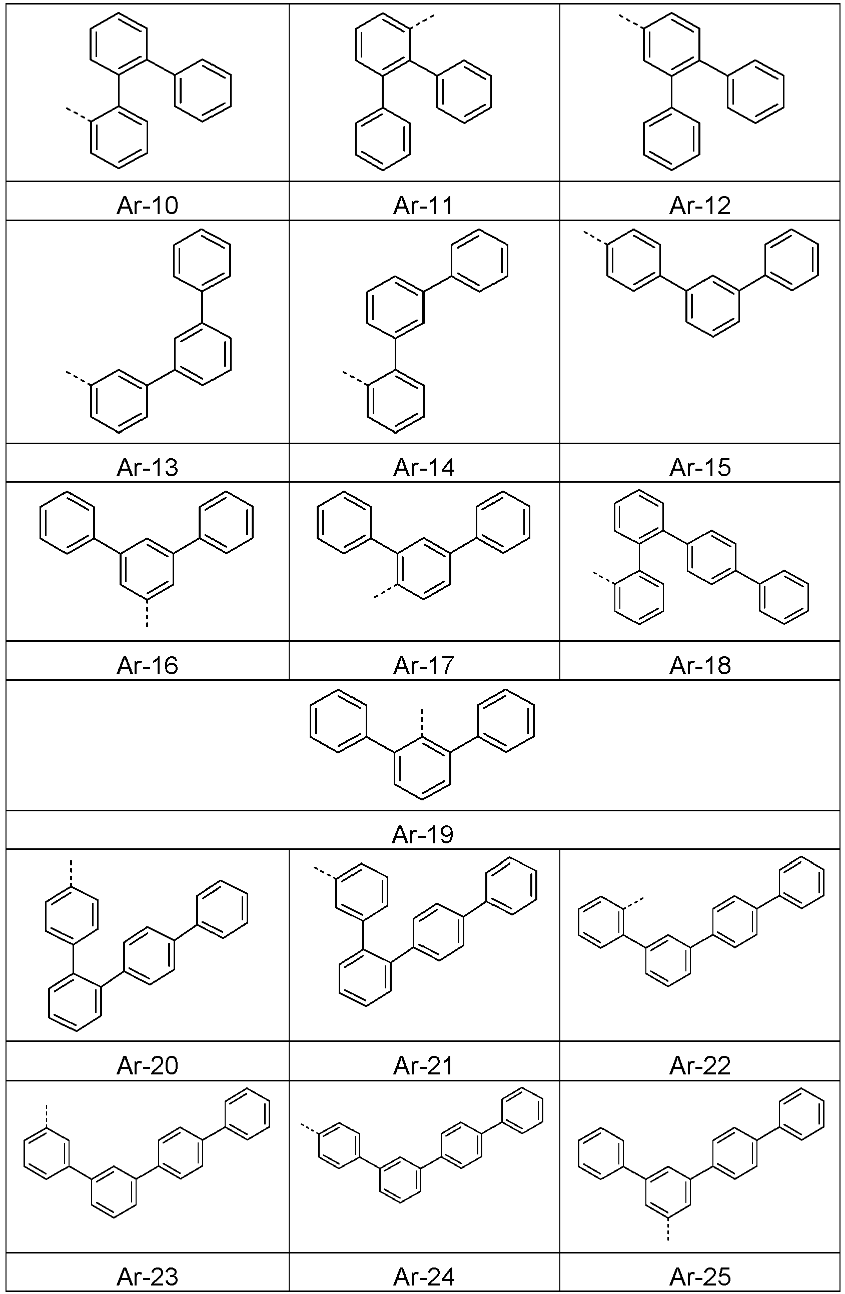

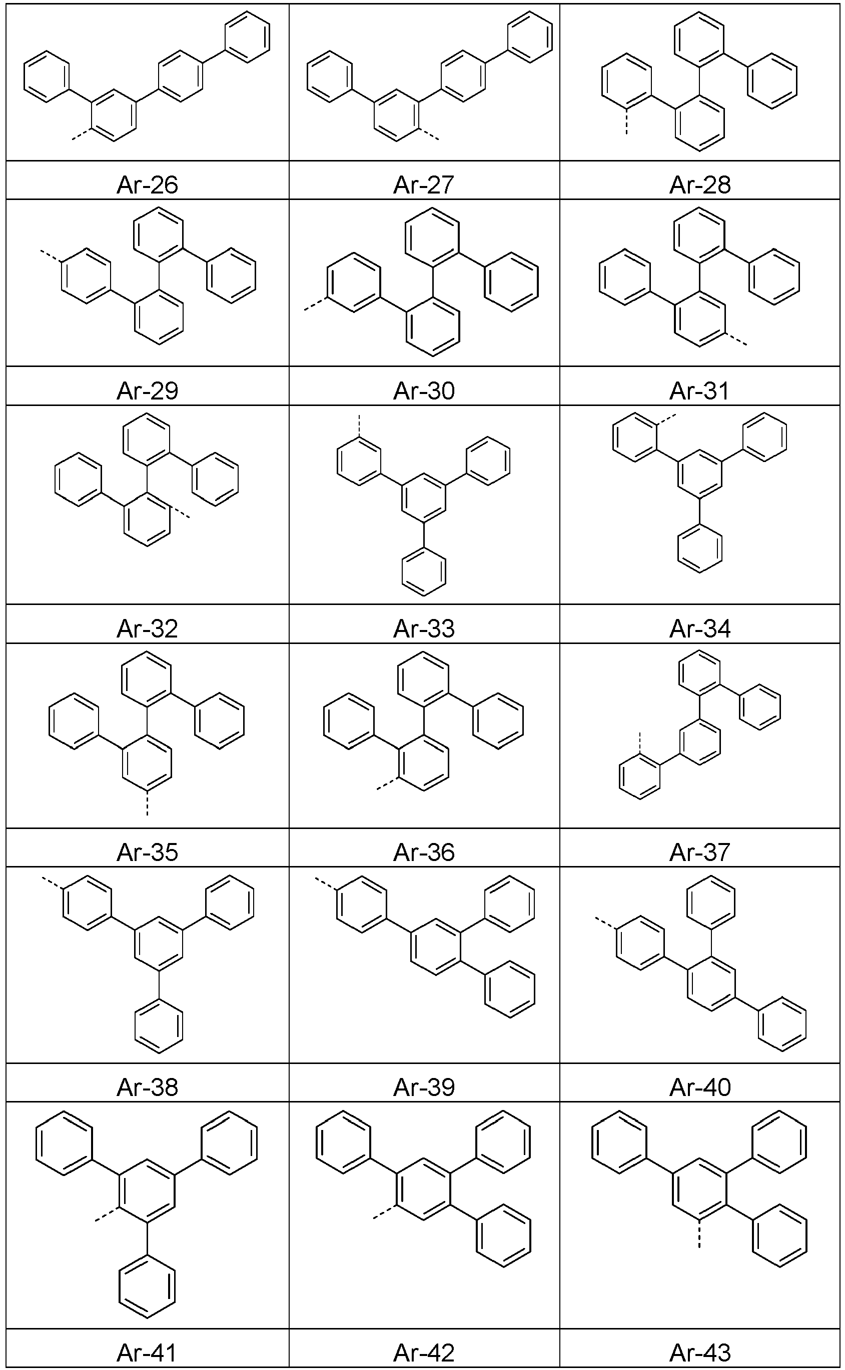

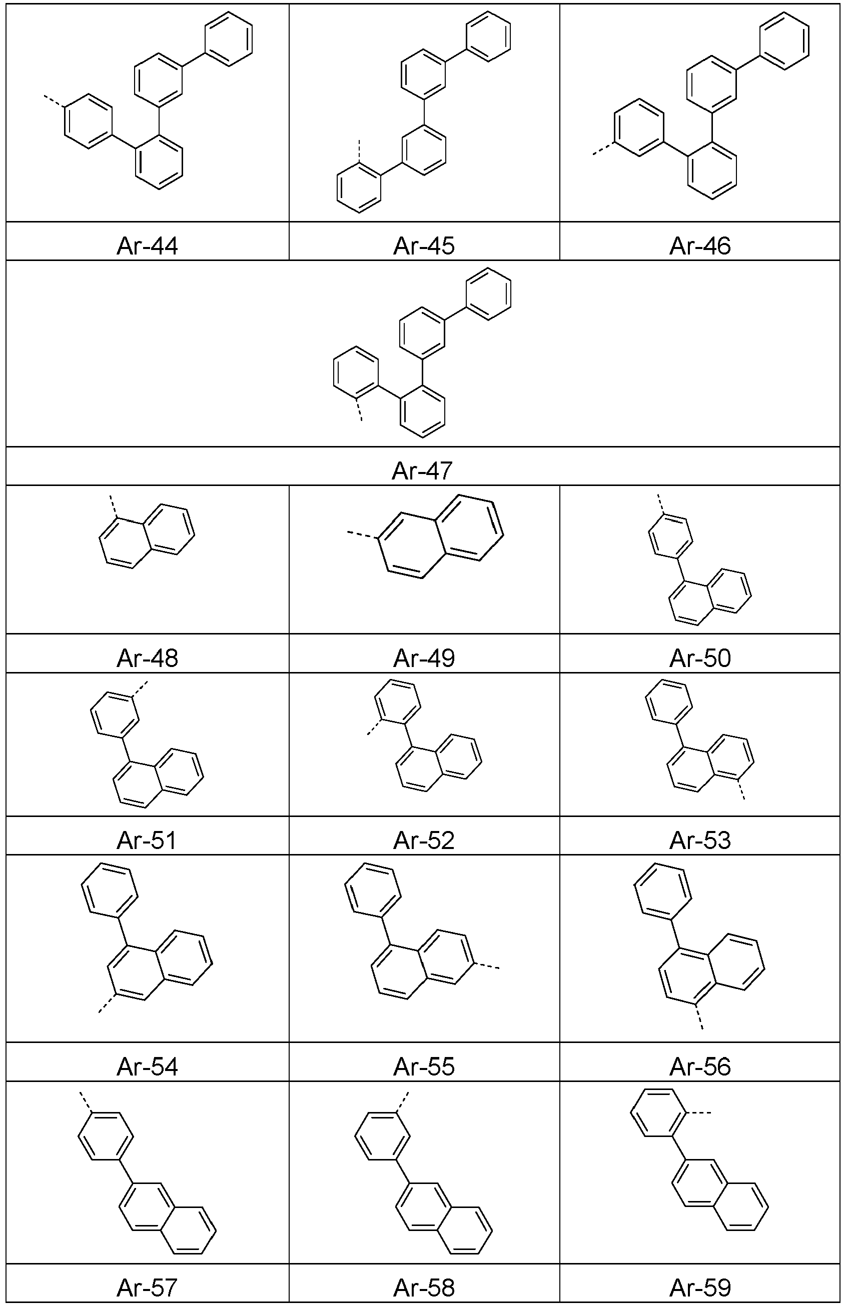

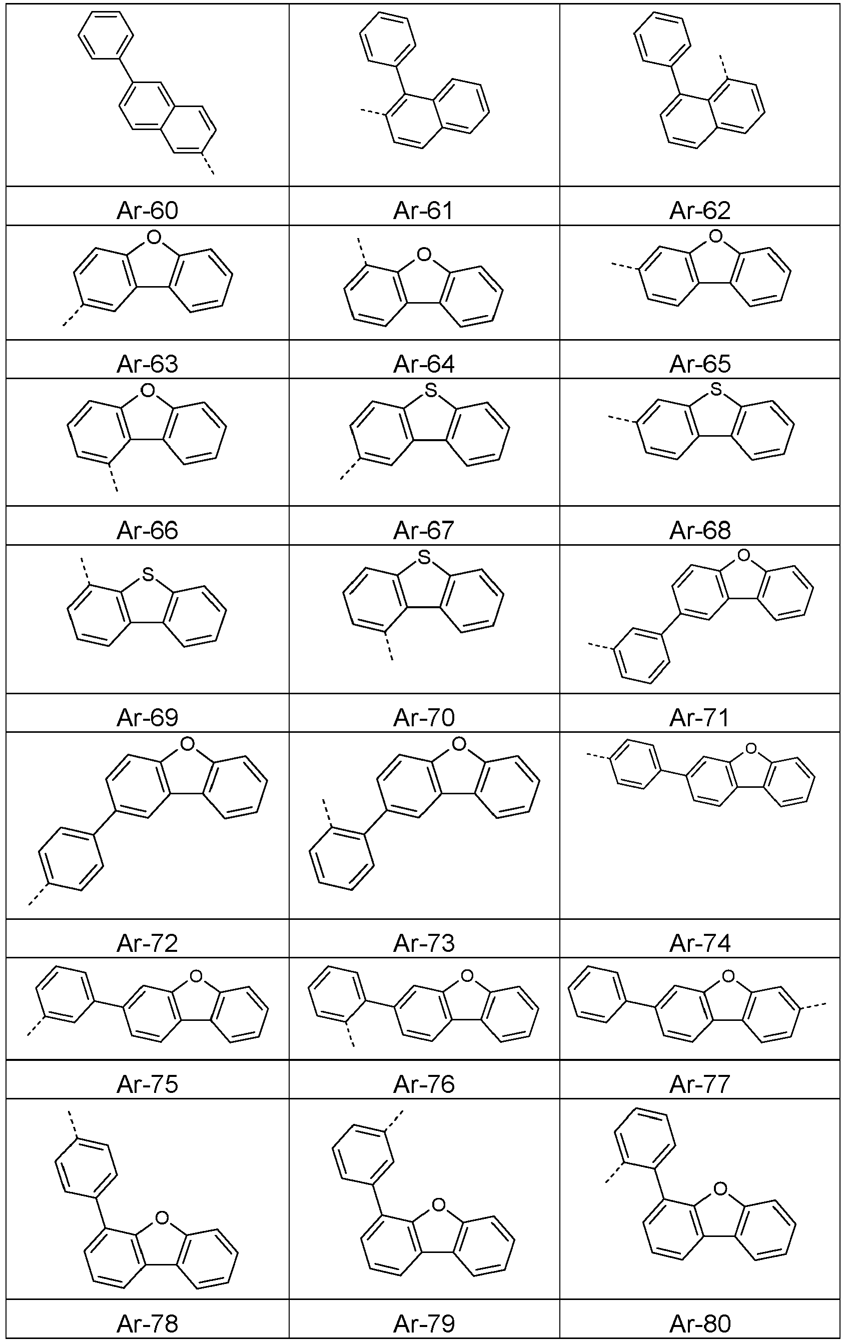

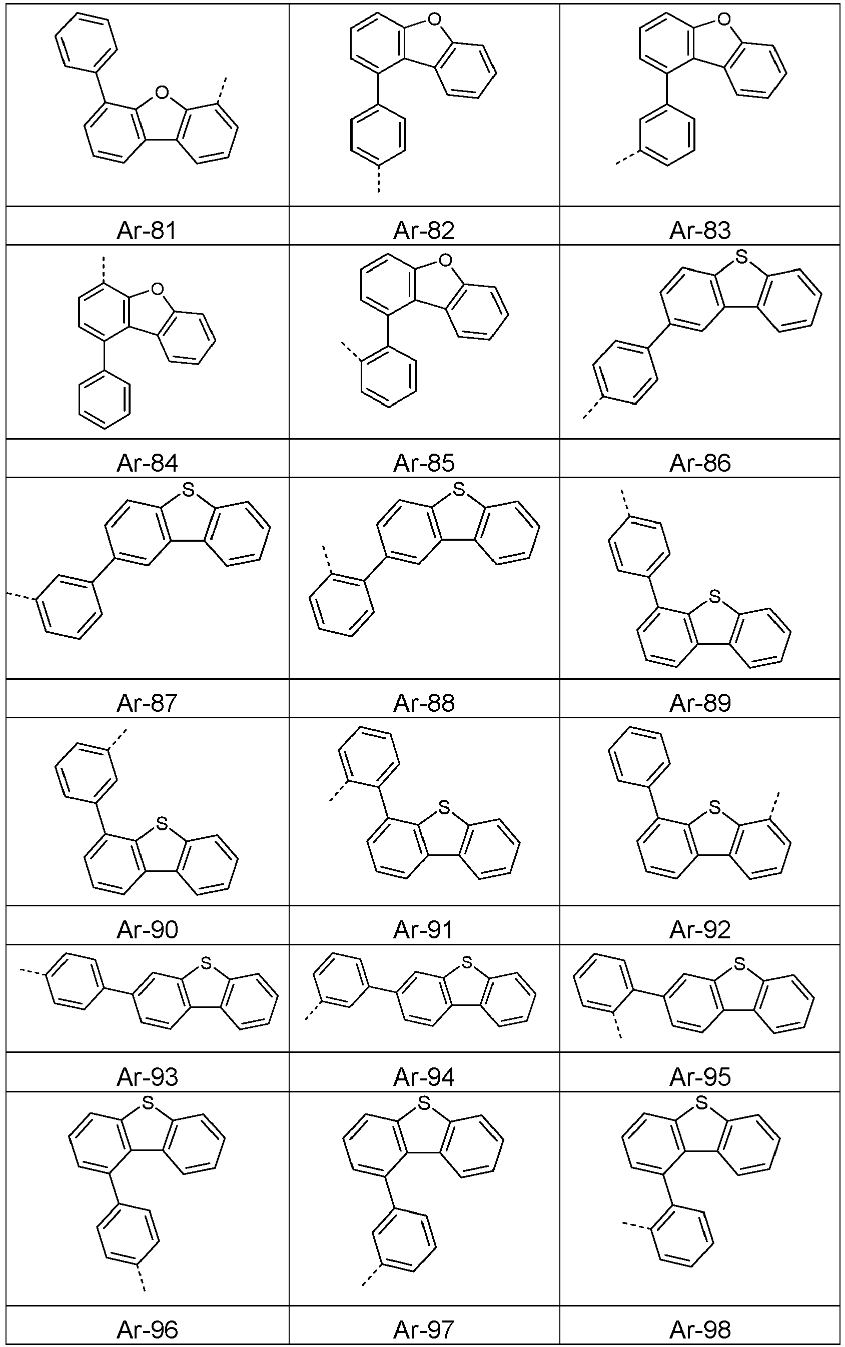

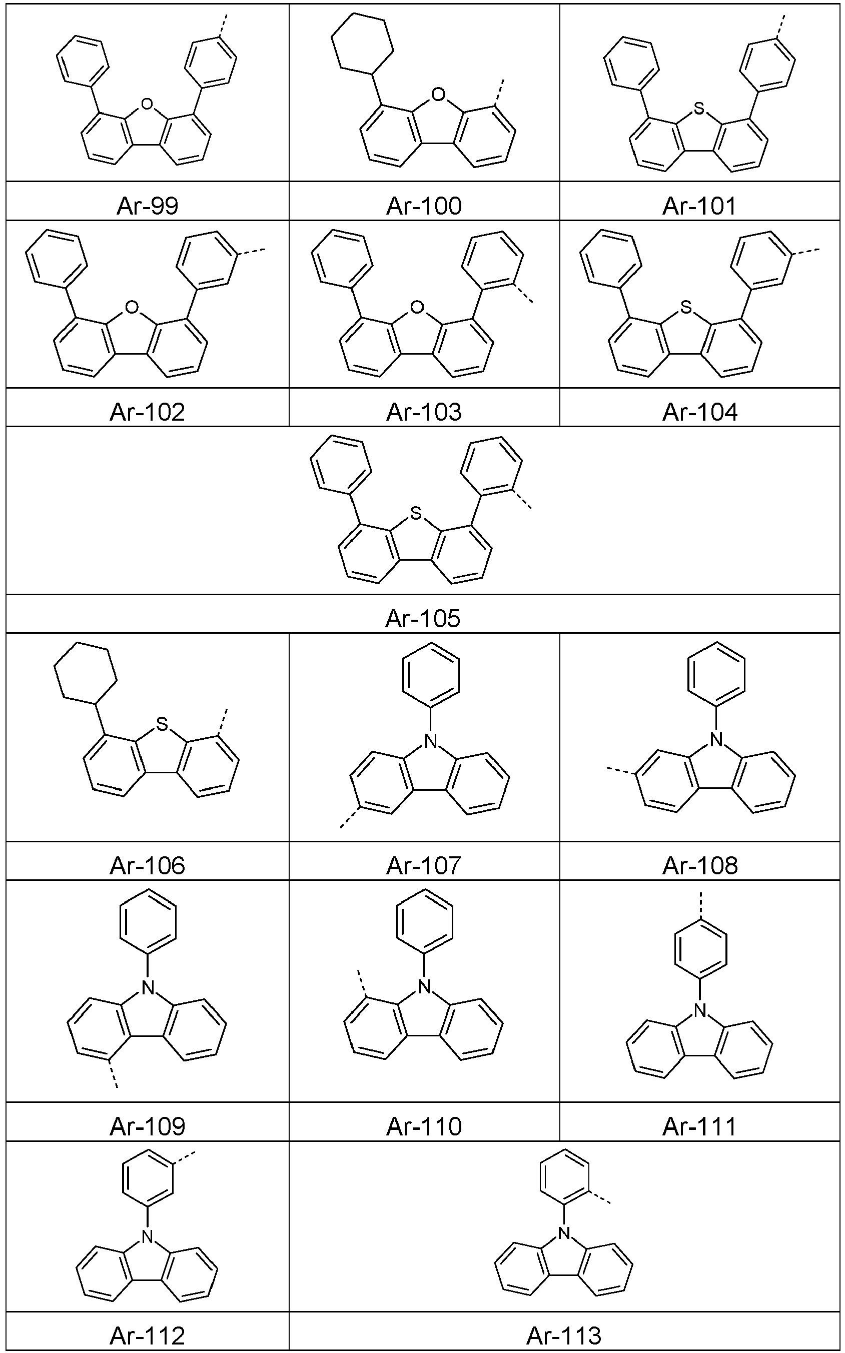

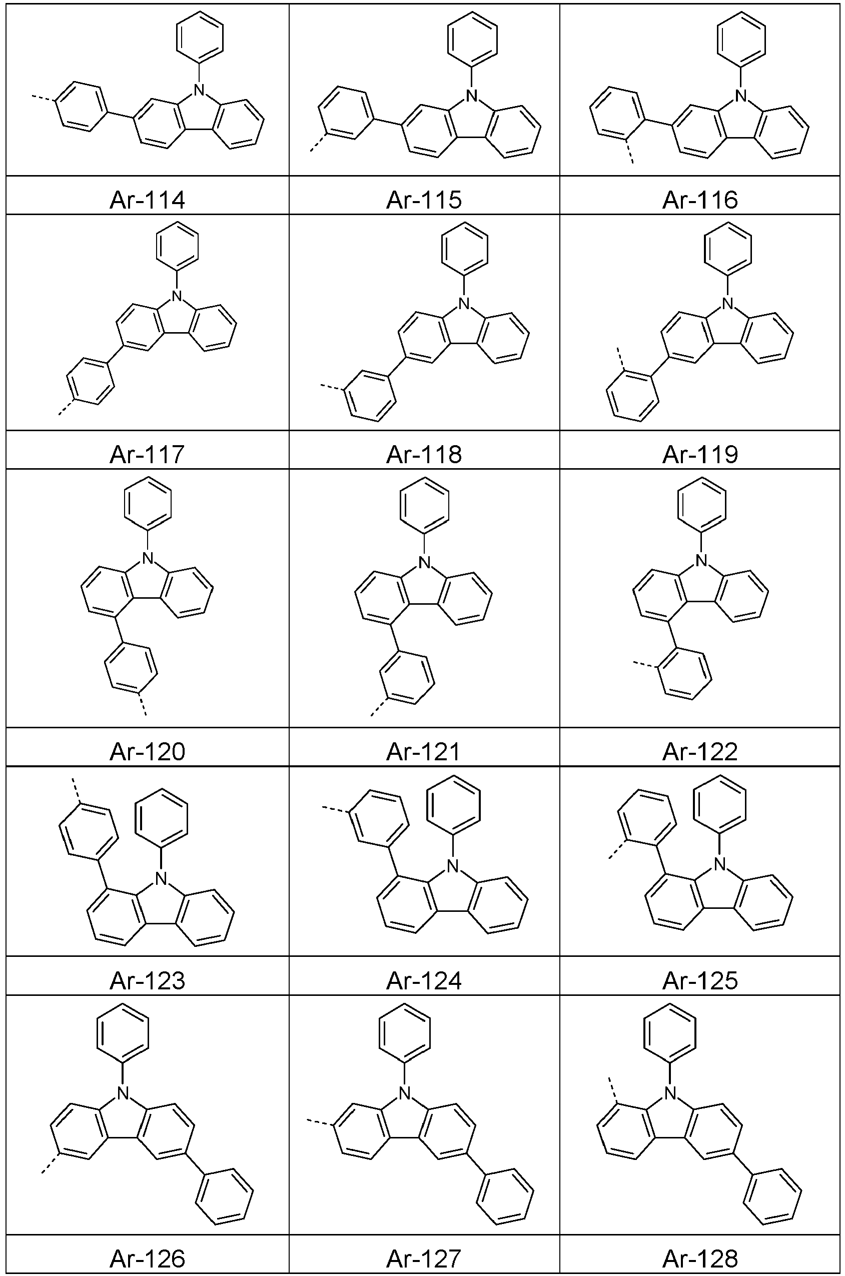

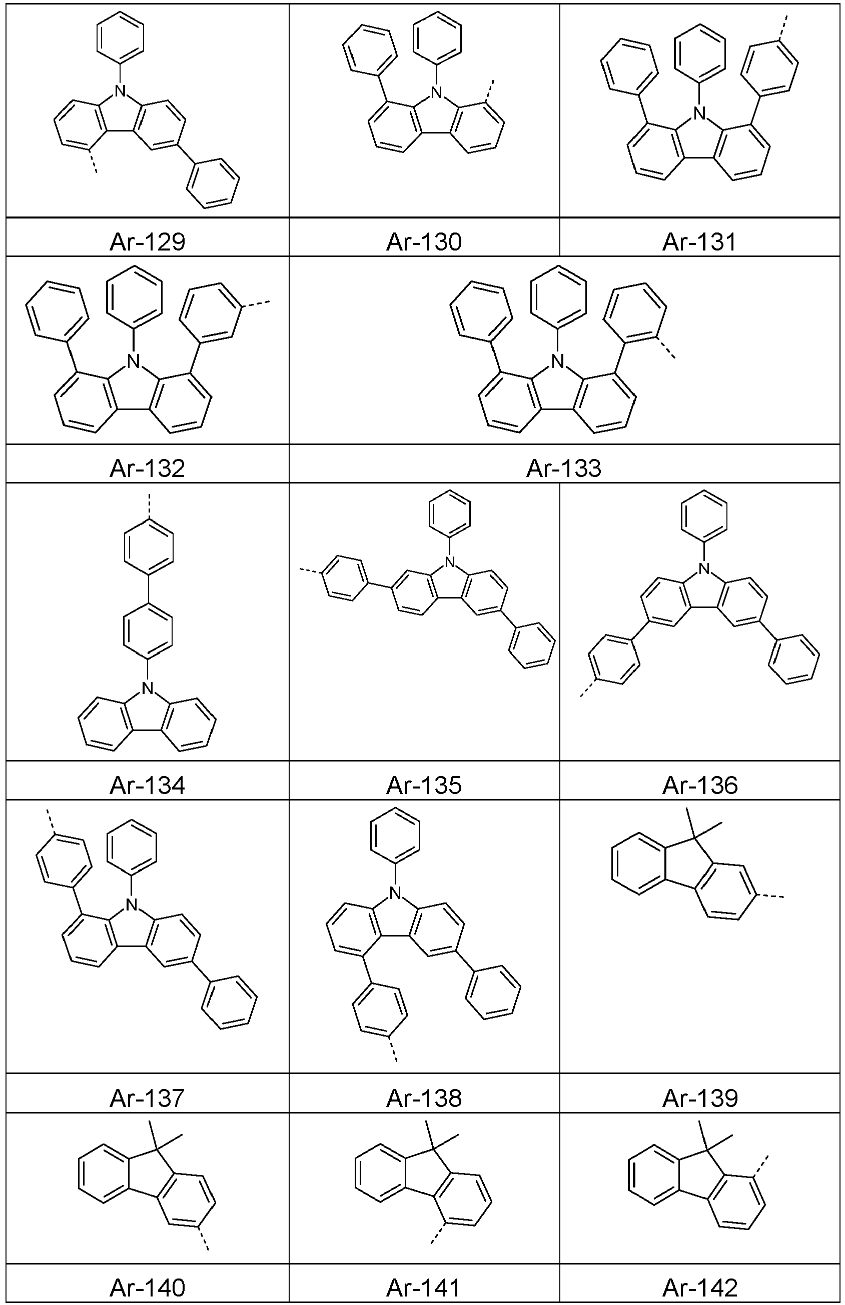

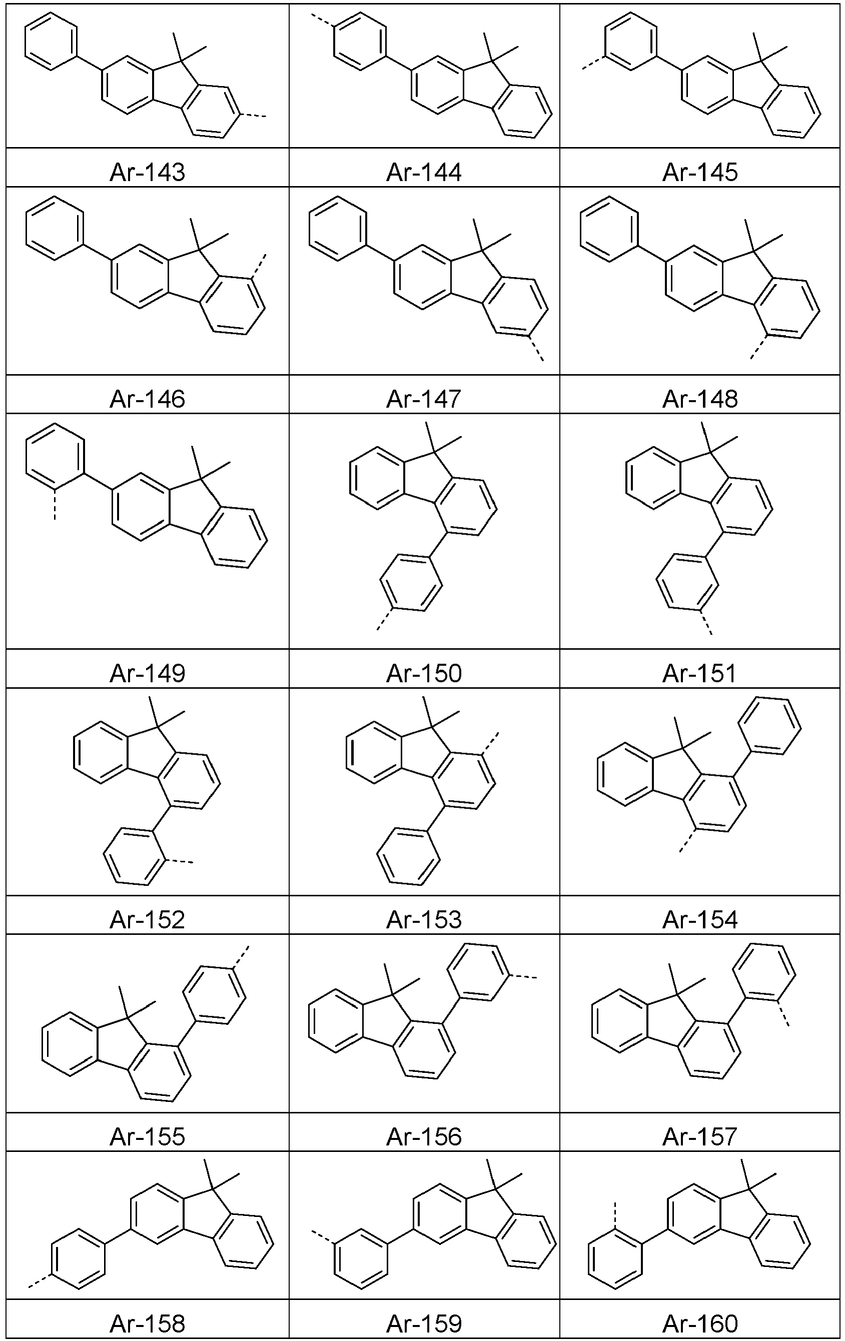

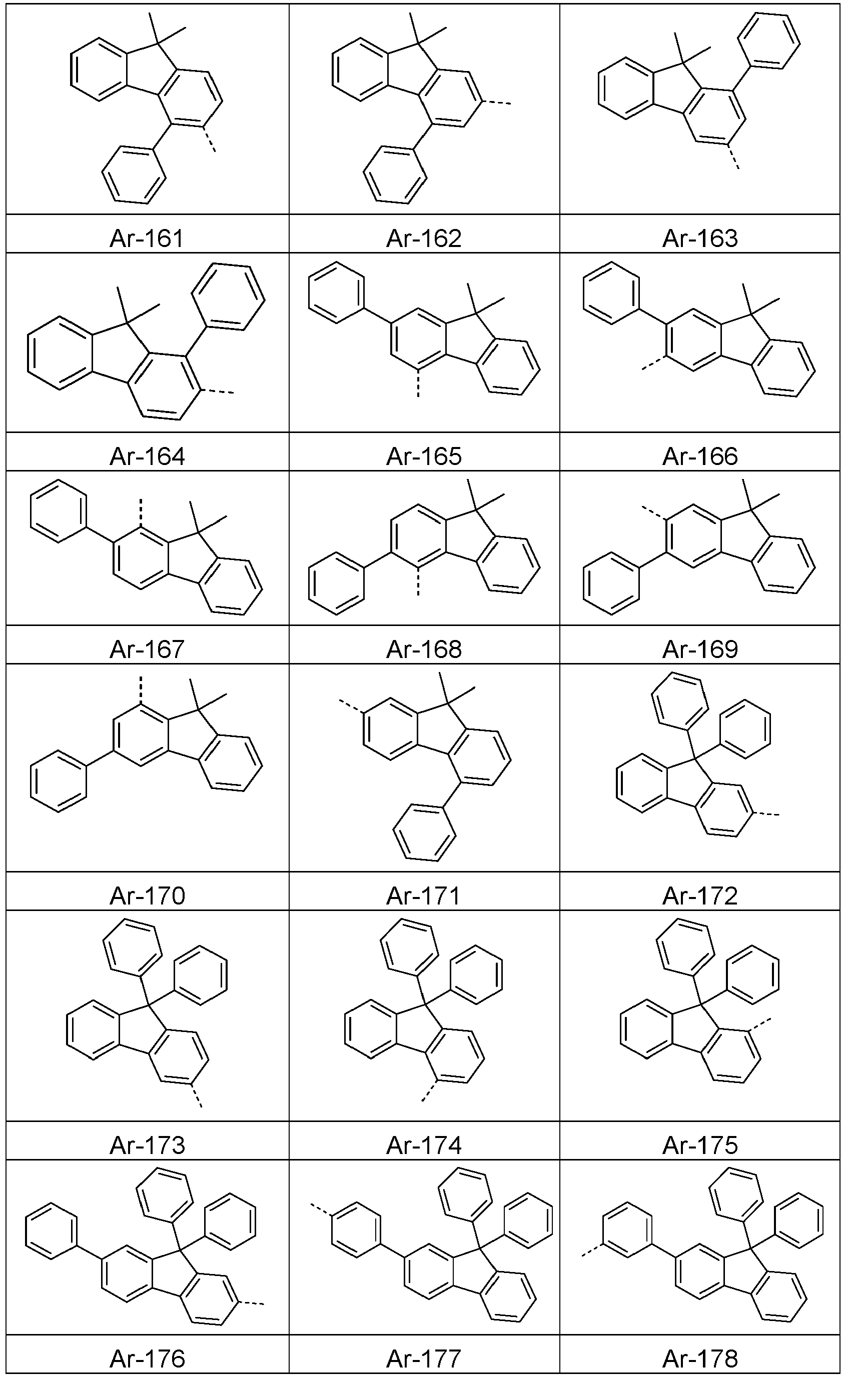

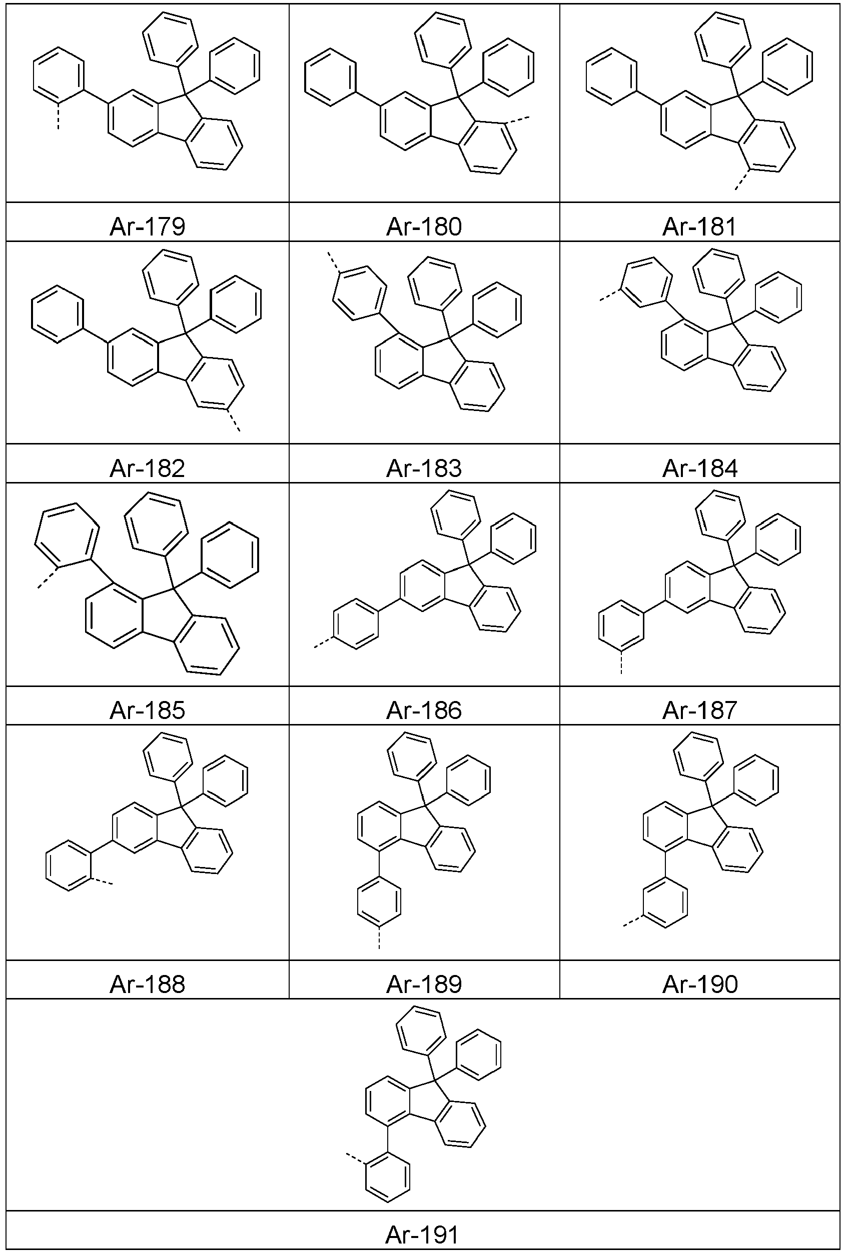

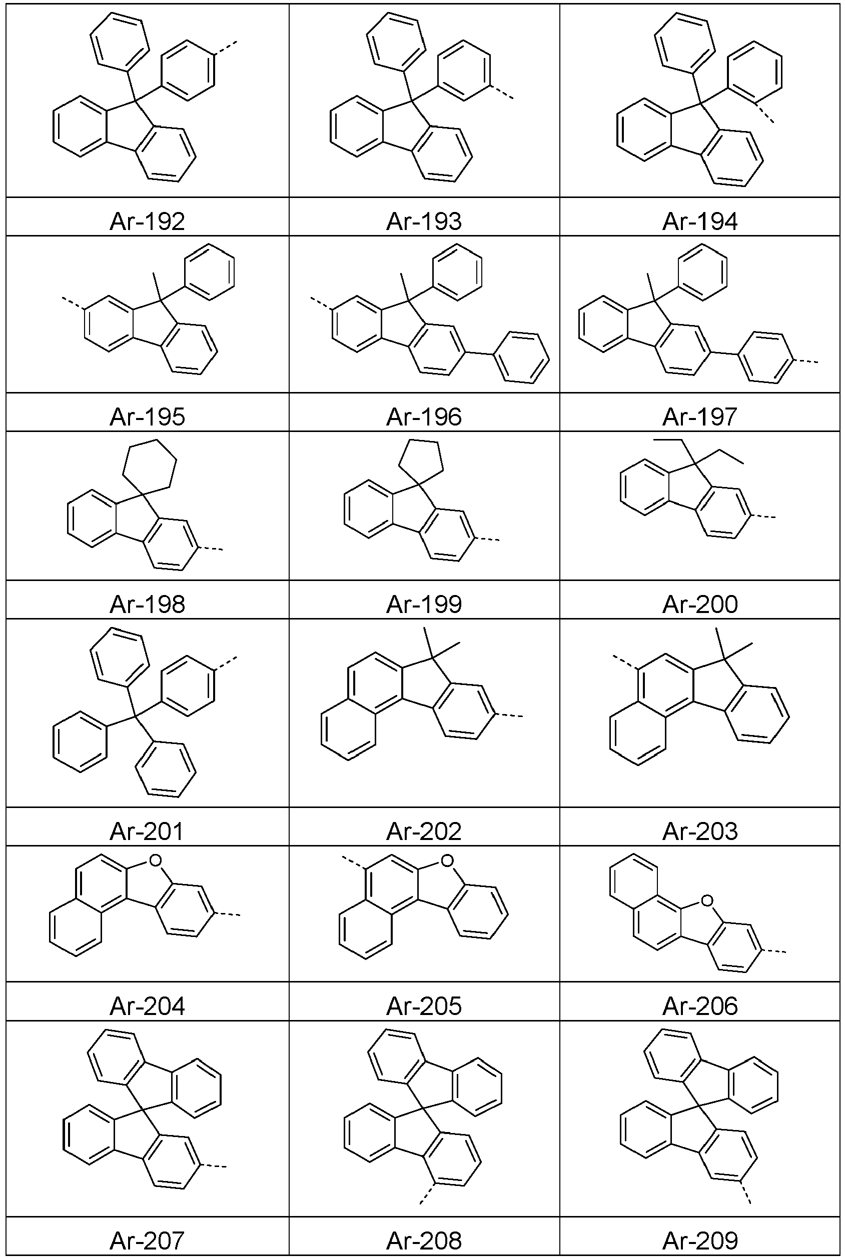

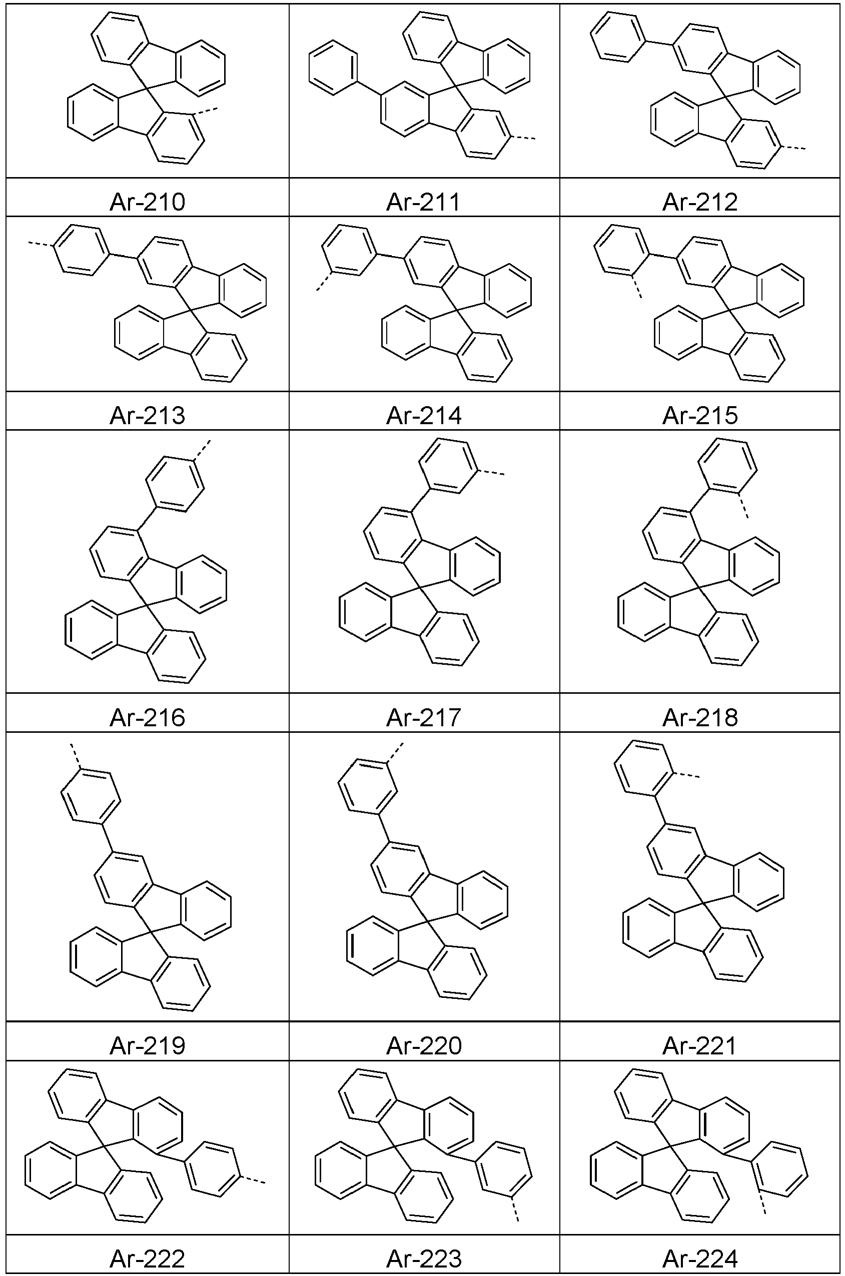

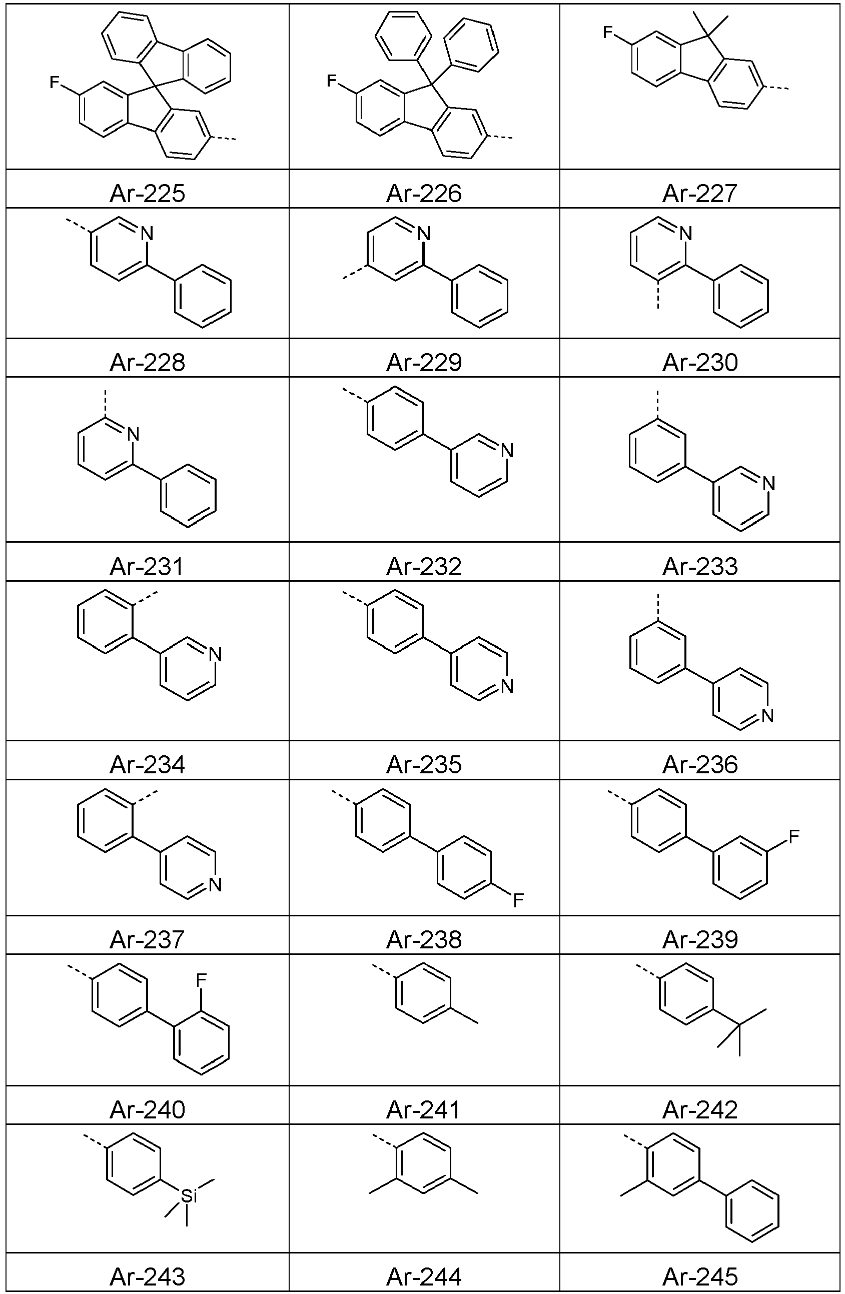

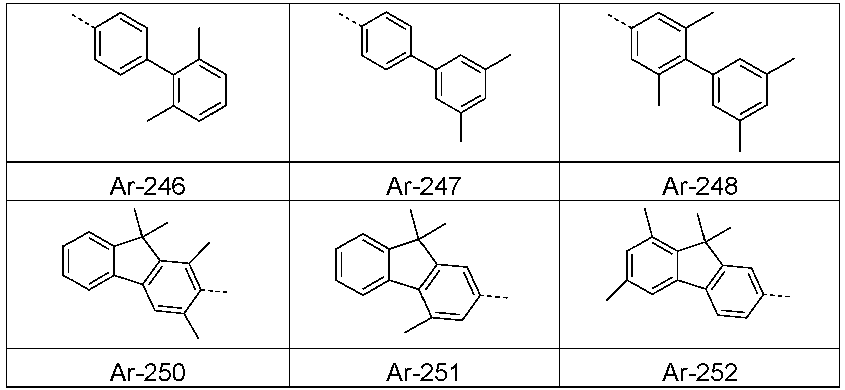

where the groups may be substituted at the free positions with groups R³, but are preferably unsubstituted in these positions, and where the dotted line symbolizes the bonding position to the nitrogen atom.

Particularly preferred groups Ar¹ and Ar² conform to the following formulae Ar-1, Ar-2, Ar-3, Ar-4, Ar-5, Ar-64, Ar-74, Ar-78, Ar-82, Ar-89, Ar-117, Ar-134, Ar-139, Ar-141, Ar-150, Ar-172, and Ar-174.

According to a preferred embodiment, groups Ar¹ and Ar² are not connected by a group E, meaning that index m is 0.

According to an alternative embodiment, which may be preferred under certain conditions, groups Ar¹ and Ar² are connected by a group E, meaning that index m is 1.

In the case that groups Ar¹ and Ar² are connected by a group E, it is preferred that groups Ar¹ and Ar² are selected, identically or differently, from phenyl and fluorenyl, each of which may be substituted by one or more groups R³. Furthermore, in such case, it is preferred that the group E which connects the group Ar¹ and the group Ar² is located on the respective group Ar¹ and Ar², preferably on the respective group Ar¹ and Ar² which is phenyl or fluorenyl, in ortho-position to the bond of the group Ar¹ and Ar² to the amine nitrogen atom. Furthermore, preferably, in such case a six-ring with the amine nitrogen atom is formed of the groups Ar¹, Ar² and E if E is selected from C(R³)₂, NR³, O and S; and a five-ring is formed if E is a single bond.

In the case that groups Ar¹ and Ar² are connected by a group E, particularly preferred embodiments of the moieties 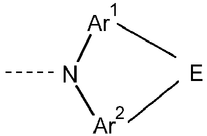 are selected from the following formulae

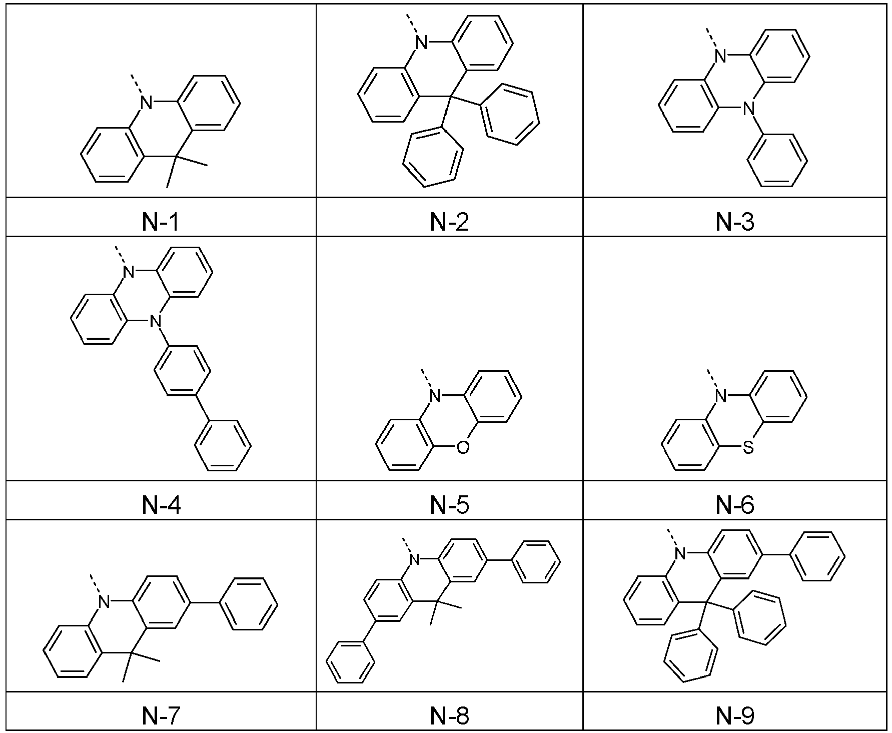

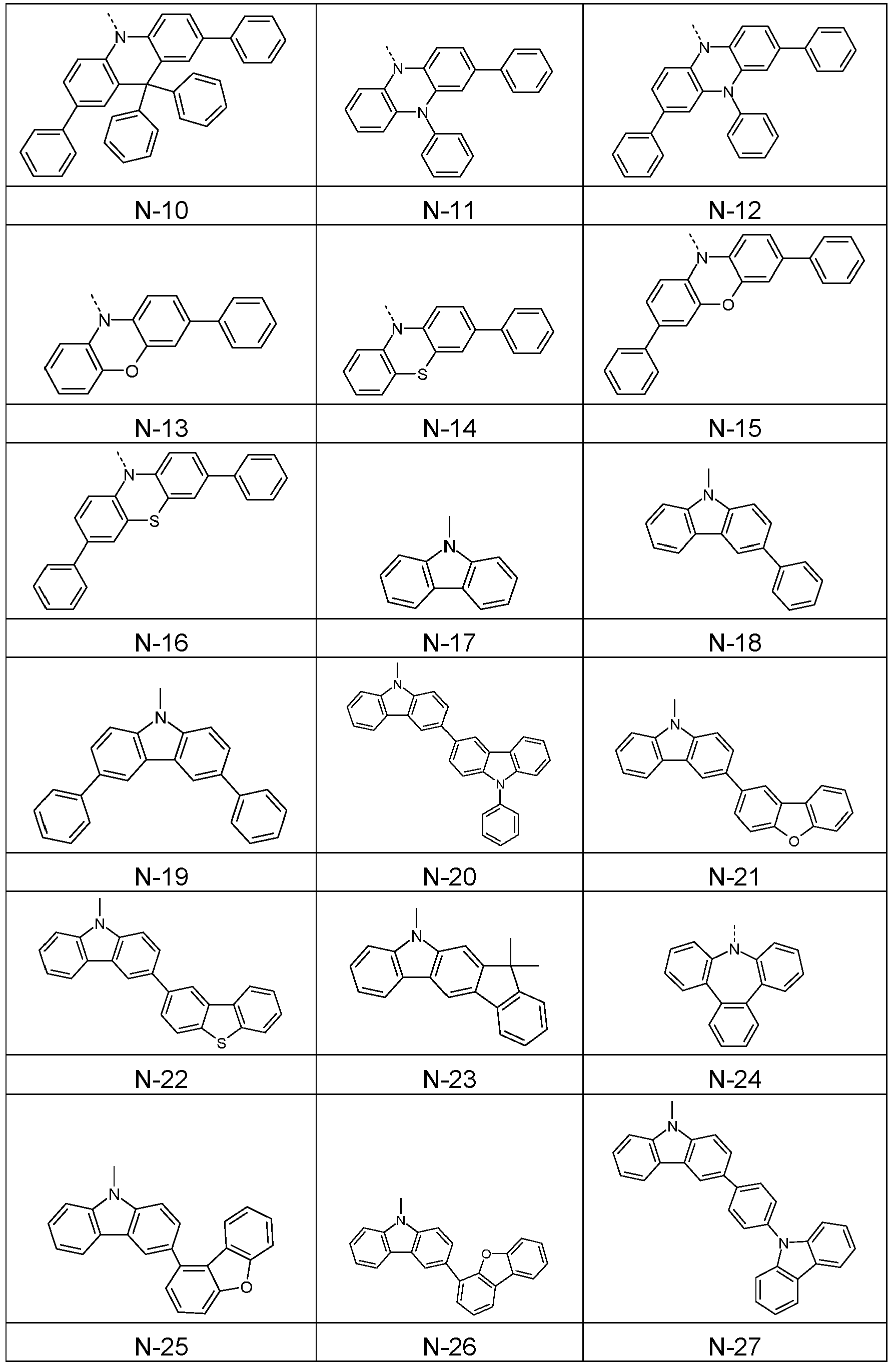

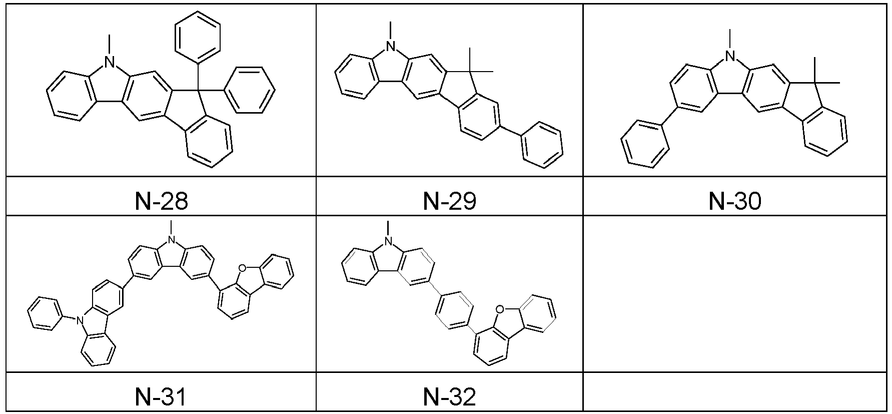

where the groups may be substituted at the free positions with groups R³, but are preferably unsubstituted in these positions, and where the dotted line symbolizes the bonding position to the nitrogen atom.

Groups R¹ are preferably selected, identically or differently on each occurrence, from straight-chain alkyl, alkoxy or thioalkyl groups having 1 to 20 C atoms, which may optionally be substituted by one or more groups F, and from branched or cyclic alkyl, alkoxy or thioalkyl groups having 3 to 20 C atoms, which may optionally be substituted by one or more groups F. Particularly preferred are alkyl groups having 1 to 20 C atoms, which may be substituted by one or more groups F, or groups F; most preferred are F, CF₃, CH₃ and C(CH₃)₃.

Particularly preferred groups R¹ conform to one of the following formulae

| | | |
|---|---|---|
| -CH₃ | -C(CH₃)₃ | -CH₂CH₃ |
| R¹-1 | R¹-2 | R¹-3 |
| -CH₂CH(CH₃)₂ | -CF₃ | -CF₂CF₃ |
| R¹-4 | R¹-5 | R¹-6 |
| -OCF₃ | -SCF₃ | -SF₅ |
| R¹-7 | R¹-8 | R¹-9 |
| -OCF₂CF₃ | -SCF₂CF₃ | 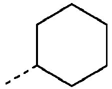 |
| R¹-10 | R¹-11 | R¹-12 |
| 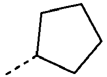 | 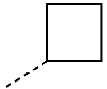 | 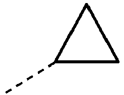 |
| R¹-13 | R¹-14 | R¹-15 |
| -OCH₃ | -SCH₃ | |
| R¹-22 | R¹-23 | |

Among these formulae, formulae R¹-1, R¹-2 and R¹-5 are preferred.

Preferably, R³ is, identically or differently on each occurrence, selected from H, D, F, CN, Si(R⁴)₃, N(R⁴)₂, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R³ may be connected to each other to form a ring; and where the said alkyl, alkoxy, alkenyl and alkynyl groups and the said aromatic and heteroaromatic ring systems may in each case be substituted by one or more radicals R⁴.

Preferably, R⁴ is, identically or differently on each occurrence, selected from H, D, F, CN, Si(R⁵)₃, N(R⁵)₂, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R⁴ may be connected to each other to form a ring; and where the said alkyl, alkoxy, alkenyl and alkynyl groups and the said aromatic and heteroaromatic ring systems may in each case be substituted by one or more radicals R⁵.

Among formulae (IA) and (IB), formula (IA) is preferred.

It is preferred that the compound according to formula (IA) or (IB) conforms to one of formulae (I-A-1) to (I-A-9) and (I-B-1) to (I-B-9), particularly preferably to one of formulae (I-A-1), (I-A-2), (I-B-1) and (I-B-2), most preferably to one of formulae (I-A-1) and (I-B-1)

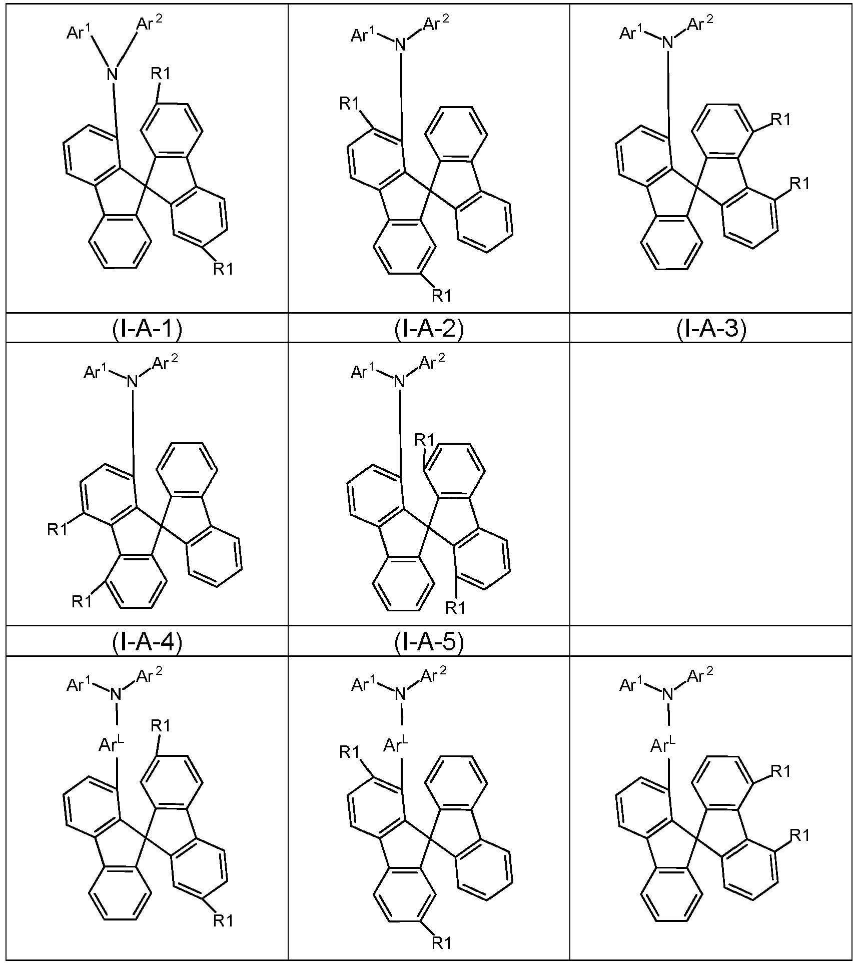

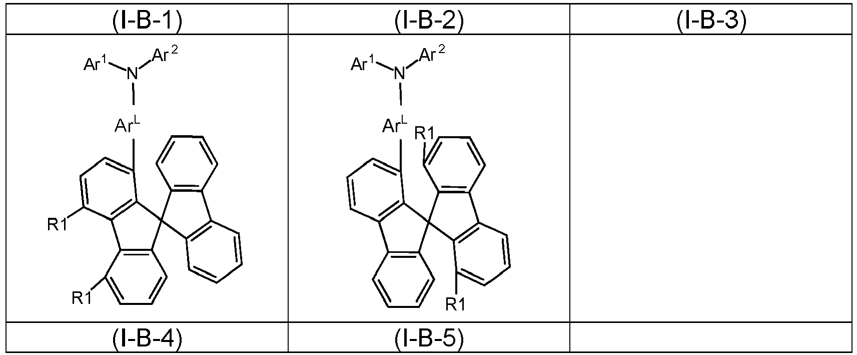

where the variables are defined as above.

Preferred embodiments of compounds according to formula (IA) or (IB) are the compounds given in the following list, where the basic structure conforms to the formula given in the second column, group Ar^{L} if present has the structure given in the third column, groups R¹ conform to the formula given in the fourth column, and groups Ar¹ and Ar² conform to the formulae given in the fifth and sixth column, respectively.

| **No.** | **Basic structure** | **Ar^{L}** | **R¹** | **Ar¹** | **Ar²** |
|---|---|---|---|---|---|
| C-1 | (I-A-1) | n.a. | R-1 | Ar-1 | Ar-1 |
| C-2 | " | " | " | " | Ar-2 |
| C-3 | " | " | " | " | Ar-3 |
| C-4 | " | " | " | " | Ar-4 |
| C-5 | " | " | " | " | Ar-5 |
| C-6 | " | " | " | " | Ar-64 |
| C-7 | " | " | " | " | Ar-74 |
| C-8 | " | " | " | " | Ar-78 |
| C-9 | " | " | " | " | Ar-82 |
| C-10 | " | " | " | " | Ar-89 |
| C-11 | " | " | " | " | Ar-117 |
| C-12 | " | " | " | " | Ar-134 |
| C-13 | " | " | " | " | Ar-139 |
| C-14 | " | " | " | " | Ar-141 |
| C-15 | " | " | " | " | Ar-150 |
| C-16 | " | " | " | " | Ar-172 |
| C-17 | " | " | " | " | Ar-174 |
| C-18 | " | " | " | Ar-2 | Ar-2 |
| C-19 | " | " | " | " | Ar-3 |
| C-20 | " | " | " | " | Ar-4 |
| C-21 | " | " | " | " | Ar-5 |
| C-22 | " | " | " | " | Ar-64 |
| C-23 | " | " | " | " | Ar-74 |
| C-24 | " | " | " | " | Ar-78 |
| C-25 | " | " | " | " | Ar-82 |
| C-26 | " | " | " | " | Ar-89 |
| C-27 | " | " | " | " | Ar-117 |
| C-28 | " | " | " | " | Ar-134 |
| C-29 | " | " | " | " | Ar-139 |
| C-30 | " | " | " | " | Ar-141 |
| C-31 | " | " | " | " | Ar-150 |
| C-32 | " | " | " | " | Ar-172 |
| C-33 | " | " | " | " | Ar-174 |
| C-34 | " | " | " | Ar-3 | Ar-3 |
| C-35 | " | " | " | " | Ar-4 |
| C-36 | " | " | " | " | Ar-5 |
| C-37 | " | " | " | " | Ar-64 |
| C-38 | " | " | " | " | Ar-74 |
| C-39 | " | " | " | " | Ar-78 |
| C-40 | " | " | " | " | Ar-82 |
| C-41 | " | " | " | " | Ar-89 |
| C-42 | " | " | " | " | Ar-117 |
| C-43 | " | " | " | " | Ar-134 |
| C-44 | " | " | " | " | Ar-139 |
| C-45 | " | " | " | " | Ar-141 |
| C-46 | " | " | " | " | Ar-150 |
| C-47 | " | " | " | " | Ar-172 |
| C-48 | " | " | " | " | Ar-174 |
| C-49 | " | " | " | Ar-4 | Ar-4 |
| C-50 | " | " | " | " | Ar-5 |
| C-51 | " | " | " | " | Ar-64 |
| C-52 | " | " | " | " | Ar-74 |
| C-53 | " | " | " | " | Ar-78 |
| C-54 | " | " | " | " | Ar-82 |
| C-55 | " | " | " | " | Ar-89 |
| C-56 | " | " | " | " | Ar-117 |
| C-57 | " | " | " | " | Ar-134 |
| C-58 | " | " | " | " | Ar-139 |
| C-59 | " | " | " | " | Ar-141 |
| C-60 | " | " | " | " | Ar-150 |
| C-61 | " | " | " | " | Ar-172 |
| C-62 | " | " | " | " | Ar-174 |
| C-63 | " | " | " | Ar-5 | Ar-5 |
| C-64 | " | " | " | " | Ar-64 |
| C-65 | " | " | " | " | Ar-74 |
| C-66 | " | " | " | " | Ar-78 |
| C-67 | " | " | " | " | Ar-82 |
| C-68 | " | " | " | " | Ar-89 |
| C-69 | " | " | " | " | Ar-117 |
| C-70 | " | " | " | " | Ar-134 |
| C-71 | " | " | " | " | Ar-139 |
| C-72 | " | " | " | " | Ar-141 |
| C-73 | " | " | " | " | Ar-150 |
| C-74 | " | " | " | " | Ar-172 |
| C-75 | " | " | " | " | Ar-174 |
| C-76 | " | " | " | Ar-64 | Ar-64 |
| C-77 | " | " | " | " | Ar-74 |
| C-78 | " | " | " | " | Ar-78 |
| C-79 | " | " | " | " | Ar-82 |
| C-80 | " | " | " | " | Ar-89 |
| C-81 | " | " | " | " | Ar-117 |
| C-82 | " | " | " | " | Ar-134 |
| C-83 | " | " | " | " | Ar-139 |
| C-84 | " | " | " | " | Ar-141 |
| C-85 | " | " | " | " | Ar-150 |
| C-86 | " | " | " | " | Ar-172 |
| C-87 | " | " | " | " | Ar-174 |
| C-88 | " | " | " | Ar-74 | Ar-74 |
| C-89 | " | " | " | " | Ar-78 |
| C-90 | " | " | " | " | Ar-82 |
| C-91 | " | " | " | " | Ar-89 |
| C-92 | " | " | " | " | Ar-117 |
| C-93 | " | " | " | " | Ar-134 |
| C-94 | " | " | " | " | Ar-139 |
| C-95 | " | " | " | " | Ar-141 |
| C-96 | " | " | " | " | Ar-150 |
| C-97 | " | " | " | " | Ar-172 |
| C-98 | " | " | " | " | Ar-174 |
| C-99 | " | " | " | Ar-78 | Ar-78 |
| C-100 | " | " | " | " | Ar-82 |
| C-101 | " | " | " | " | Ar-89 |
| C-102 | " | " | " | " | Ar-117 |
| C-103 | " | " | " | " | Ar-134 |
| C-104 | " | " | " | " | Ar-139 |
| C-105 | " | " | " | " | Ar-141 |
| C-106 | " | " | " | " | Ar-150 |
| C-107 | " | " | " | " | Ar-172 |
| C-108 | " | " | " | " | Ar-174 |
| C-109 | " | " | " | Ar-82 | Ar-82 |
| C-110 | " | " | " | " | Ar-89 |
| C-111 | " | " | " | " | Ar-117 |
| C-112 | " | " | " | " | Ar-134 |
| C-113 | " | " | " | " | Ar-139 |
| C-114 | " | " | " | " | Ar-141 |
| C-115 | " | " | " | " | Ar-150 |
| C-116 | " | " | " | " | Ar-172 |
| C-117 | " | " | " | " | Ar-174 |
| C-118 | " | " | " | Ar-89 | Ar-89 |
| C-119 | " | " | " | " | Ar-117 |
| C-120 | " | " | " | " | Ar-134 |
| C-121 | " | " | " | " | Ar-139 |
| C-122 | " | " | " | " | Ar-141 |
| C-123 | " | " | " | " | Ar-150 |
| C-124 | " | " | " | " | Ar-172 |
| C-125 | " | " | " | " | Ar-174 |
| C-126 | " | " | " | Ar-117 | Ar-117 |
| C-127 | " | " | " | " | Ar-134 |
| C-128 | " | " | " | " | Ar-139 |
| C-129 | " | " | " | " | Ar-141 |
| C-130 | " | " | " | " | Ar-150 |
| C-131 | " | " | " | " | Ar-172 |
| C-132 | " | " | " | " | Ar-174 |
| C-133 | " | " | " | Ar-134 | Ar-134 |
| C-134 | " | " | " | " | Ar-139 |
| C-135 | " | " | " | " | Ar-141 |
| C-136 | " | " | " | " | Ar-150 |
| C-137 | " | " | " | " | Ar-172 |
| C-138 | " | " | " | " | Ar-174 |
| C-139 | " | " | " | Ar-139 | Ar-139 |
| C-140 | " | " | " | " | Ar-141 |
| C-141 | " | " | " | " | Ar-150 |
| C-142 | " | " | " | " | Ar-172 |
| C-143 | " | " | " | " | Ar-174 |
| C-144 | " | " | " | Ar-141 | Ar-141 |
| C-145 | " | " | " | " | Ar-150 |
| C-146 | " | " | " | " | Ar-172 |
| C-147 | " | " | " | " | Ar-174 |
| C-148 | " | " | " | Ar-150 | Ar-150 |
| C-149 | " | " | " | " | Ar-172 |
| C-150 | " | " | " | " | Ar-174 |
| C-151 | " | " | " | Ar-172 | Ar-172 |
| C-152 | " | " | " | " | Ar-174 |
| C-153 | " | " | " | Ar-174 | Ar-174 |
| C-154 | " | " | R-2 | Ar-1 | Ar-1 |
| C-155 | " | " | " | " | Ar-2 |
| C-156 | " | " | " | " | Ar-3 |
| C-157 | " | " | " | " | Ar-4 |
| C-158 | " | " | " | " | Ar-5 |
| C-159 | " | " | " | " | Ar-64 |
| C-160 | " | " | " | " | Ar-74 |
| C-161 | " | " | " | " | Ar-78 |
| C-162 | " | " | " | " | Ar-82 |
| C-163 | " | " | " | " | Ar-89 |
| C-164 | " | " | " | " | Ar-117 |
| C-165 | " | " | " | " | Ar-134 |
| C-166 | " | " | " | " | Ar-139 |
| C-167 | " | " | " | " | Ar-141 |
| C-168 | " | " | " | " | Ar-150 |
| C-169 | " | " | " | " | Ar-172 |
| C-170 | " | " | " | " | Ar-174 |
| C-171 | " | " | " | Ar-2 | Ar-2 |
| C-172 | " | " | " | " | Ar-3 |
| C-173 | " | " | " | " | Ar-4 |
| C-174 | " | " | " | " | Ar-5 |
| C-175 | " | " | " | " | Ar-64 |
| C-176 | " | " | " | " | Ar-74 |
| C-177 | " | " | " | " | Ar-78 |
| C-178 | " | " | " | " | Ar-82 |
| C-179 | " | " | " | " | Ar-89 |
| C-180 | " | " | " | " | Ar-117 |
| C-181 | " | " | " | " | Ar-134 |
| C-182 | " | " | " | " | Ar-139 |
| C-183 | " | " | " | " | Ar-141 |
| C-184 | " | " | " | " | Ar-150 |
| C-185 | " | " | " | " | Ar-172 |
| C-186 | " | " | " | " | Ar-174 |
| C-187 | " | " | " | Ar-3 | Ar-3 |
| C-188 | " | " | " | " | Ar-4 |
| C-189 | " | " | " | " | Ar-5 |
| C-190 | " | " | " | " | Ar-64 |
| C-191 | " | " | " | " | Ar-74 |
| C-192 | " | " | " | " | Ar-78 |
| C-193 | " | " | " | " | Ar-82 |
| C-194 | " | " | " | " | Ar-89 |
| C-195 | " | " | " | " | Ar-117 |
| C-196 | " | " | " | " | Ar-134 |
| C-197 | " | " | " | " | Ar-139 |
| C-198 | " | " | " | " | Ar-141 |
| C-199 | " | " | " | " | Ar-150 |
| C-200 | " | " | " | " | Ar-172 |
| C-201 | " | " | " | " | Ar-174 |
| C-202 | " | " | " | Ar-4 | Ar-4 |
| C-203 | " | " | " | " | Ar-5 |
| C-204 | " | " | " | " | Ar-64 |
| C-205 | " | " | " | " | Ar-74 |
| C-206 | " | " | " | " | Ar-78 |
| C-207 | " | " | " | " | Ar-82 |
| C-208 | " | " | " | " | Ar-89 |
| C-209 | " | " | " | " | Ar-117 |
| C-210 | " | " | " | " | Ar-134 |
| C-211 | " | " | " | " | Ar-139 |
| C-212 | " | " | " | " | Ar-141 |
| C-213 | " | " | " | " | Ar-150 |
| C-214 | " | " | " | " | Ar-172 |
| C-215 | " | " | " | " | Ar-174 |
| C-216 | " | " | " | Ar-5 | Ar-5 |
| C-217 | " | " | " | " | Ar-64 |
| C-218 | " | " | " | " | Ar-74 |
| C-219 | " | " | " | " | Ar-78 |
| C-220 | " | " | " | " | Ar-82 |
| C-221 | " | " | " | " | Ar-89 |
| C-222 | " | " | " | " | Ar-117 |
| C-223 | " | " | " | " | Ar-134 |
| C-224 | " | " | " | " | Ar-139 |
| C-225 | " | " | " | " | Ar-141 |
| C-226 | " | " | " | " | Ar-150 |
| C-227 | " | " | " | " | Ar-172 |
| C-228 | " | " | " | " | Ar-174 |
| C-229 | " | " | " | Ar-64 | Ar-64 |
| C-230 | " | " | " | " | Ar-74 |
| C-231 | " | " | " | " | Ar-78 |
| C-232 | " | " | " | " | Ar-82 |
| C-233 | " | " | " | " | Ar-89 |
| C-234 | " | " | " | " | Ar-117 |
| C-235 | " | " | " | " | Ar-134 |
| C-236 | " | " | " | " | Ar-139 |
| C-237 | " | " | " | " | Ar-141 |
| C-238 | " | " | " | " | Ar-150 |
| C-239 | " | " | " | " | Ar-172 |
| C-240 | " | " | " | " | Ar-174 |
| C-241 | " | " | " | Ar-74 | Ar-74 |
| C-242 | " | " | " | " | Ar-78 |
| C-243 | " | " | " | " | Ar-82 |
| C-244 | " | " | " | " | Ar-89 |
| C-245 | " | " | " | " | Ar-117 |
| C-246 | " | " | " | " | Ar-134 |
| C-247 | " | " | " | " | Ar-139 |
| C-248 | " | " | " | " | Ar-141 |
| C-249 | " | " | " | " | Ar-150 |
| C-250 | " | " | " | " | Ar-172 |
| C-251 | " | " | " | " | Ar-174 |
| C-252 | " | " | " | Ar-78 | Ar-78 |
| C-253 | " | " | " | " | Ar-82 |
| C-254 | " | " | " | " | Ar-89 |
| C-255 | " | " | " | " | Ar-117 |
| C-256 | " | " | " | " | Ar-134 |
| C-257 | " | " | " | " | Ar-139 |
| C-258 | " | " | " | " | Ar-141 |
| C-259 | " | " | " | " | Ar-150 |
| C-260 | " | " | " | " | Ar-172 |
| C-261 | " | " | " | " | Ar-174 |
| C-262 | " | " | " | Ar-82 | Ar-82 |
| C-263 | " | " | " | " | Ar-89 |
| C-264 | " | " | " | " | Ar-117 |
| C-265 | " | " | " | " | Ar-134 |
| C-266 | " | " | " | " | Ar-139 |
| C-267 | " | " | " | " | Ar-141 |
| C-268 | " | " | " | " | Ar-150 |
| C-269 | " | " | " | " | Ar-172 |
| C-270 | " | " | " | " | Ar-174 |
| C-271 | " | " | " | Ar-89 | Ar-89 |
| C-272 | " | " | " | " | Ar-117 |
| C-273 | " | " | " | " | Ar-134 |
| C-274 | " | " | " | " | Ar-139 |
| C-275 | " | " | " | " | Ar-141 |
| C-276 | " | " | " | " | Ar-150 |
| C-277 | " | " | " | " | Ar-172 |
| C-278 | " | " | " | " | Ar-174 |
| C-279 | " | " | " | Ar-117 | Ar-117 |
| C-280 | " | " | " | " | Ar-134 |
| C-281 | " | " | " | " | Ar-139 |
| C-282 | " | " | " | " | Ar-141 |
| C-283 | " | " | " | " | Ar-150 |
| C-284 | " | " | " | " | Ar-172 |
| C-285 | " | " | " | " | Ar-174 |
| C-286 | " | " | " | Ar-134 | Ar-134 |
| C-287 | " | " | " | " | Ar-139 |
| C-288 | " | " | " | " | Ar-141 |
| C-289 | " | " | " | " | Ar-150 |
| C-290 | " | " | " | " | Ar-172 |
| C-291 | " | " | " | " | Ar-174 |
| C-292 | " | " | " | Ar-139 | Ar-139 |
| C-293 | " | " | " | " | Ar-141 |
| C-294 | " | " | " | " | Ar-150 |
| C-295 | " | " | " | " | Ar-172 |
| C-296 | " | " | " | " | Ar-174 |
| C-297 | " | " | " | Ar-141 | Ar-141 |
| C-298 | " | " | " | " | Ar-150 |
| C-299 | " | " | " | " | Ar-172 |
| C-300 | " | " | " | " | Ar-174 |
| C-301 | " | " | " | Ar-150 | Ar-150 |
| C-302 | " | " | " | " | Ar-172 |
| C-303 | " | " | " | " | Ar-174 |
| C-304 | " | " | " | Ar-172 | Ar-172 |
| C-305 | " | " | " | " | Ar-174 |
| C-306 | " | " | " | Ar-174 | Ar-174 |
| C-307 | " | " | R-5 | Ar-1 | Ar-1 |
| C-308 | " | " | " | " | Ar-2 |
| C-309 | " | " | " | " | Ar-3 |
| C-310 | " | " | " | " | Ar-4 |
| C-311 | " | " | " | " | Ar-5 |
| C-312 | " | " | " | " | Ar-64 |
| C-313 | " | " | " | " | Ar-74 |
| C-314 | " | " | " | " | Ar-78 |
| C-315 | " | " | " | " | Ar-82 |
| C-316 | " | " | " | " | Ar-89 |
| C-317 | " | " | " | " | Ar-117 |
| C-318 | " | " | " | " | Ar-134 |
| C-319 | " | " | " | " | Ar-139 |
| C-320 | " | " | " | " | Ar-141 |
| C-321 | " | " | " | " | Ar-150 |
| C-322 | " | " | " | " | Ar-172 |
| C-323 | " | " | " | " | Ar-174 |
| C-324 | " | " | " | Ar-2 | Ar-2 |
| C-325 | " | " | " | " | Ar-3 |
| C-326 | " | " | " | " | Ar-4 |
| C-327 | " | " | " | " | Ar-5 |
| C-328 | " | " | " | " | Ar-64 |
| C-329 | " | " | " | " | Ar-74 |
| C-330 | " | " | " | " | Ar-78 |
| C-331 | " | " | " | " | Ar-82 |
| C-332 | " | " | " | " | Ar-89 |
| C-333 | " | " | " | " | Ar-117 |
| C-334 | " | " | " | " | Ar-134 |
| C-335 | " | " | " | " | Ar-139 |
| C-336 | " | " | " | " | Ar-141 |
| C-337 | " | " | " | " | Ar-150 |
| C-338 | " | " | " | " | Ar-172 |
| C-339 | " | " | " | " | Ar-174 |
| C-340 | " | " | " | Ar-3 | Ar-3 |
| C-341 | " | " | " | " | Ar-4 |
| C-342 | " | " | " | " | Ar-5 |
| C-343 | " | " | " | " | Ar-64 |
| C-344 | " | " | " | " | Ar-74 |
| C-345 | " | " | " | " | Ar-78 |
| C-346 | " | " | " | " | Ar-82 |
| C-347 | " | " | " | " | Ar-89 |
| C-348 | " | " | " | " | Ar-117 |
| C-349 | " | " | " | " | Ar-134 |
| C-350 | " | " | " | " | Ar-139 |
| C-351 | " | " | " | " | Ar-141 |
| C-352 | " | " | " | " | Ar-150 |
| C-353 | " | " | " | " | Ar-172 |
| C-354 | " | " | " | " | Ar-174 |
| C-355 | " | " | " | Ar-4 | Ar-4 |
| C-356 | " | " | " | " | Ar-5 |
| C-357 | " | " | " | " | Ar-64 |
| C-358 | " | " | " | " | Ar-74 |
| C-359 | " | " | " | " | Ar-78 |
| C-360 | " | " | " | " | Ar-82 |
| C-361 | " | " | " | " | Ar-89 |
| C-362 | " | " | " | " | Ar-117 |
| C-363 | " | " | " | " | Ar-134 |
| C-364 | " | " | " | " | Ar-139 |
| C-365 | " | " | " | " | Ar-141 |
| C-366 | " | " | " | " | Ar-150 |
| C-367 | " | " | " | " | Ar-172 |
| C-368 | " | " | " | " | Ar-174 |
| C-369 | " | " | " | Ar-5 | Ar-5 |
| C-370 | " | " | " | " | Ar-64 |
| C-371 | " | " | " | " | Ar-74 |
| C-372 | " | " | " | " | Ar-78 |
| C-373 | " | " | " | " | Ar-82 |
| C-374 | " | " | " | " | Ar-89 |
| C-375 | " | " | " | " | Ar-117 |
| C-376 | " | " | " | " | Ar-134 |
| C-377 | " | " | " | " | Ar-139 |
| C-378 | " | " | " | " | Ar-141 |
| C-379 | " | " | " | " | Ar-150 |
| C-380 | " | " | " | " | Ar-172 |
| C-381 | " | " | " | " | Ar-174 |
| C-382 | " | " | " | Ar-64 | Ar-64 |
| C-383 | " | " | " | " | Ar-74 |
| C-384 | " | " | " | " | Ar-78 |
| C-385 | " | " | " | " | Ar-82 |
| C-386 | " | " | " | " | Ar-89 |
| C-387 | " | " | " | " | Ar-117 |
| C-388 | " | " | " | " | Ar-134 |
| C-389 | " | " | " | " | Ar-139 |
| C-390 | " | " | " | " | Ar-141 |
| C-391 | " | " | " | " | Ar-150 |
| C-392 | " | " | " | " | Ar-172 |
| C-393 | " | " | " | " | Ar-174 |
| C-394 | " | " | " | Ar-74 | Ar-74 |
| C-395 | " | " | " | " | Ar-78 |
| C-396 | " | " | " | " | Ar-82 |
| C-397 | " | " | " | " | Ar-89 |
| C-398 | " | " | " | " | Ar-117 |
| C-399 | " | " | " | " | Ar-134 |
| C-400 | " | " | " | " | Ar-139 |
| C-401 | " | " | " | " | Ar-141 |
| C-402 | " | " | " | " | Ar-150 |
| C-403 | " | " | " | " | Ar-172 |
| C-404 | " | " | " | " | Ar-174 |
| C-405 | " | " | " | Ar-78 | Ar-78 |
| C-406 | " | " | " | " | Ar-82 |
| C-407 | " | " | " | " | Ar-89 |
| C-408 | " | " | " | " | Ar-117 |
| C-409 | " | " | " | " | Ar-134 |
| C-410 | " | " | " | " | Ar-139 |
| C-411 | " | " | " | " | Ar-141 |
| C-412 | " | " | " | " | Ar-150 |
| C-413 | " | " | " | " | Ar-172 |
| C-414 | " | " | " | " | Ar-174 |
| C-415 | " | " | " | Ar-82 | Ar-82 |
| C-416 | " | " | " | " | Ar-89 |
| C-417 | " | " | " | " | Ar-117 |
| C-418 | " | " | " | " | Ar-134 |
| C-419 | " | " | " | " | Ar-139 |
| C-420 | " | " | " | " | Ar-141 |
| C-421 | " | " | " | " | Ar-150 |
| C-422 | " | " | " | " | Ar-172 |
| C-423 | " | " | " | " | Ar-174 |
| C-424 | " | " | " | Ar-89 | Ar-89 |
| C-425 | " | " | " | " | Ar-117 |
| C-426 | " | " | " | " | Ar-134 |
| C-427 | " | " | " | " | Ar-139 |
| C-428 | " | " | " | " | Ar-141 |
| C-429 | " | " | " | " | Ar-150 |
| C-430 | " | " | " | " | Ar-172 |
| C-431 | " | " | " | " | Ar-174 |
| C-432 | " | " | " | Ar-117 | Ar-117 |
| C-433 | " | " | " | " | Ar-134 |
| C-434 | " | " | " | " | Ar-139 |
| C-435 | " | " | " | " | Ar-141 |
| C-436 | " | " | " | " | Ar-150 |
| C-437 | " | " | " | " | Ar-172 |
| C-438 | " | " | " | " | Ar-174 |
| C-439 | " | " | " | Ar-134 | Ar-134 |
| C-440 | " | " | " | " | Ar-139 |
| C-441 | " | " | " | " | Ar-141 |
| C-442 | " | " | " | " | Ar-150 |
| C-443 | " | " | " | " | Ar-172 |
| C-444 | " | " | " | " | Ar-174 |
| C-445 | " | " | " | Ar-139 | Ar-139 |
| C-446 | " | " | " | " | Ar-141 |
| C-447 | " | " | " | " | Ar-150 |
| C-448 | " | " | " | " | Ar-172 |
| C-449 | " | " | " | " | Ar-174 |
| C-450 | " | " | " | Ar-141 | Ar-141 |
| C-451 | " | " | " | " | Ar-150 |
| C-452 | " | " | " | " | Ar-172 |
| C-453 | " | " | " | " | Ar-174 |
| C-454 | " | " | " | Ar-150 | Ar-150 |
| C-455 | " | " | " | " | Ar-172 |
| C-456 | " | " | " | " | Ar-174 |
| C-457 | " | " | " | Ar-172 | Ar-172 |
| C-458 | " | " | " | " | Ar-174 |
| C-459 | " | " | " | Ar-174 | Ar-174 |
| C-613 | (I-B-1) | 1,4-phenylene | R-1 | Ar-1 | Ar-1 |
| C-614 | " | " | " | " | Ar-2 |
| C-615 | " | " | " | " | Ar-3 |
| C-616 | " | " | " | " | Ar-4 |
| C-617 | " | " | " | " | Ar-5 |
| C-618 | " | " | " | " | Ar-64 |
| C-619 | " | " | " | " | Ar-74 |
| C-620 | " | " | " | " | Ar-78 |
| C-621 | " | " | " | " | Ar-82 |
| C-622 | " | " | " | " | Ar-89 |
| C-623 | " | " | " | " | Ar-117 |
| C-624 | " | " | " | " | Ar-134 |
| C-625 | " | " | " | " | Ar-139 |
| C-626 | " | " | " | " | Ar-141 |
| C-627 | " | " | " | " | Ar-150 |
| C-628 | " | " | " | " | Ar-172 |
| C-629 | " | " | " | " | Ar-174 |
| C-630 | " | " | " | Ar-2 | Ar-2 |
| C-631 | " | " | " | " | Ar-3 |
| C-632 | " | " | " | " | Ar-4 |
| C-633 | " | " | " | " | Ar-5 |
| C-634 | " | " | " | " | Ar-64 |
| C-635 | " | " | " | " | Ar-74 |
| C-636 | " | " | " | " | Ar-78 |
| C-637 | " | " | " | " | Ar-82 |
| C-638 | " | " | " | " | Ar-89 |
| C-639 | " | " | " | " | Ar-117 |
| C-640 | " | " | " | " | Ar-134 |
| C-641 | " | " | " | " | Ar-139 |
| C-642 | " | " | " | " | Ar-141 |
| C-643 | " | " | " | " | Ar-150 |
| C-644 | " | " | " | " | Ar-172 |
| C-645 | " | " | " | " | Ar-174 |
| C-646 | " | " | " | Ar-3 | Ar-3 |
| C-647 | " | " | " | " | Ar-4 |
| C-648 | " | " | " | " | Ar-5 |
| C-649 | " | " | " | " | Ar-64 |
| C-650 | " | " | " | " | Ar-74 |
| C-651 | " | " | " | " | Ar-78 |
| C-652 | " | " | " | " | Ar-82 |
| C-653 | " | " | " | " | Ar-89 |
| C-654 | " | " | " | " | Ar-117 |
| C-655 | " | " | " | " | Ar-134 |
| C-656 | " | " | " | " | Ar-139 |
| C-657 | " | " | " | " | Ar-141 |
| C-658 | " | " | " | " | Ar-150 |
| C-659 | " | " | " | " | Ar-172 |
| C-660 | " | " | " | " | Ar-174 |
| C-661 | " | " | " | Ar-4 | Ar-4 |
| C-662 | " | " | " | " | Ar-5 |
| C-663 | " | " | " | " | Ar-64 |
| C-664 | " | " | " | " | Ar-74 |
| C-665 | " | " | " | " | Ar-78 |
| C-666 | " | " | " | " | Ar-82 |
| C-667 | " | " | " | " | Ar-89 |
| C-668 | " | " | " | " | Ar-117 |
| C-669 | " | " | " | " | Ar-134 |
| C-670 | " | " | " | " | Ar-139 |
| C-671 | " | " | " | " | Ar-141 |
| C-672 | " | " | " | " | Ar-150 |
| C-673 | " | " | " | " | Ar-172 |
| C-674 | " | " | " | " | Ar-174 |
| C-675 | " | " | " | Ar-5 | Ar-5 |
| C-676 | " | " | " | " | Ar-64 |
| C-677 | " | " | " | " | Ar-74 |
| C-678 | " | " | " | " | Ar-78 |
| C-679 | " | " | " | " | Ar-82 |
| C-680 | " | " | " | " | Ar-89 |
| C-681 | " | " | " | " | Ar-117 |
| C-682 | " | " | " | " | Ar-134 |
| C-683 | " | " | " | " | Ar-139 |
| C-684 | " | " | " | " | Ar-141 |
| C-685 | " | " | " | " | Ar-150 |
| C-686 | " | " | " | " | Ar-172 |
| C-687 | " | " | " | " | Ar-174 |
| C-688 | " | " | " | Ar-64 | Ar-64 |
| C-689 | " | " | " | " | Ar-74 |
| C-690 | " | " | " | " | Ar-78 |
| C-691 | " | " | " | " | Ar-82 |
| C-692 | " | " | " | " | Ar-89 |
| C-693 | " | " | " | " | Ar-117 |
| C-694 | " | " | " | " | Ar-134 |
| C-695 | " | " | " | " | Ar-139 |
| C-696 | " | " | " | " | Ar-141 |
| C-697 | " | " | " | " | Ar-150 |
| C-698 | " | " | " | " | Ar-172 |
| C-699 | " | " | " | " | Ar-174 |
| C-700 | " | " | " | Ar-74 | Ar-74 |
| C-701 | " | " | " | " | Ar-78 |
| C-702 | " | " | " | " | Ar-82 |
| C-703 | " | " | " | " | Ar-89 |
| C-704 | " | " | " | " | Ar-117 |
| C-705 | " | " | " | " | Ar-134 |
| C-706 | " | " | " | " | Ar-139 |
| C-707 | " | " | " | " | Ar-141 |
| C-708 | " | " | " | " | Ar-150 |
| C-709 | " | " | " | " | Ar-172 |
| C-710 | " | " | " | " | Ar-174 |
| C-711 | " | " | " | Ar-78 | Ar-78 |
| C-712 | " | " | " | " | Ar-82 |
| C-713 | " | " | " | " | Ar-89 |
| C-714 | " | " | " | " | Ar-117 |
| C-715 | " | " | " | " | Ar-134 |
| C-716 | " | " | " | " | Ar-139 |
| C-717 | " | " | " | " | Ar-141 |
| C-718 | " | " | " | " | Ar-150 |
| C-719 | " | " | " | " | Ar-172 |
| C-720 | " | " | " | " | Ar-174 |
| C-721 | " | " | " | Ar-82 | Ar-82 |
| C-722 | " | " | " | " | Ar-89 |
| C-723 | " | " | " | " | Ar-117 |
| C-724 | " | " | " | " | Ar-134 |
| C-725 | " | " | " | " | Ar-139 |
| C-726 | " | " | " | " | Ar-141 |
| C-727 | " | " | " | " | Ar-150 |
| C-728 | " | " | " | " | Ar-172 |
| C-729 | " | " | " | " | Ar-174 |
| C-730 | " | " | " | Ar-89 | Ar-89 |
| C-731 | " | " | " | " | Ar-117 |
| C-732 | " | " | " | " | Ar-134 |
| C-733 | " | " | " | " | Ar-139 |
| C-734 | " | " | " | " | Ar-141 |
| C-735 | " | " | " | " | Ar-150 |
| C-736 | " | " | " | " | Ar-172 |
| C-737 | " | " | " | " | Ar-174 |
| C-738 | " | " | " | Ar-117 | Ar-117 |
| C-739 | " | " | " | " | Ar-134 |
| C-740 | " | " | " | " | Ar-139 |
| C-741 | " | " | " | " | Ar-141 |
| C-742 | " | " | " | " | Ar-150 |
| C-743 | " | " | " | " | Ar-172 |
| C-744 | " | " | " | " | Ar-174 |
| C-745 | " | " | " | Ar-134 | Ar-134 |
| C-746 | " | " | " | " | Ar-139 |
| C-747 | " | " | " | " | Ar-141 |
| C-748 | " | " | " | " | Ar-150 |
| C-749 | " | " | " | " | Ar-172 |
| C-750 | " | " | " | " | Ar-174 |
| C-751 | " | " | " | Ar-139 | Ar-139 |
| C-752 | " | " | " | " | Ar-141 |
| C-753 | " | " | " | " | Ar-150 |
| C-754 | " | " | " | " | Ar-172 |
| C-755 | " | " | " | " | Ar-174 |
| C-756 | " | " | " | Ar-141 | Ar-141 |
| C-757 | " | " | " | " | Ar-150 |
| C-758 | " | " | " | " | Ar-172 |
| C-759 | " | " | " | " | Ar-174 |
| C-760 | " | " | " | Ar-150 | Ar-150 |
| C-761 | " | " | " | " | Ar-172 |
| C-762 | " | " | " | " | Ar-174 |
| C-763 | " | " | " | Ar-172 | Ar-172 |
| C-764 | " | " | " | " | Ar-174 |
| C-765 | " | " | " | Ar-174 | Ar-174 |
| C-766 | " | " | R-2 | Ar-1 | Ar-1 |
| C-767 | " | " | " | " | Ar-2 |
| C-768 | " | " | " | " | Ar-3 |
| C-769 | " | " | " | " | Ar-4 |
| C-770 | " | " | " | " | Ar-5 |
| C-771 | " | " | " | " | Ar-64 |
| C-772 | " | " | " | " | Ar-74 |
| C-773 | " | " | " | " | Ar-78 |
| C-774 | " | " | " | " | Ar-82 |
| C-775 | " | " | " | " | Ar-89 |
| C-776 | " | " | " | " | Ar-117 |
| C-777 | " | " | " | " | Ar-134 |
| C-778 | " | " | " | " | Ar-139 |
| C-779 | " | " | " | " | Ar-141 |
| C-780 | " | " | " | " | Ar-150 |
| C-781 | " | " | " | " | Ar-172 |
| C-782 | " | " | " | " | Ar-174 |
| C-783 | " | " | " | Ar-2 | Ar-2 |
| C-784 | " | " | " | " | Ar-3 |
| C-785 | " | " | " | " | Ar-4 |
| C-786 | " | " | " | " | Ar-5 |
| C-787 | " | " | " | " | Ar-64 |
| C-788 | " | " | " | " | Ar-74 |
| C-789 | " | " | " | " | Ar-78 |
| C-790 | " | " | " | " | Ar-82 |
| C-791 | " | " | " | " | Ar-89 |
| C-792 | " | " | " | " | Ar-117 |
| C-793 | " | " | " | " | Ar-134 |
| C-794 | " | " | " | " | Ar-139 |
| C-795 | " | " | " | " | Ar-141 |
| C-796 | " | " | " | " | Ar-150 |
| C-797 | " | " | " | " | Ar-172 |
| C-798 | " | " | " | " | Ar-174 |
| C-799 | " | " | " | Ar-3 | Ar-3 |
| C-800 | " | " | " | " | Ar-4 |
| C-801 | " | " | " | " | Ar-5 |
| C-802 | " | " | " | " | Ar-64 |
| C-803 | " | " | " | " | Ar-74 |
| C-804 | " | " | " | " | Ar-78 |
| C-805 | " | " | " | " | Ar-82 |
| C-806 | " | " | " | " | Ar-89 |
| C-807 | " | " | " | " | Ar-117 |
| C-808 | " | " | " | " | Ar-134 |
| C-809 | " | " | " | " | Ar-139 |
| C-810 | " | " | " | " | Ar-141 |
| C-811 | " | " | " | " | Ar-150 |
| C-812 | " | " | " | " | Ar-172 |
| C-813 | " | " | " | " | Ar-174 |
| C-814 | " | " | " | Ar-4 | Ar-4 |
| C-815 | " | " | " | " | Ar-5 |
| C-816 | " | " | " | " | Ar-64 |
| C-817 | " | " | " | " | Ar-74 |
| C-818 | " | " | " | " | Ar-78 |
| C-819 | " | " | " | " | Ar-82 |
| C-820 | " | " | " | " | Ar-89 |
| C-821 | " | " | " | " | Ar-117 |
| C-822 | " | " | " | " | Ar-134 |
| C-823 | " | " | " | " | Ar-139 |
| C-824 | " | " | " | " | Ar-141 |
| C-825 | " | " | " | " | Ar-150 |
| C-826 | " | " | " | " | Ar-172 |
| C-827 | " | " | " | " | Ar-174 |
| C-828 | " | " | " | Ar-5 | Ar-5 |
| C-829 | " | " | " | " | Ar-64 |
| C-830 | " | " | " | " | Ar-74 |
| C-831 | " | " | " | " | Ar-78 |
| C-832 | " | " | " | " | Ar-82 |
| C-833 | " | " | " | " | Ar-89 |
| C-834 | " | " | " | " | Ar-117 |
| C-835 | " | " | " | " | Ar-134 |
| C-836 | " | " | " | " | Ar-139 |
| C-837 | " | " | " | " | Ar-141 |
| C-838 | " | " | " | " | Ar-150 |
| C-839 | " | " | " | " | Ar-172 |
| C-840 | " | " | " | " | Ar-174 |
| C-841 | " | " | " | Ar-64 | Ar-64 |
| C-842 | " | " | " | " | Ar-74 |
| C-843 | " | " | " | " | Ar-78 |
| C-844 | " | " | " | " | Ar-82 |
| C-845 | " | " | " | " | Ar-89 |
| C-846 | " | " | " | " | Ar-117 |
| C-847 | " | " | " | " | Ar-134 |
| C-848 | " | " | " | " | Ar-139 |
| C-849 | " | " | " | " | Ar-141 |
| C-850 | " | " | " | " | Ar-150 |
| C-851 | " | " | " | " | Ar-172 |
| C-852 | " | " | " | " | Ar-174 |
| C-853 | " | " | " | Ar-74 | Ar-74 |
| C-854 | " | " | " | " | Ar-78 |
| C-855 | " | " | " | " | Ar-82 |
| C-856 | " | " | " | " | Ar-89 |
| C-857 | " | " | " | " | Ar-117 |
| C-858 | " | " | " | " | Ar-134 |
| C-859 | " | " | " | " | Ar-139 |
| C-860 | " | " | " | " | Ar-141 |
| C-861 | " | " | " | " | Ar-150 |
| C-862 | " | " | " | " | Ar-172 |
| C-863 | " | " | " | " | Ar-174 |
| C-864 | " | " | " | Ar-78 | Ar-78 |
| C-865 | " | " | " | " | Ar-82 |
| C-866 | " | " | " | " | Ar-89 |
| C-867 | " | " | " | " | Ar-117 |
| C-868 | " | " | " | " | Ar-134 |
| C-869 | " | " | " | " | Ar-139 |
| C-870 | " | " | " | " | Ar-141 |
| C-871 | " | " | " | " | Ar-150 |
| C-872 | " | " | " | " | Ar-172 |
| C-873 | " | " | " | " | Ar-174 |
| C-874 | " | " | " | Ar-82 | Ar-82 |
| C-875 | " | " | " | " | Ar-89 |
| C-876 | " | " | " | " | Ar-117 |
| C-877 | " | " | " | " | Ar-134 |
| C-878 | " | " | " | " | Ar-139 |
| C-879 | " | " | " | " | Ar-141 |
| C-880 | " | " | " | " | Ar-150 |
| C-881 | " | " | " | " | Ar-172 |
| C-882 | " | " | " | " | Ar-174 |
| C-883 | " | " | " | Ar-89 | Ar-89 |
| C-884 | " | " | " | " | Ar-117 |
| C-885 | " | " | " | " | Ar-134 |
| C-886 | " | " | " | " | Ar-139 |
| C-887 | " | " | " | " | Ar-141 |
| C-888 | " | " | " | " | Ar-150 |
| C-889 | " | " | " | " | Ar-172 |
| C-890 | " | " | " | " | Ar-174 |
| C-891 | " | " | " | Ar-117 | Ar-117 |
| C-892 | " | " | " | " | Ar-134 |
| C-893 | " | " | " | " | Ar-139 |
| C-894 | " | " | " | " | Ar-141 |
| C-895 | " | " | " | " | Ar-150 |
| C-896 | " | " | " | " | Ar-172 |
| C-897 | " | " | " | " | Ar-174 |
| C-898 | " | " | " | Ar-134 | Ar-134 |
| C-899 | " | " | " | " | Ar-139 |
| C-900 | " | " | " | " | Ar-141 |
| C-901 | " | " | " | " | Ar-150 |
| C-902 | " | " | " | " | Ar-172 |
| C-903 | " | " | " | " | Ar-174 |
| C-904 | " | " | " | Ar-139 | Ar-139 |
| C-905 | " | " | " | " | Ar-141 |
| C-906 | " | " | " | " | Ar-150 |
| C-907 | " | " | " | " | Ar-172 |
| C-908 | " | " | " | " | Ar-174 |
| C-909 | " | " | " | Ar-141 | Ar-141 |
| C-910 | " | " | " | " | Ar-150 |
| C-911 | " | " | " | " | Ar-172 |
| C-912 | " | " | " | " | Ar-174 |
| C-913 | " | " | " | Ar-150 | Ar-150 |
| C-914 | " | " | " | " | Ar-172 |
| C-915 | " | " | " | " | Ar-174 |
| C-916 | " | " | " | Ar-172 | Ar-172 |
| C-917 | " | " | " | " | Ar-174 |
| C-918 | " | " | " | Ar-174 | Ar-174 |
| C-919 | " | " | R-5 | Ar-1 | Ar-1 |
| C-920 | " | " | " | " | Ar-2 |
| C-921 | " | " | " | " | Ar-3 |
| C-922 | " | " | " | " | Ar-4 |
| C-923 | " | " | " | " | Ar-5 |
| C-924 | " | " | " | " | Ar-64 |
| C-925 | " | " | " | " | Ar-74 |
| C-926 | " | " | " | " | Ar-78 |
| C-927 | " | " | " | " | Ar-82 |
| C-928 | " | " | " | " | Ar-89 |
| C-929 | " | " | " | " | Ar-117 |
| C-930 | " | " | " | " | Ar-134 |
| C-931 | " | " | " | " | Ar-139 |
| C-932 | " | " | " | " | Ar-141 |
| C-933 | " | " | " | " | Ar-150 |
| C-934 | " | " | " | " | Ar-172 |
| C-935 | " | " | " | " | Ar-174 |
| C-936 | " | " | " | Ar-2 | Ar-2 |
| C-937 | " | " | " | " | Ar-3 |
| C-938 | " | " | " | " | Ar-4 |
| C-939 | " | " | " | " | Ar-5 |
| C-940 | " | " | " | " | Ar-64 |
| C-941 | " | " | " | " | Ar-74 |
| C-942 | " | " | " | " | Ar-78 |
| C-943 | " | " | " | " | Ar-82 |
| C-944 | " | " | " | " | Ar-89 |
| C-945 | " | " | " | " | Ar-117 |
| C-946 | " | " | " | " | Ar-134 |
| C-947 | " | " | " | " | Ar-139 |
| C-948 | " | " | " | " | Ar-141 |
| C-949 | " | " | " | " | Ar-150 |
| C-950 | " | " | " | " | Ar-172 |
| C-951 | " | " | " | " | Ar-174 |
| C-952 | " | " | " | Ar-3 | Ar-3 |
| C-953 | " | " | " | " | Ar-4 |
| C-954 | " | " | " | " | Ar-5 |
| C-955 | " | " | " | " | Ar-64 |
| C-956 | " | " | " | " | Ar-74 |
| C-957 | " | " | " | " | Ar-78 |
| C-958 | " | " | " | " | Ar-82 |
| C-959 | " | " | " | " | Ar-89 |
| C-960 | " | " | " | " | Ar-117 |
| C-961 | " | " | " | " | Ar-134 |
| C-962 | " | " | " | " | Ar-139 |
| C-963 | " | " | " | " | Ar-141 |
| C-964 | " | " | " | " | Ar-150 |
| C-965 | " | " | " | " | Ar-172 |
| C-966 | " | " | " | " | Ar-174 |
| C-967 | " | " | " | Ar-4 | Ar-4 |
| C-968 | " | " | " | " | Ar-5 |
| C-969 | " | " | " | " | Ar-64 |
| C-970 | " | " | " | " | Ar-74 |
| C-971 | " | " | " | " | Ar-78 |
| C-972 | " | " | " | " | Ar-82 |
| C-973 | " | " | " | " | Ar-89 |
| C-974 | " | " | " | " | Ar-117 |
| C-975 | " | " | " | " | Ar-134 |
| C-976 | " | " | " | " | Ar-139 |
| C-977 | " | " | " | " | Ar-141 |
| C-978 | " | " | " | " | Ar-150 |
| C-979 | " | " | " | " | Ar-172 |
| C-980 | " | " | " | " | Ar-174 |
| C-981 | " | " | " | Ar-5 | Ar-5 |
| C-982 | " | " | " | " | Ar-64 |
| C-983 | " | " | " | " | Ar-74 |
| C-984 | " | " | " | " | Ar-78 |
| C-985 | " | " | " | " | Ar-82 |
| C-986 | " | " | " | " | Ar-89 |
| C-987 | " | " | " | " | Ar-117 |
| C-988 | " | " | " | " | Ar-134 |
| C-989 | " | " | " | " | Ar-139 |
| C-990 | " | " | " | " | Ar-141 |
| C-991 | " | " | " | " | Ar-150 |
| C-992 | " | " | " | " | Ar-172 |
| C-993 | " | " | " | " | Ar-174 |
| C-994 | " | " | " | Ar-64 | Ar-64 |
| C-995 | " | " | " | " | Ar-74 |
| C-996 | " | " | " | " | Ar-78 |
| C-997 | " | " | " | " | Ar-82 |
| C-998 | " | " | " | " | Ar-89 |
| C-999 | " | " | " | " | Ar-117 |
| C-1000 | " | " | " | " | Ar-134 |
| C-1001 | " | " | " | " | Ar-139 |
| C-1002 | " | " | " | " | Ar-141 |
| C-1003 | " | " | " | " | Ar-150 |
| C-1004 | " | " | " | " | Ar-172 |
| C-1005 | " | " | " | " | Ar-174 |
| C-1006 | " | " | " | Ar-74 | Ar-74 |
| C-1007 | " | " | " | " | Ar-78 |
| C-1008 | " | " | " | " | Ar-82 |
| C-1009 | " | " | " | " | Ar-89 |
| C-1010 | " | " | " | " | Ar-117 |
| C-1011 | " | " | " | " | Ar-134 |
| C-1012 | " | " | " | " | Ar-139 |
| C-1013 | " | " | " | " | Ar-141 |
| C-1014 | " | " | " | " | Ar-150 |
| C-1015 | " | " | " | " | Ar-172 |
| C-1016 | " | " | " | " | Ar-174 |
| C-1017 | " | " | " | Ar-78 | Ar-78 |
| C-1018 | " | " | " | " | Ar-82 |
| C-1019 | " | " | " | " | Ar-89 |
| C-1020 | " | " | " | " | Ar-117 |
| C-1021 | " | " | " | " | Ar-134 |
| C-1022 | " | " | " | " | Ar-139 |
| C-1023 | " | " | " | " | Ar-141 |
| C-1024 | " | " | " | " | Ar-150 |
| C-1025 | " | " | " | " | Ar-172 |
| C-1026 | " | " | " | " | Ar-174 |
| C-1027 | " | " | " | Ar-82 | Ar-82 |
| C-1028 | " | " | " | " | Ar-89 |
| C-1029 | " | " | " | " | Ar-117 |
| C-1030 | " | " | " | " | Ar-134 |
| C-1031 | " | " | " | " | Ar-139 |
| C-1032 | " | " | " | " | Ar-141 |
| C-1033 | " | " | " | " | Ar-150 |
| C-1034 | " | " | " | " | Ar-172 |
| C-1035 | " | " | " | " | Ar-174 |
| C-1036 | " | " | " | Ar-89 | Ar-89 |
| C-1037 | " | " | " | " | Ar-117 |
| C-1038 | " | " | " | " | Ar-134 |
| C-1039 | " | " | " | " | Ar-139 |
| C-1040 | " | " | " | " | Ar-141 |
| C-1041 | " | " | " | " | Ar-150 |
| C-1042 | " | " | " | " | Ar-172 |
| C-1043 | " | " | " | " | Ar-174 |
| C-1044 | " | " | " | Ar-117 | Ar-117 |
| C-1045 | " | " | " | " | Ar-134 |
| C-1046 | " | " | " | " | Ar-139 |
| C-1047 | " | " | " | " | Ar-141 |
| C-1048 | " | " | " | " | Ar-150 |
| C-1049 | " | " | " | " | Ar-172 |
| C-1050 | " | " | " | " | Ar-174 |
| C-1051 | " | " | " | Ar-134 | Ar-134 |
| C-1052 | " | " | " | " | Ar-139 |
| C-1053 | " | " | " | " | Ar-141 |
| C-1054 | " | " | " | " | Ar-150 |
| C-1055 | " | " | " | " | Ar-172 |
| C-1056 | " | " | " | " | Ar-174 |
| C-1057 | " | " | " | Ar-139 | Ar-139 |
| C-1058 | " | " | " | " | Ar-141 |
| C-1059 | " | " | " | " | Ar-150 |
| C-1060 | " | " | " | " | Ar-172 |
| C-1061 | " | " | " | " | Ar-174 |
| C-1062 | " | " | " | Ar-141 | Ar-141 |
| C-1063 | " | " | " | " | Ar-150 |
| C-1064 | " | " | " | " | Ar-172 |
| C-1065 | " | " | " | " | Ar-174 |
| C-1066 | " | " | " | Ar-150 | Ar-150 |
| C-1067 | " | " | " | " | Ar-172 |
| C-1068 | " | " | " | " | Ar-174 |
| C-1069 | " | " | " | Ar-172 | Ar-172 |
| C-1070 | " | " | " | " | Ar-174 |
| C-1071 | " | " | " | Ar-174 | Ar-174 |

Further preferred compounds are analogues of the compounds of the above table, which differ in the feature that they have a basic structure according to one of formulae (I-A-2) to (I-A-9) and (I-B-2) to (I-B-9).

Further preferred compounds are analogues of the compounds C-613 to C-1071 of the above table, which differ in the feature that they have instead of a group Ar^{L} which is 1 ,4-phenylene a group Ar^{L} which conforms to one of formulae Ar^{L}-1, Ar^{L}-2, Ar^{L}-3, Ar^{L}-9, Ar^{L}-12, Ar^{L}-16, Ar^{L}-17, Ar^{L}-36, Ar^{L}-64, and Ar^{L}-73.

The below with # marked compounds are not in scope with the claims.

Preferred specific compounds according to formula (IA) or (IB) are the following ones:

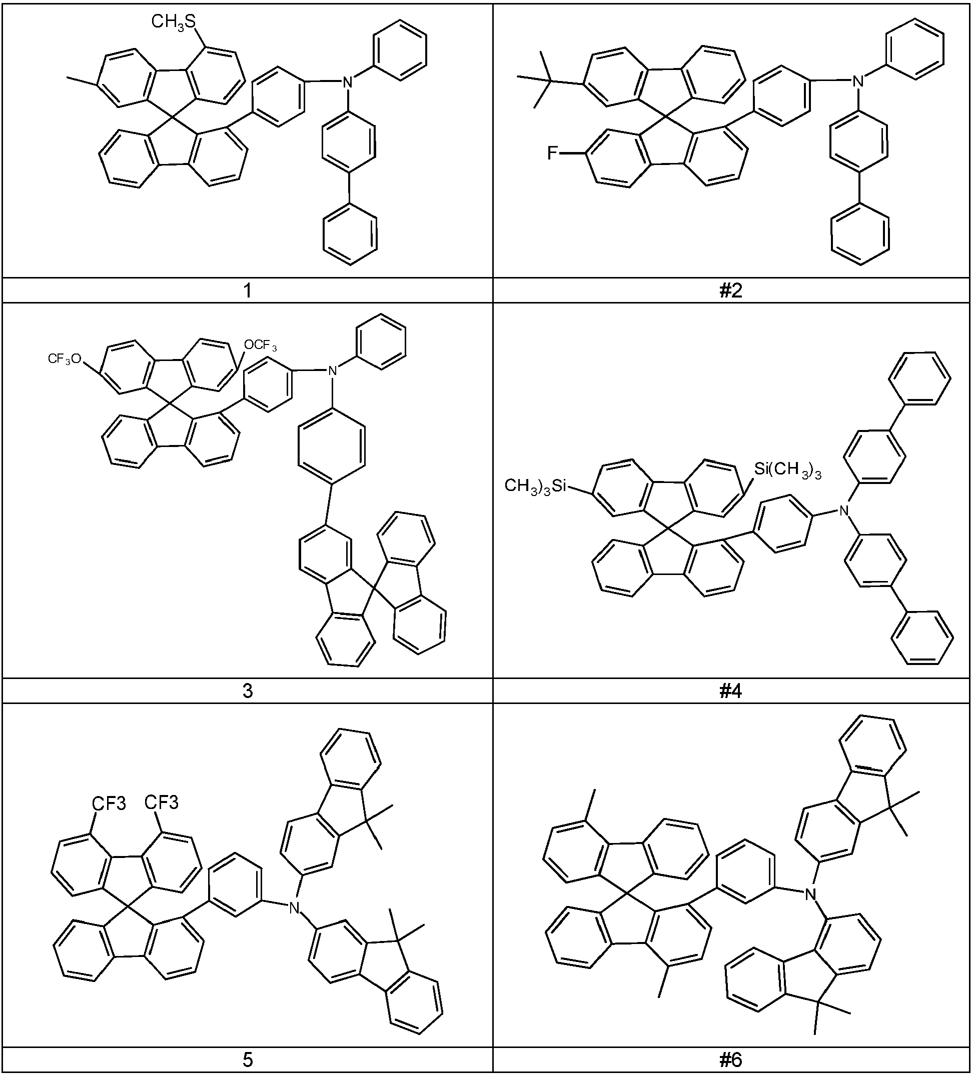

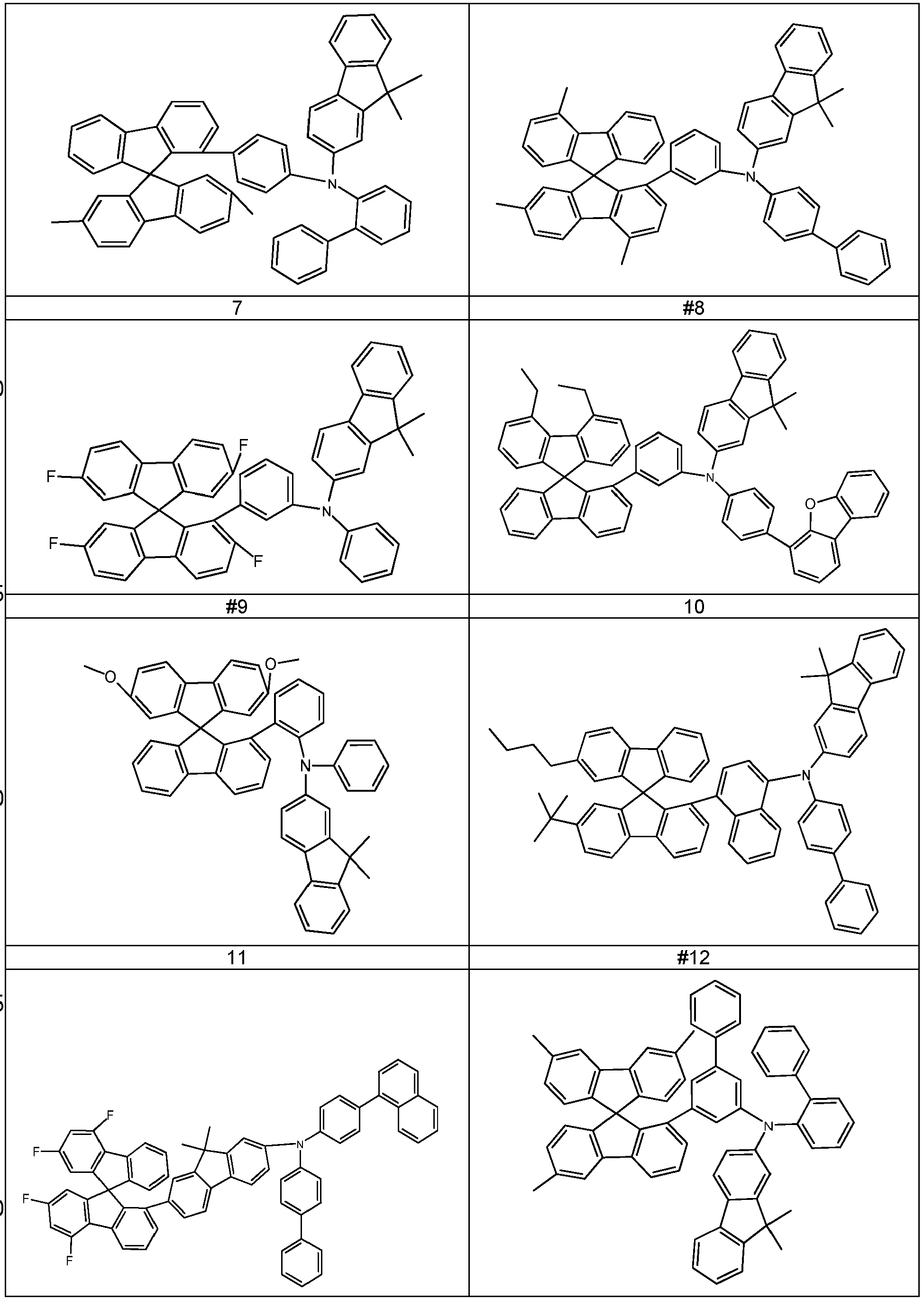

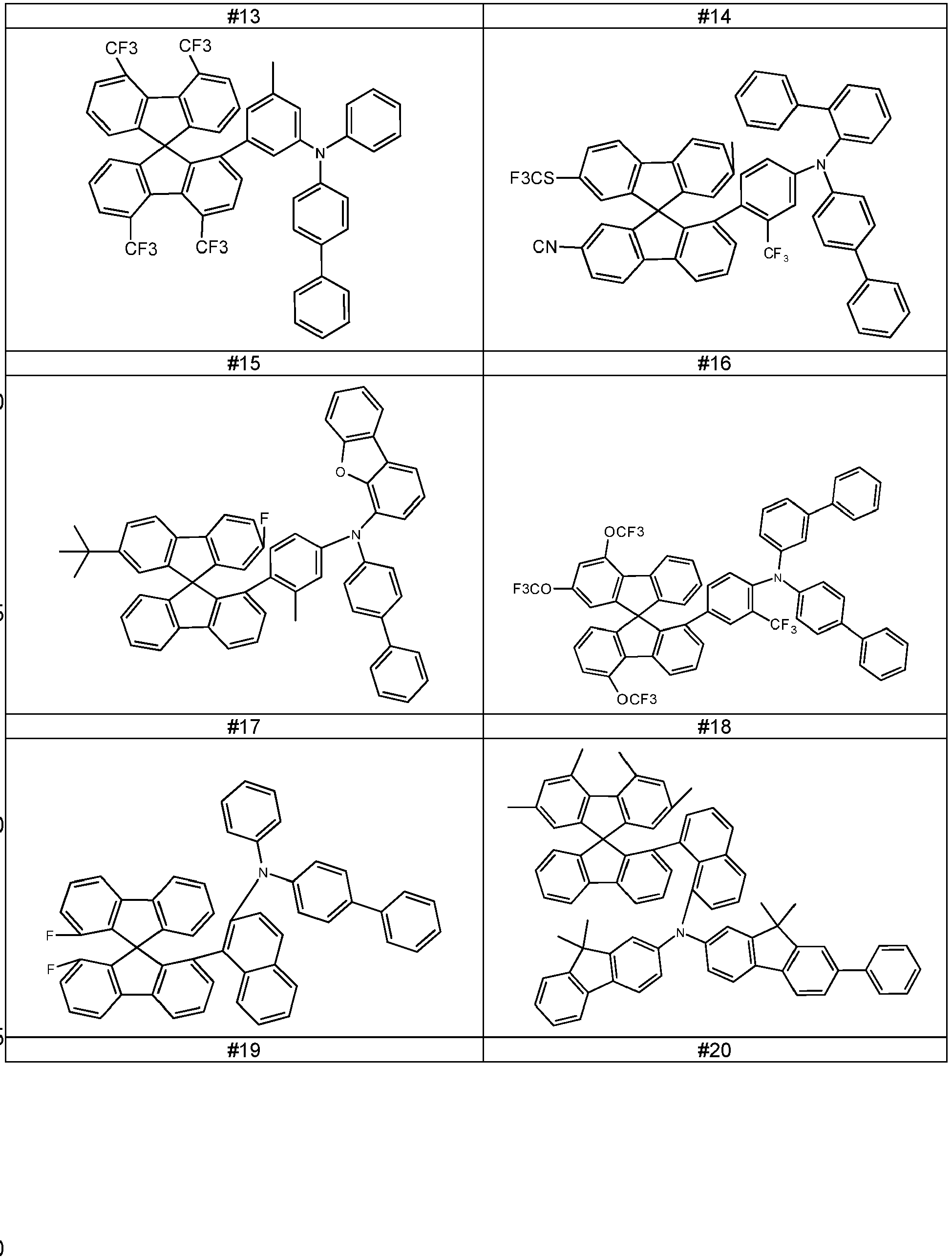

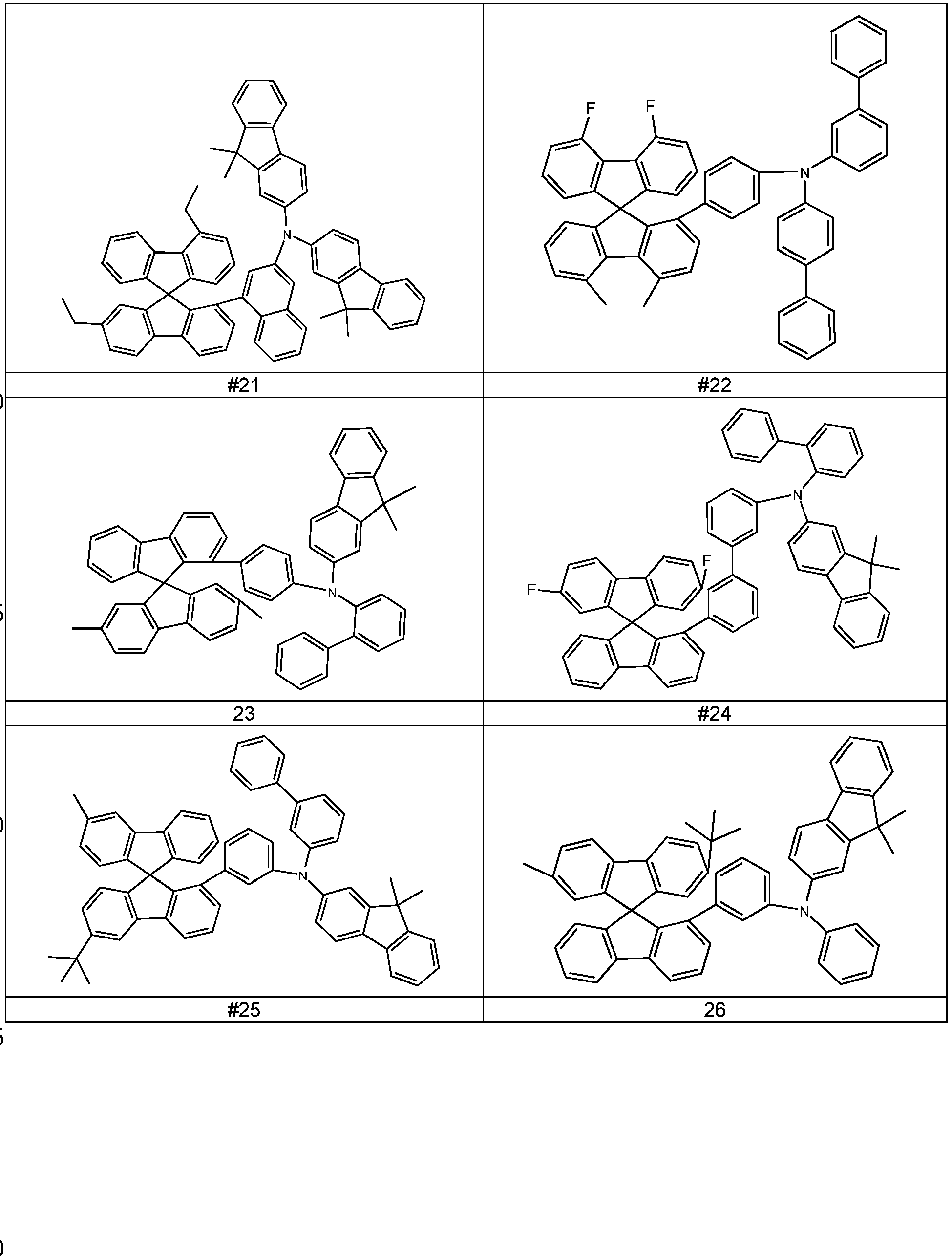

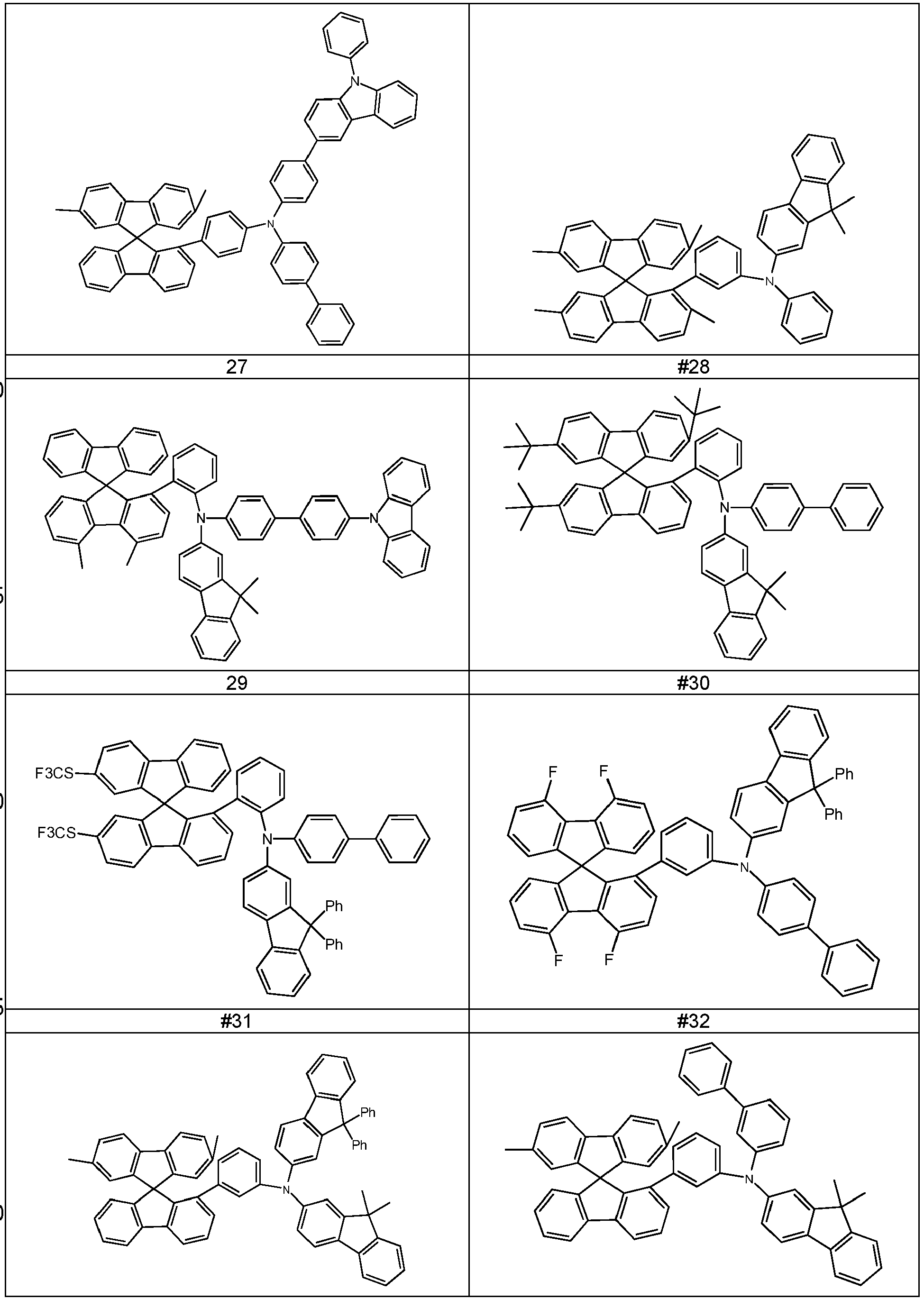

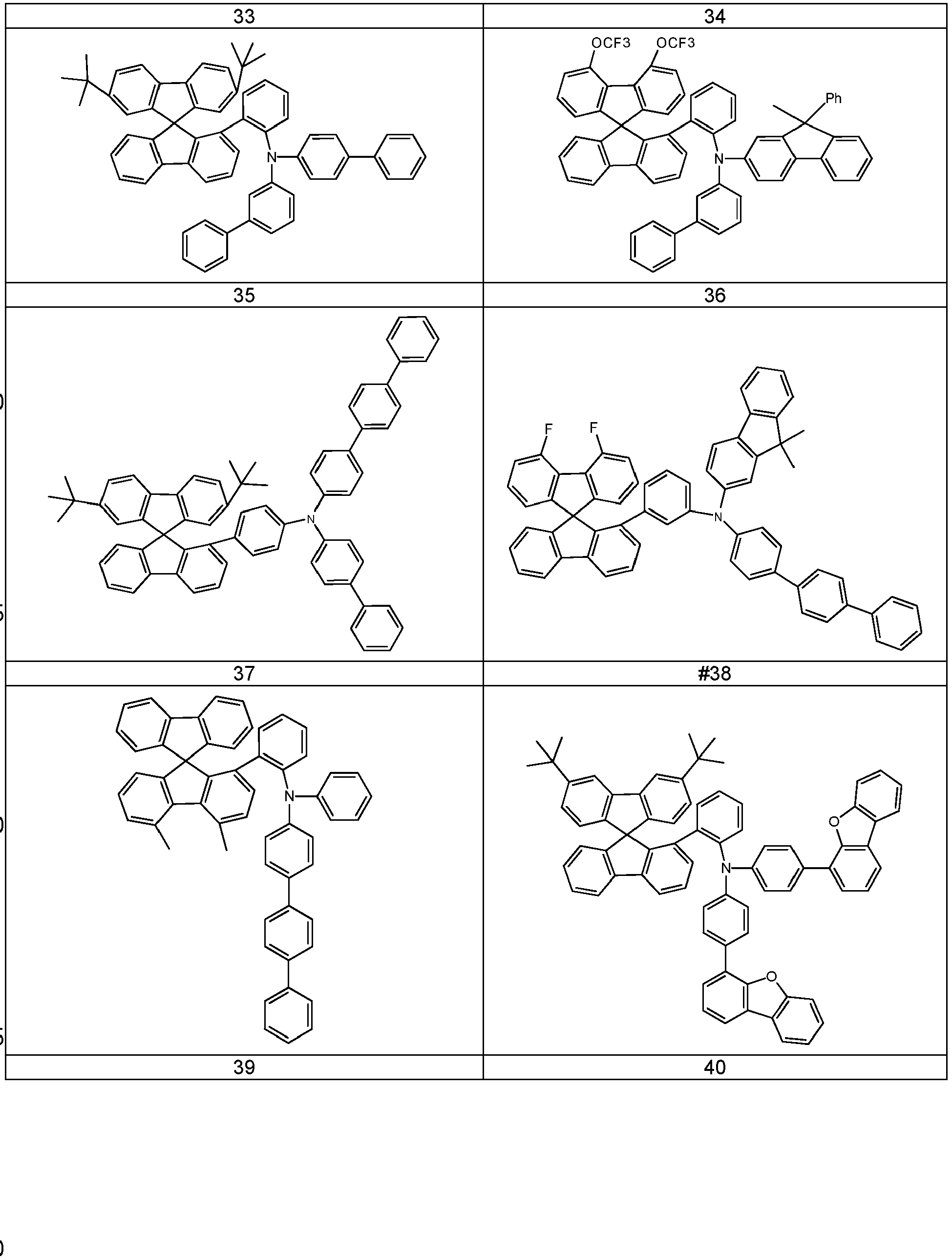

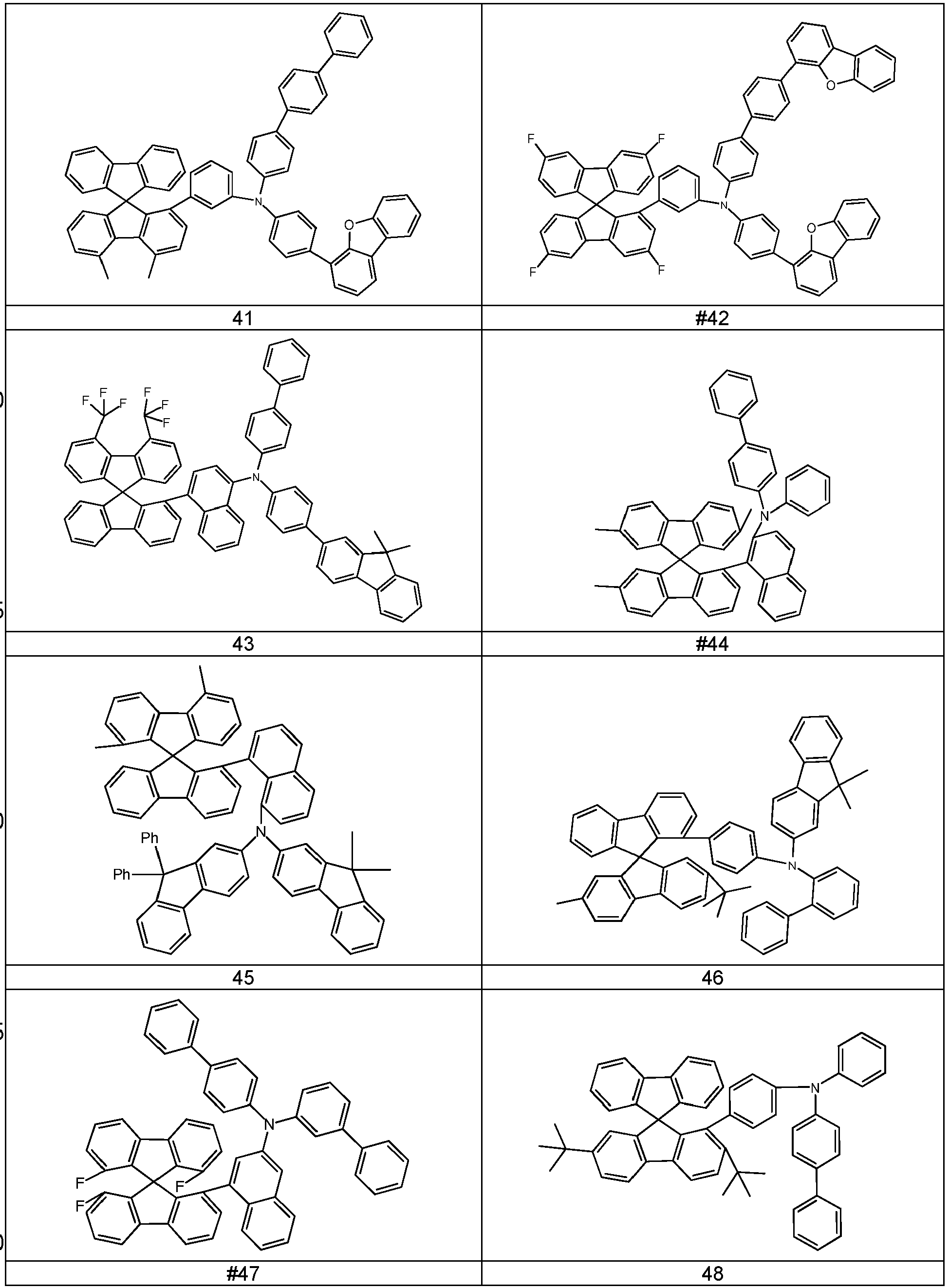

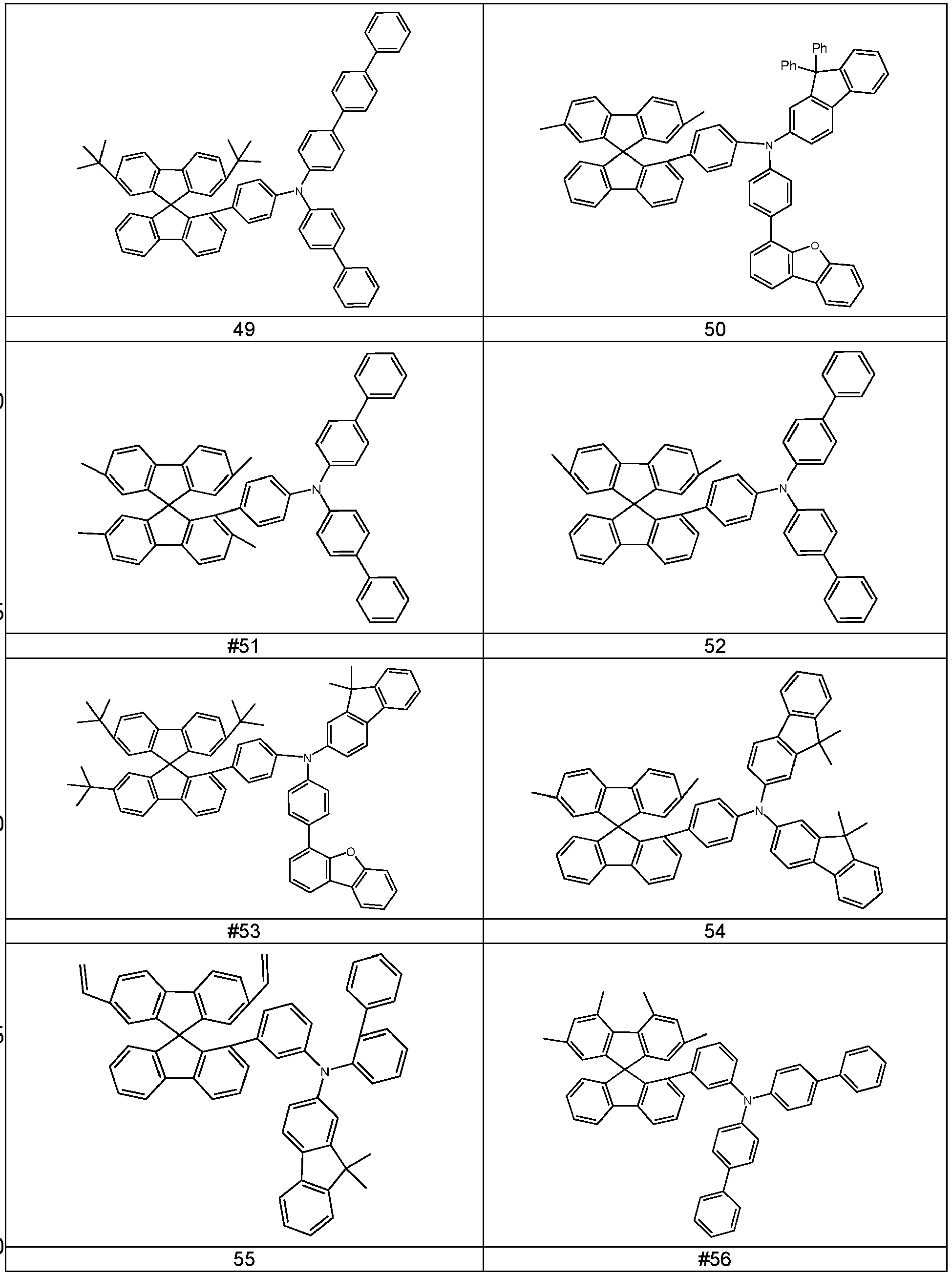

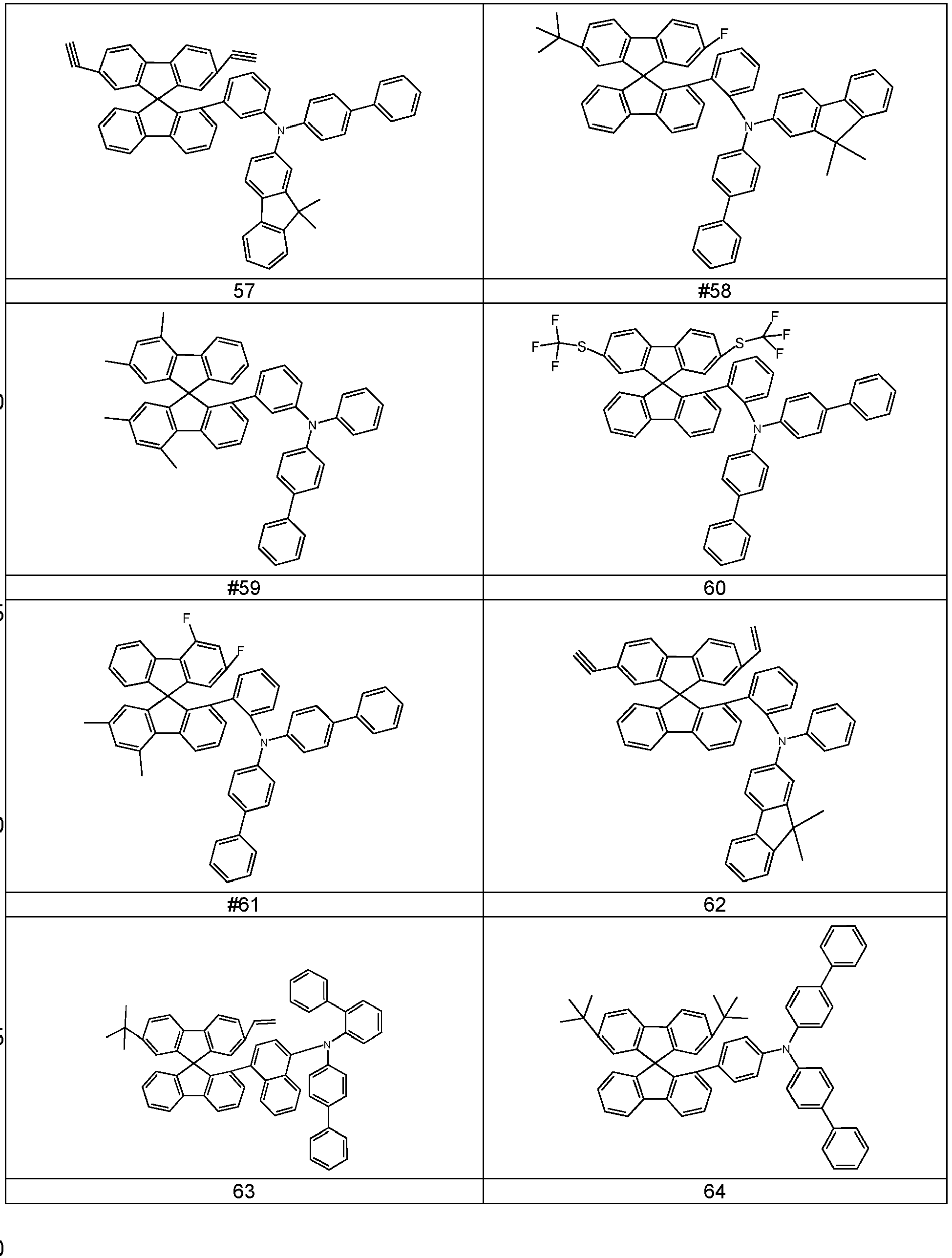

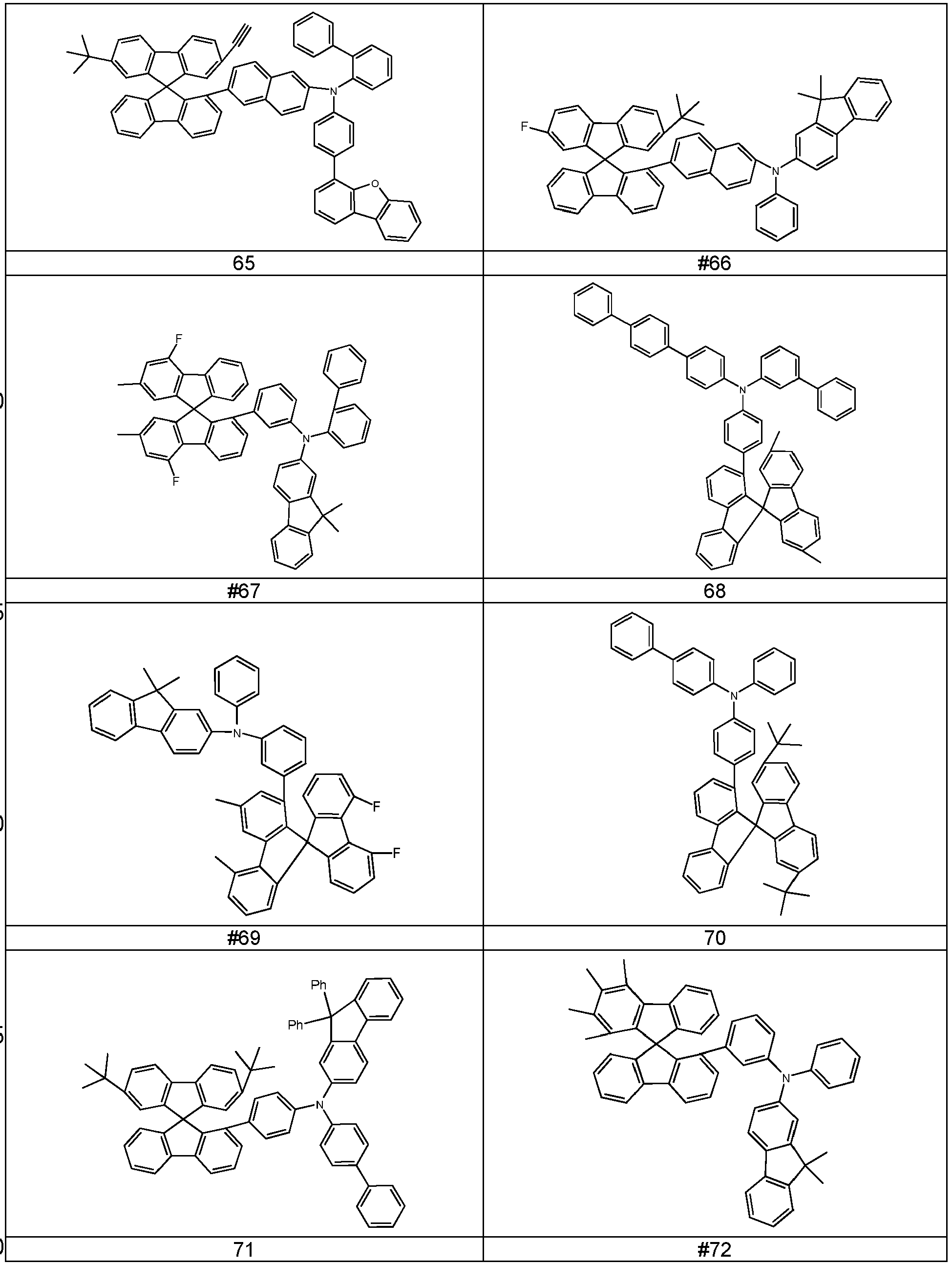

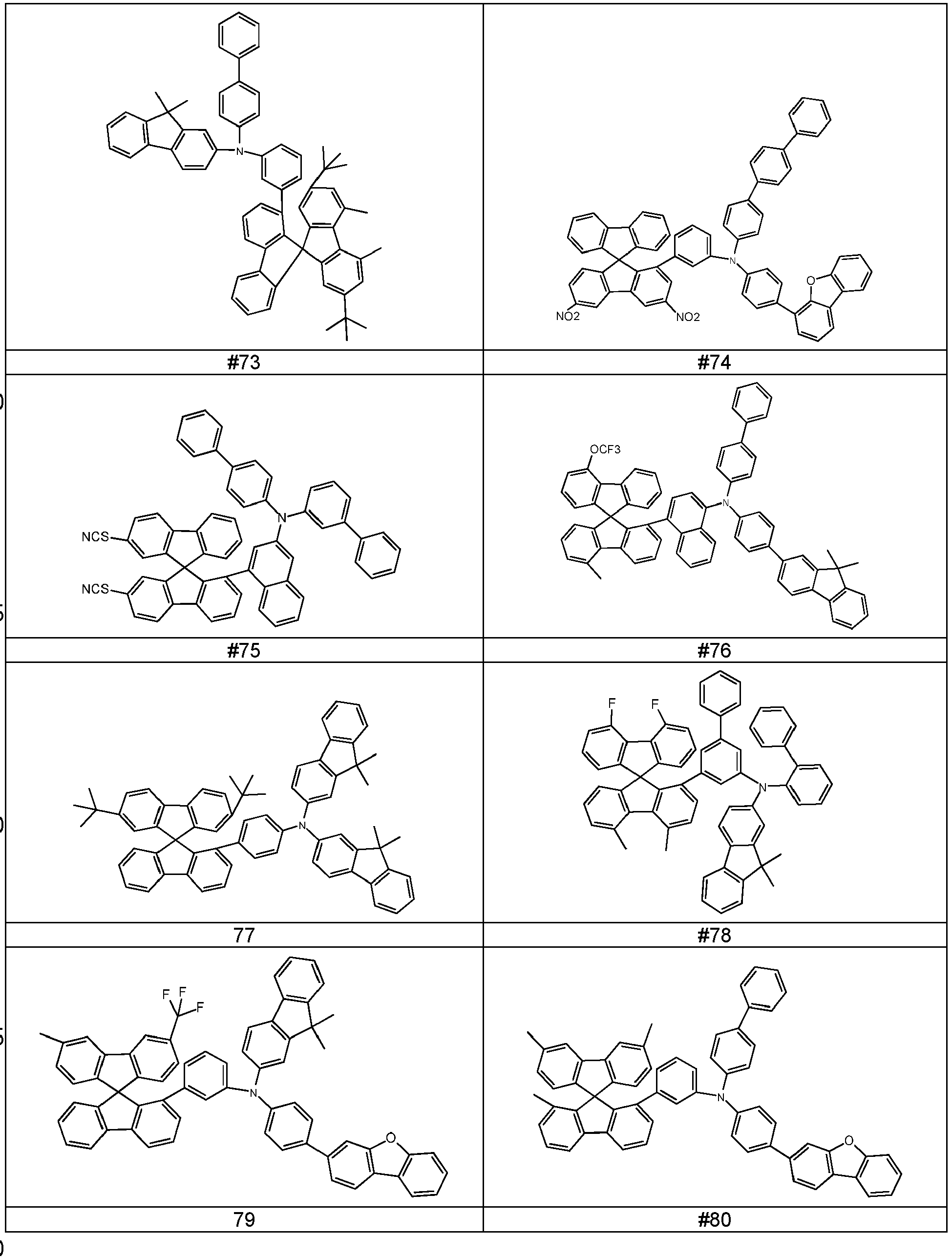

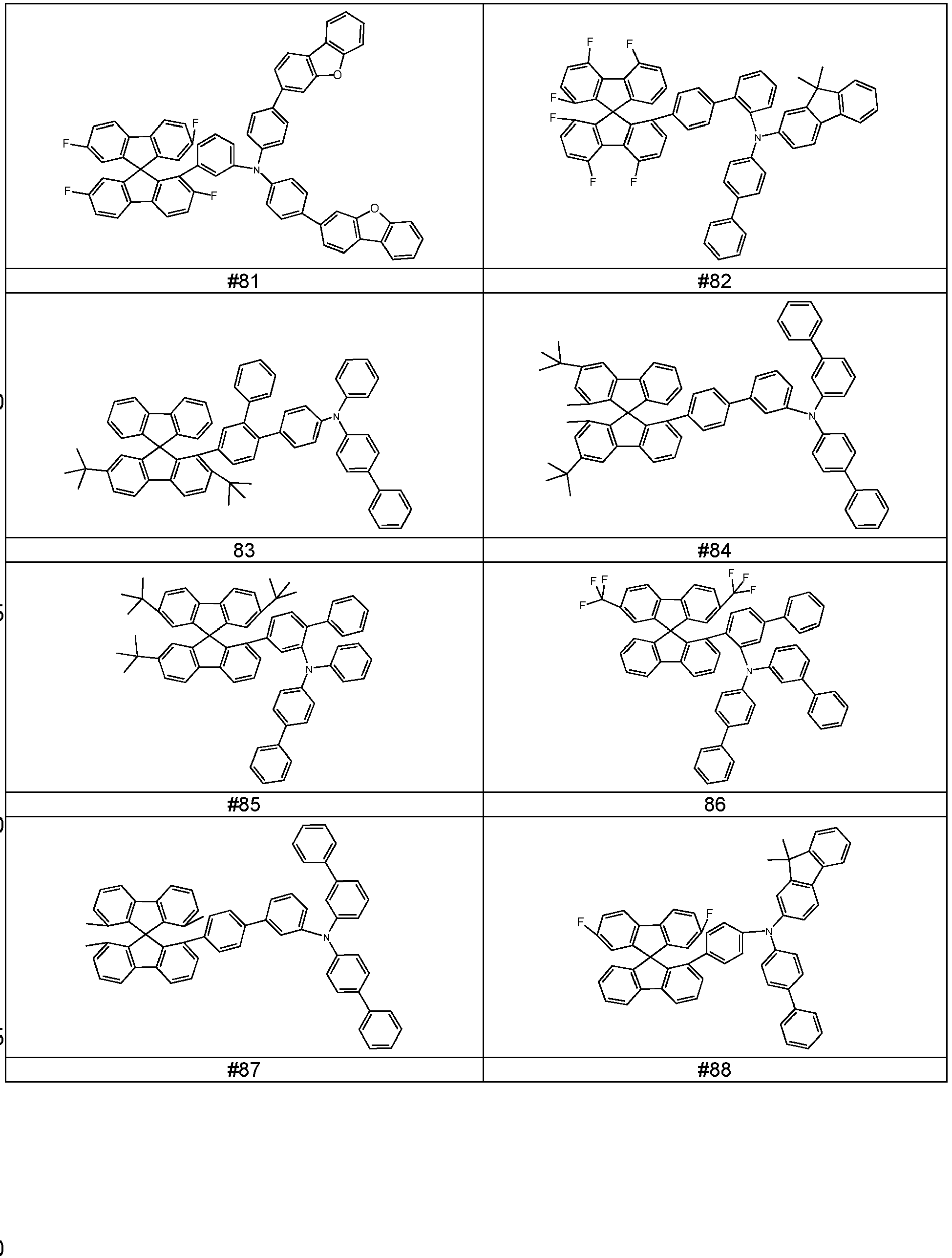

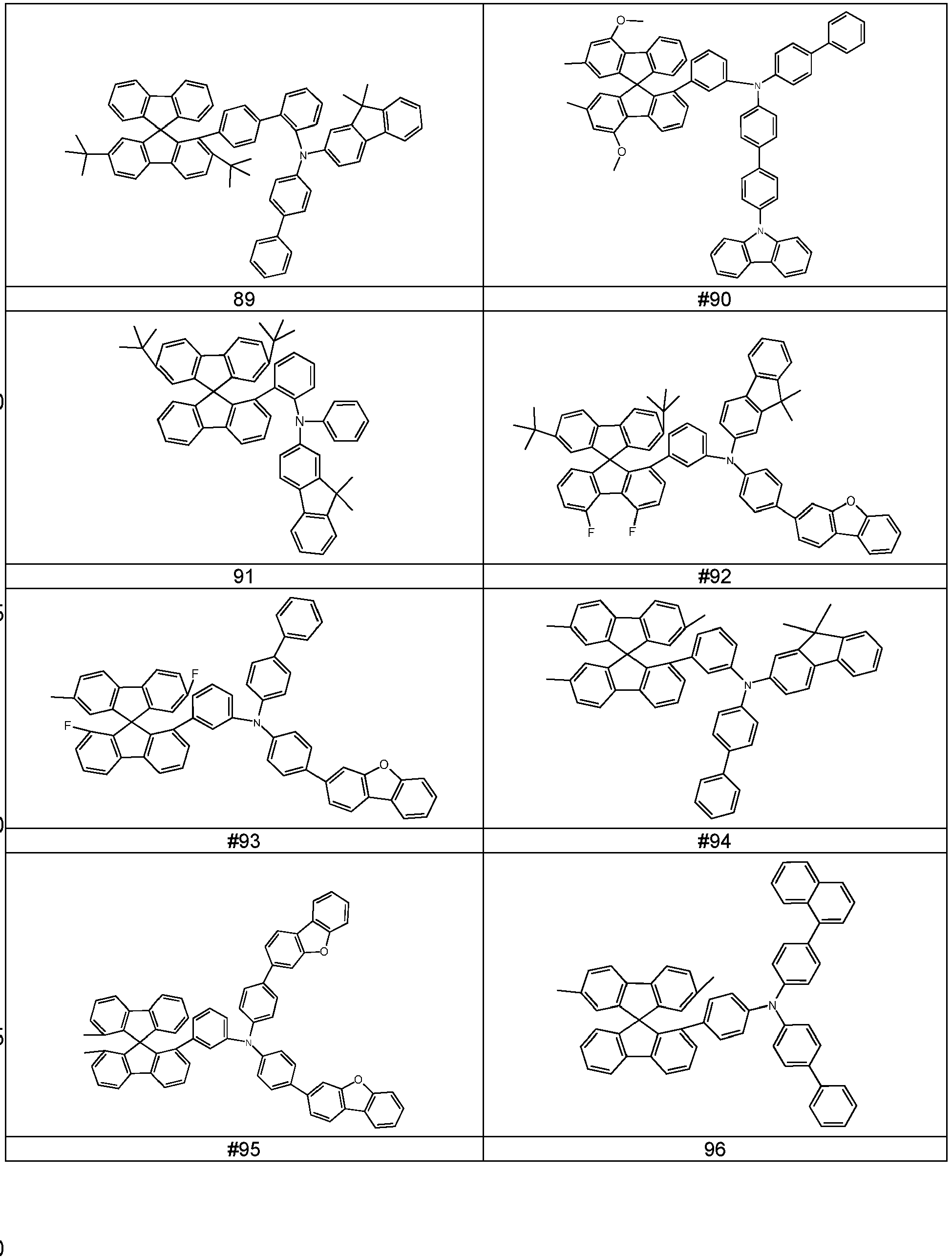

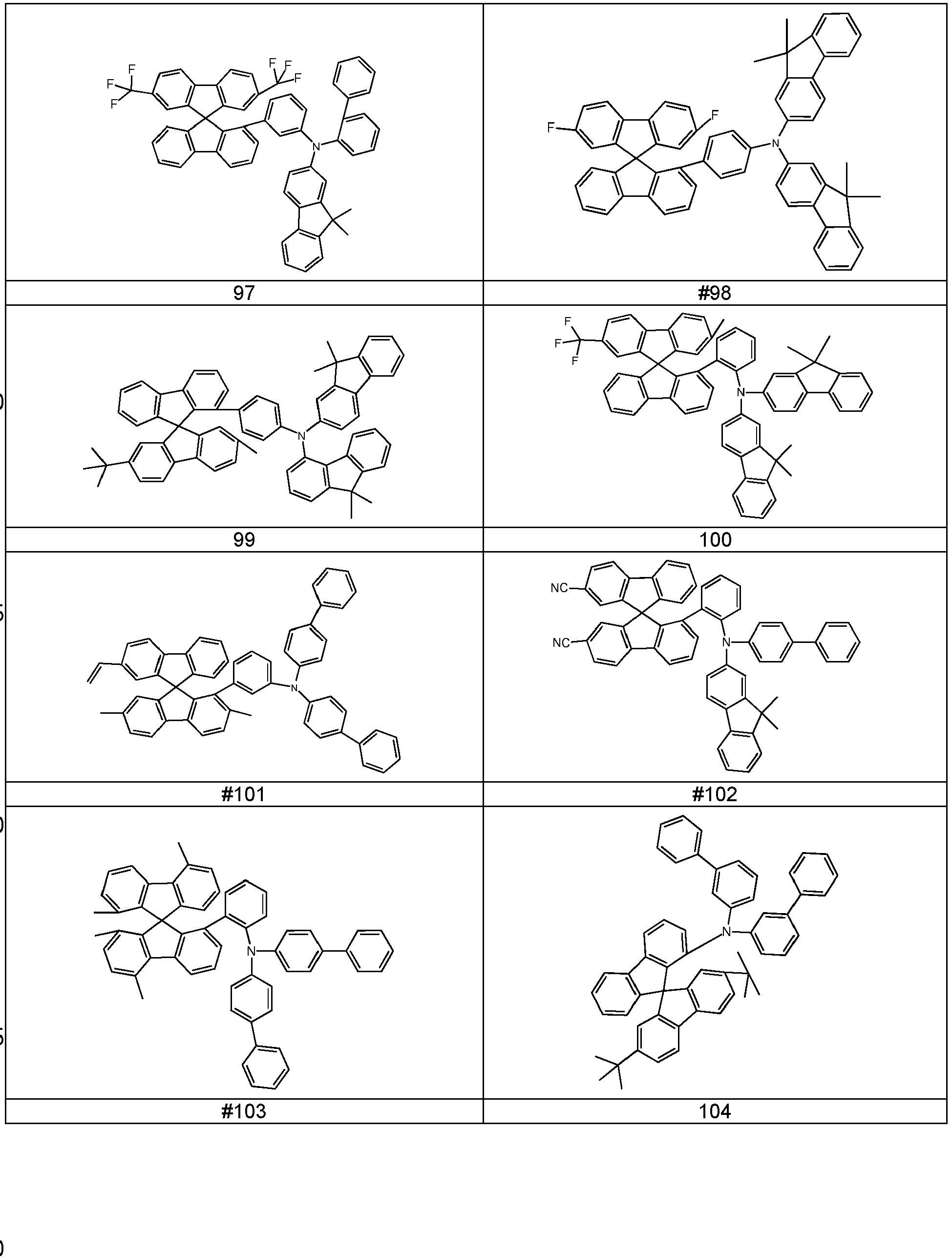

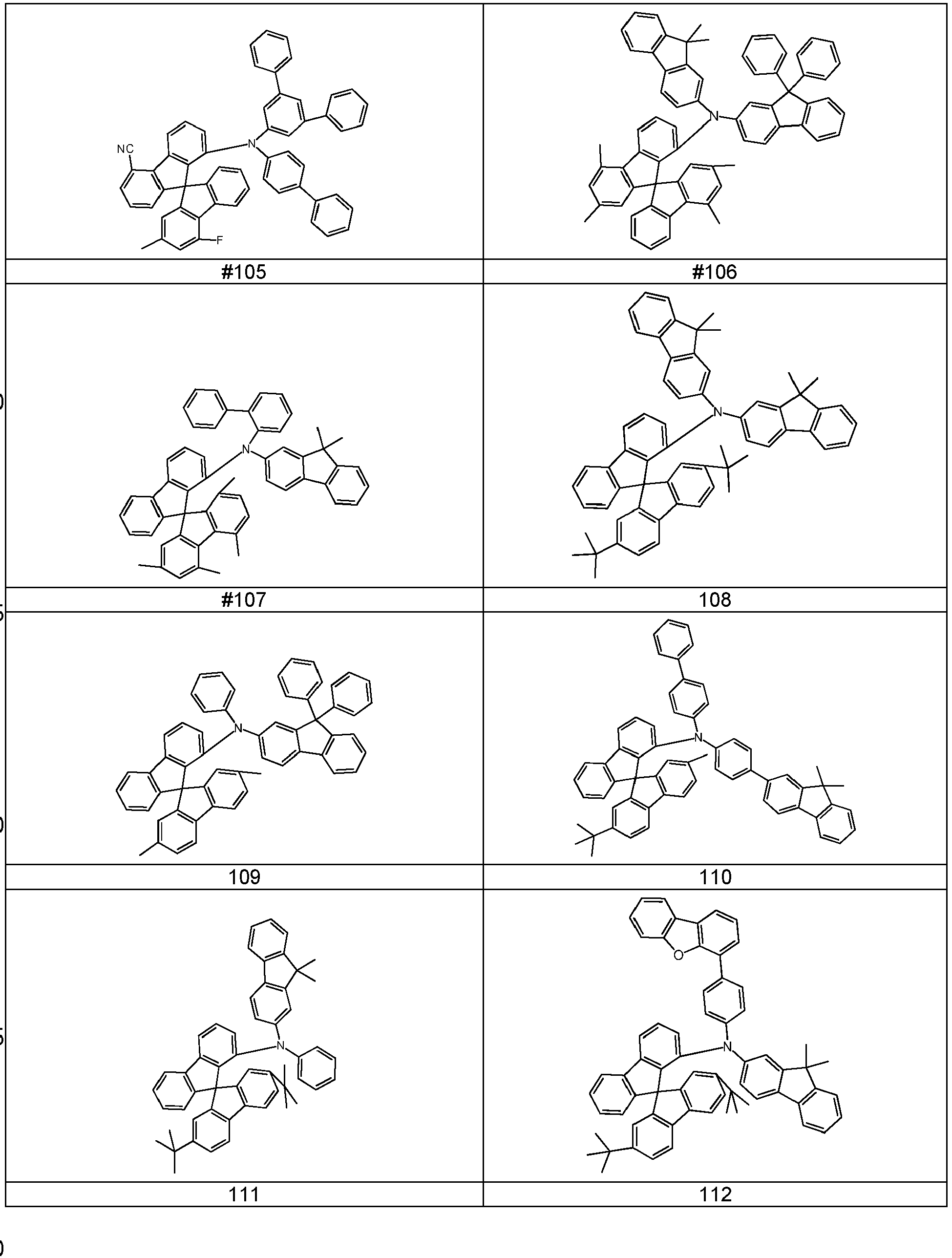

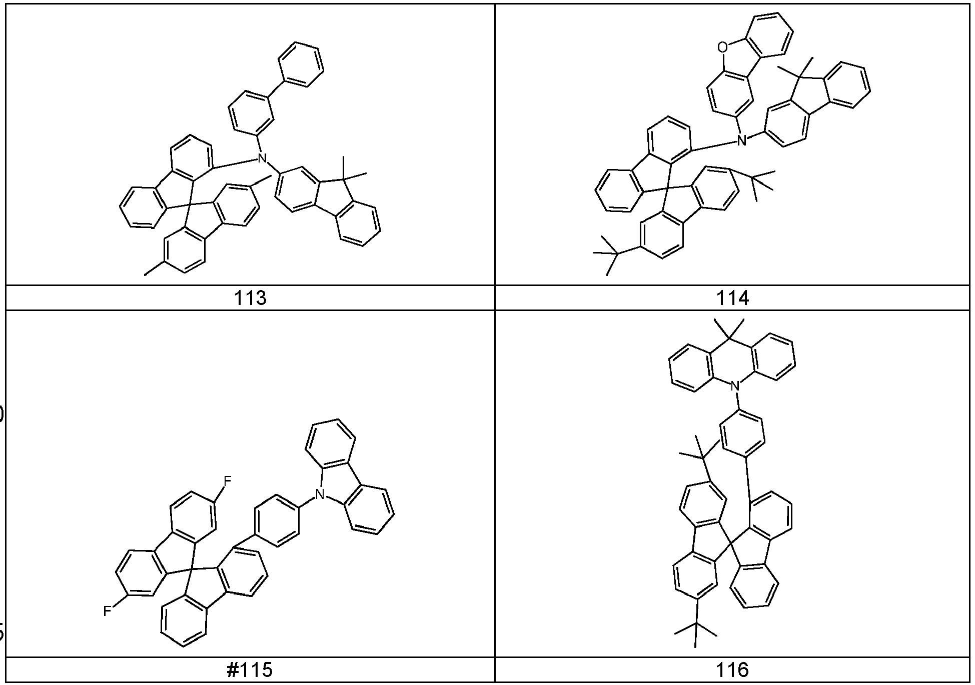

The compounds according to the present application are prepared by using standard methods known in the art of organic synthesis, such as metal catalysed coupling reactions, in particular Suzuki reactions and Buchwald reactions, nucleophilic addition reactions of metallated aryl derivatives to carbonyl groups, and acid-catalysed cyclisation reactions.

Preferably, for the synthesis of compounds according to formula (IA) or (IB), a biphenyl derivative which has a reactive group in the position ortho to the phenyl-phenyl bond is metallated, preferably lithiated or subjected to ) a Grignard reaction (see Scheme 1). The metallated biphenyl derivative is then reacted with a fluorenone derivative, which has a group A in the 1-position. The group A is selected from i) X, or ii) -Ar-X, or iii) -NAr₂, or iv) -Ar-NAr₂, where Ar is an aromatic or heteroaromatic group, and X is selected from reactive groups, preferably from halogen groups. The resulting addition product is cyclized under acidic conditions, or with a Lewis acid, to a spirobifluorene.

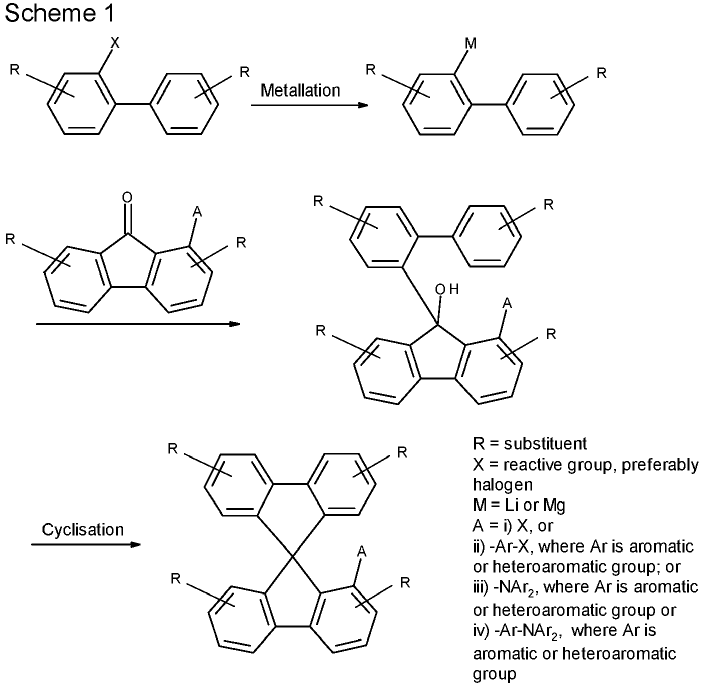

In the case i) (Group A=X), the resulting spirobifluorene can be further reacted in a Suzuki coupling with an aryl derivative which has two suitable reactive groups, and a subsequent Buchwald coupling with a diaryl amine, to give a spirobifluorene derivative which has an arylene-diarylamine group in its 1-position. As an alternative, the spirobifluorene can be reacted in a Buchwald coupling with a diaryl amine or a NH-carbazole derivative, to give a spirobifluorene derivative which has a diarylamine group or an N-carbazole group in its 1-position. As a still further alternative, the resulting spirobifluorene can be further reacted in a Suzuki coupling with a triarylamine which has a boronic acid derivative.

In the case ii) (Group A = -Ar-X), the resulting spirobifluorene can be further reacted in a Buchwald coupling with a diaryl amine or a NH-carbazole derivative, to give a spirobifluorene derivative which has a diarylamine group or an N-carbazole group in its 1-position.

In the cases iii) and iv), the spirobifluorene which results from the cyclisation reaction is already a compound according to formula (IA) or (IB). In the case iii) (Group A = -NAr₂), the fluorenone derivative which is used in the reaction sequence can be obtained from the respective halogen-substituted fluorenone derivative by Buchwald reaction with a diarylamine. In the case iv) (Group A = -Ar-NAr₂), the fluorenone derivative which is used in the reaction sequence can be obtained from the respective halogen-substituted fluorenone derivative by Suzuki coupling with an aryl derivative which has two suitable reactive groups, and a subsequent Buchwald coupling with a diaryl amine.

A further embodiment of the present invention is therefore a process for preparation of a compound according to formula (IA) or (IB), characterized in that it comprises the reactions steps
1) metallation of a biphenyl derivative which has a reactive group in a position which is ortho to the phenyl-phenyl bond;
2) addition of the metallated biphenyl derivative to a fluorenone derivative which has a group A in its 1-position; where the group A is selected from i) X, or ii) -Ar-X, or iii) -NAr₂, or iv) -Ar-NAr₂, where Ar is aromatic or heteroaromatic group, and where X is a reactive group; and
3) cyclisation of the resulting addition product to a spirobifluorene derivative under acidic conditions or with a Lewis acid.

The metallation of step 1) is preferably a lithiation or a Grignard reaction. Group X is preferably a halogen group, more preferably Cl or Br. Steps 1) to 3) are preferably carried out in their numeric sequence. Furthermore, preferably, step 2) is carried out directly after step 1), and step 3) is carried out directly after step 3). "Directly" means in this regard that no chemical reactions are carried out in between the reaction steps.

The above-described compounds, especially compounds substituted by reactive leaving groups, such as bromine, iodine, chlorine, boronic acid or boronic ester, may find use as monomers for production of corresponding oligomers, dendrimers or polymers. Suitable reactive leaving groups are, for example, bromine, iodine, chlorine, boronic acids, boronic esters, amines, alkenyl or alkynyl groups having a terminal C-C double bond or C-C triple bond, oxiranes, oxetanes, groups which enter into a cycloaddition, for example a 1,3-dipolar cycloaddition, for example dienes or azides, carboxylic acid derivatives, alcohols and silanes.

The invention therefore further provides oligomers, polymers or dendrimers containing one or more compounds of formula (IA) or (IB), wherein the bond(s) to the polymer, oligomer or dendrimer may be localized at any desired positions substituted by R¹ or R³ in formula (IA) or (IB). According to the linkage of the compound of formula (IA) or (IB), the compound is part of a side chain of the oligomer or polymer or part of the main chain. An oligomer in the context of this invention is understood to mean a compound formed from at least three monomer units. A polymer in the context of the invention is understood to mean a compound formed from at least ten monomer units. The polymers, oligomers or dendrimers of the invention may be conjugated, partly conjugated or nonconjugated. The oligomers or polymers of the invention may be linear, branched or dendritic. In the structures having linear linkage, the units of formula (IA) or (IB) may be joined directly to one another, or they may be joined to one another via a bivalent group, for example via a substituted or unsubstituted alkylene group, via a heteroatom or via a bivalent aromatic or heteroaromatic group. In branched and dendritic structures, it is possible, for example, for three or more units of formula (IA) or (IB) to be joined via a trivalent or higher-valency group, for example via a trivalent or higher-valency aromatic or heteroaromatic group, to give a branched or dendritic oligomer or polymer.

For the repeat units of formula (IA) or (IB) in oligomers, dendrimers and polymers, the same preferences apply as described above for compounds of formula (IA) or (IB).

For preparation of the oligomers or polymers, the monomers of the invention are homopolymerized or copolymerized with further monomers. Suitable and preferred comonomers are chosen from fluorenes (for example according to EP 842208 or WO 2000/22026), spirobifluorenes (for example according to EP 707020, EP 894107 or WO 2006/061181), paraphenylenes (for example according to WO 1992/18552), carbazoles (for example according to WO 2004/070772 or WO 2004/113468), thiophenes (for example according to EP 1028136), dihydrophenanthrenes (for example according to WO 2005/014689 or WO 2007/006383), cis- and trans-indenofluorenes (for example according to WO 2004/041901 or WO 2004/113412), ketones (for example according to WO 2005/040302), phenanthrenes (for example according to WO 2005/104264 or WO 2007/017066) or else a plurality of these units. The polymers, oligomers and dendrimers typically contain still further units, for example emitting (fluorescent or phosphorescent) units, for example vinyltriarylamines (for example according to WO 2007/068325) or phosphorescent metal complexes (for example according to WO 2006/003000), and/or charge transport units, especially those based on triarylamines.

The polymers and oligomers of the invention are generally prepared by polymerization of one or more monomer types, of which at least one monomer leads to repeat units of the formula (IA) or (IB) in the polymer. Suitable polymerization reactions are known to those skilled in the art and are described in the literature. Particularly suitable and preferred polymerization reactions which lead to formation of C-C or C-N bonds are the Suzuki polymerization, the Yamamoto polymerization, the Stille polymerization and the Hartwig-Buchwald polymerization.

For the processing of the compounds of the invention from a liquid phase, for example by spin-coating or by printing methods, formulations of the compounds of the invention are required. These formulations may, for example, be solutions, dispersions or emulsions. For this purpose, it may be preferable to use mixtures of two or more solvents. Suitable and preferred solvents are, for example, toluene, anisole, o-, m- or p-xylene, methyl benzoate, mesitylene, tetralin, veratrole, THF, methyl-THF, THP, chlorobenzene, dioxane, phenoxytoluene, especially 3-phenoxytoluene, (-)-fenchone, 1,2,3,5-tetramethylbenzene, 1,2,4,5-tetramethylbenzene, 1-methylnaphthalene, 2-methylbenzothiazole, 2-phenoxyethanol, 2-pyrrolidinone, 3-methylanisole, 4-methylanisole, 3,4-dimethylanisole, 3,5-dimethylanisole, acetophenone, α-terpineol, benzothiazole, butyl benzoate, cumene, cyclohexanol, cyclohexanone, cyclohexylbenzene, decalin, dodecylbenzene, ethyl benzoate, indane, methyl benzoate, NMP, p-cymene, phenetole, 1,4-diisopropylbenzene, dibenzyl ether, diethylene glycol butyl methyl ether, triethylene glycol butyl methyl ether, diethylene glycol dibutyl ether, triethylene glycol dimethyl ether, diethylene glycol monobutyl ether, tripropylene glycol dimethyl ether, tetraethylene glycol dimethyl ether, 2-isopropylnaphthalene, pentylbenzene, hexylbenzene, heptylbenzene, octylbenzene, 1,1-bis(3,4-dimethylphenyl)ethane or mixtures of these solvents.

The invention therefore further provides a formulation, especially a solution, dispersion or emulsion, comprising at least one compound of formula (IA) or (IB) and at least one solvent, preferably an organic solvent. The way in which such solutions can be prepared is known to those skilled in the art and is described, for example, in WO 2002/072714, WO 2003/019694 and the literature cited therein.

The compounds of the invention are suitable for use in electronic devices, especially in organic electroluminescent devices (OLEDs). Depending on the substitution, the compounds are used in different functions and layers.

The invention therefore further provides for the use of the compound of formula (IA) or (IB) in an electronic device. This electronic device is preferably selected from the group consisting of organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic fieldquench devices (OFQDs), organic light-emitting electrochemical cells (OLECs), organic laser diodes (O-lasers) and more preferably organic electroluminescent devices (OLEDs).

The invention further provides, as already set out above, an electronic device comprising at least one compound of formula (IA) or (IB). This electronic device is preferably selected from the abovementioned devices.

It is more preferably an organic electroluminescent device (OLED) comprising anode, cathode and at least one emitting layer, characterized in that at least one organic layer, which may be an emitting layer, a hole transport layer or another layer, preferably an emitting layer or a hole transport layer, particularly preferably a hole transport layer, comprises at least one compound of formula (IA) or (IB).

Apart from the cathode, anode and emitting layer, the organic electroluminescent device may also comprise further layers. These are selected, for example, from in each case one or more hole injection layers, hole transport layers, hole blocking layers, electron transport layers, electron injection layers, electron blocking layers, exciton blocking layers, interlayers, charge generation layers (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) and/or organic or inorganic p/n junctions.

The sequence of the layers of the organic electroluminescent device comprising the compound of the formula (IA) or (IB) is preferably as follows: anode-hole injection layer-hole transport layer-optionally further hole transport layer(s)-optionally electron blocking layer-emitting layer-optionally hole blocking layer-electron transport layer-electron injection layer-cathode. It is additionally possible for further layers to be present in the OLED.

The organic electroluminescent device of the invention may contain two or more emitting layers. More preferably, these emission layers in this case have several emission maxima between 380 nm and 750 nm overall, such that the overall result is white emission; in other words, various emitting compounds which may fluoresce or phosphoresce and which emit blue, green, yellow, orange or red light are used in the emitting layers. Especially preferred are three-layer systems, i.e. systems having three emitting layers, where the three layers show blue, green and orange or red emission (for the basic construction see, for example, WO 2005/011013). The compounds of the invention are preferably present in the hole transport layer, hole injection layer or electron blocking layer.

It is preferable in accordance with the invention when the compound of formula (IA) or (IB) is used in an electronic device comprising one or more phosphorescent emitting compounds. In this case, the compound may be present in different layers, preferably in a hole transport layer, an electron blocking layer, a hole injection layer or in an emitting layer.

The term "phosphorescent emitting compounds" typically encompasses compounds where the emission of light is effected through a spin-forbidden transition, for example a transition from an excited triplet state or a state having a higher spin quantum number, for example a quintet state.

Suitable phosphorescent emitting compounds (= triplet emitters) are especially compounds which, when suitably excited, emit light, preferably in the visible region, and also contain at least one atom of atomic number greater than 20, preferably greater than 38, and less than 84, more preferably greater than 56 and less than 80. Preference is given to using, as phosphorescent emitting compounds, compounds containing copper, molybdenum, tungsten, rhenium, ruthenium, osmium, rhodium, iridium, palladium, platinum, silver, gold or europium, especially compounds containing iridium, platinum or copper. In the context of the present invention, all luminescent iridium, platinum or copper complexes are considered to be phosphorescent emitting compounds.

Examples of the above-described emitting compounds can be found in applications WO 00/70655, WO 01/41512, WO 02/02714, WO 02/15645, EP 1191613, EP 1191612, EP 1191614, WO 05/033244, WO 05/019373 and US 2005/0258742. In general, all phosphorescent complexes as used for phosphorescent OLEDs according to the prior art and as known to those skilled in the art in the field of organic electroluminescent devices are suitable. It is also possible for the person skilled in the art, without exercising inventive skill, to use further phosphorescent complexes in combination with the compounds of formula (IA) or (IB) in organic electroluminescent devices. Further examples are listed in a table which follows.

It is also possible in accordance with the invention to use the compound of formula (IA) or (IB) in an electronic device comprising one or more fluorescent emitting compounds.

In a preferred embodiment of the invention, the compounds of formula (IA) or (IB) are used as hole-transporting material. In that case, the compounds are preferably present in a hole transport layer, an electron blocking layer or a hole injection layer. Particular preference is given to use in an electron blocking layer.

A hole transport layer according to the present application is a layer having a hole-transporting function between the anode and emitting layer.

Hole injection layers and electron blocking layers are understood in the context of the present application to be specific embodiments of hole transport layers. A hole injection layer, in the case of a plurality of hole transport layers between the anode and emitting layer, is a hole transport layer which directly adjoins the anode or is separated therefrom only by a single coating of the anode. An electron blocking layer, in the case of a plurality of hole transport layers between the anode and emitting layer, is that hole transport layer which directly adjoins the emitting layer on the anode side. Preferably, the OLED of the invention comprises two, three or four hole-transporting layers between the anode and emitting layer, at least one of which preferably contains a compound of formula (IA) or (IB), and more preferably exactly one or two contain a compound of formula (IA) or (IB).

If the compound of formula (IA) or (IB) is used as hole transport material in a hole transport layer, a hole injection layer or an electron blocking layer, the compound can be used as pure material, i.e. in a proportion of 100%, in the hole transport layer, or it can be used in combination with one or more further compounds. In a preferred embodiment, the organic layer comprising the compound of the formula (IA) or (IB) then additionally contains one or more p-dopants. p-Dopants used according to the present invention are preferably those organic electron acceptor compounds capable of oxidizing one or more of the other compounds in the mixture.

Particularly preferred embodiments of p-dopants are the compounds disclosed in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600, WO 2012/095143 and DE 102012209523.

Particularly preferred p-dopants are quinodimethane compounds, azaindenofluorenediones, azaphenalenes, azatriphenylenes, I₂, metal halides, preferably transition metal halides, metal oxides, preferably metal oxides containing at least one transition metal or a metal of main group 3, and transition metal complexes, preferably complexes of Cu, Co, Ni, Pd and Pt with ligands containing at least one oxygen atom as bonding site. Preference is further given to transition metal oxides as dopants, preferably oxides of rhenium, molybdenum and tungsten, more preferably Re₂O₇, MoO₃, WO₃ and ReO₃.

The p-dopants are preferably in substantially homogeneous distribution in the p-doped layers. This can be achieved, for example, by coevaporation of the p-dopant and the hole transport material matrix.

Preferred p-dopants are especially the following compounds:

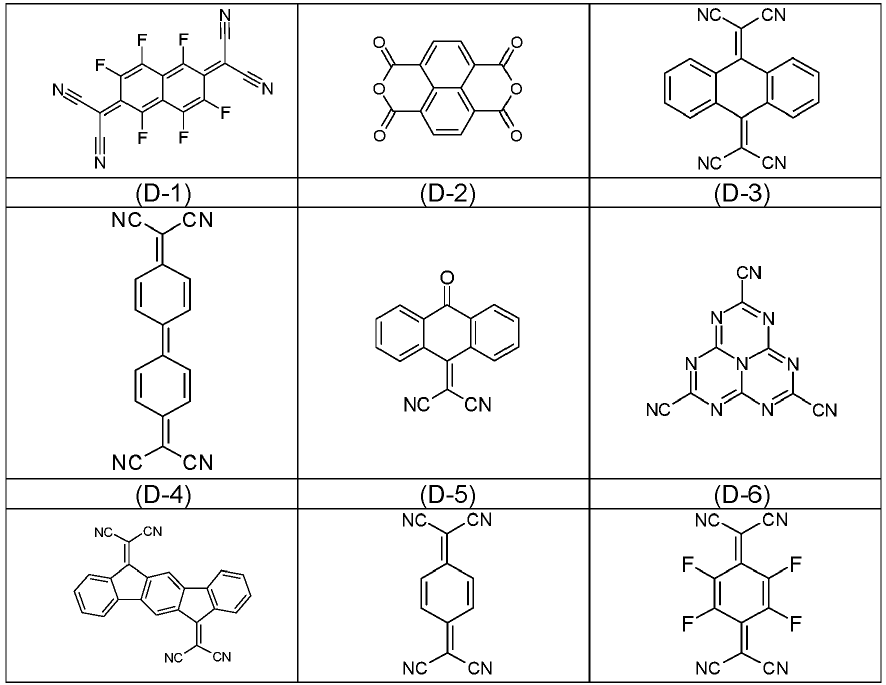

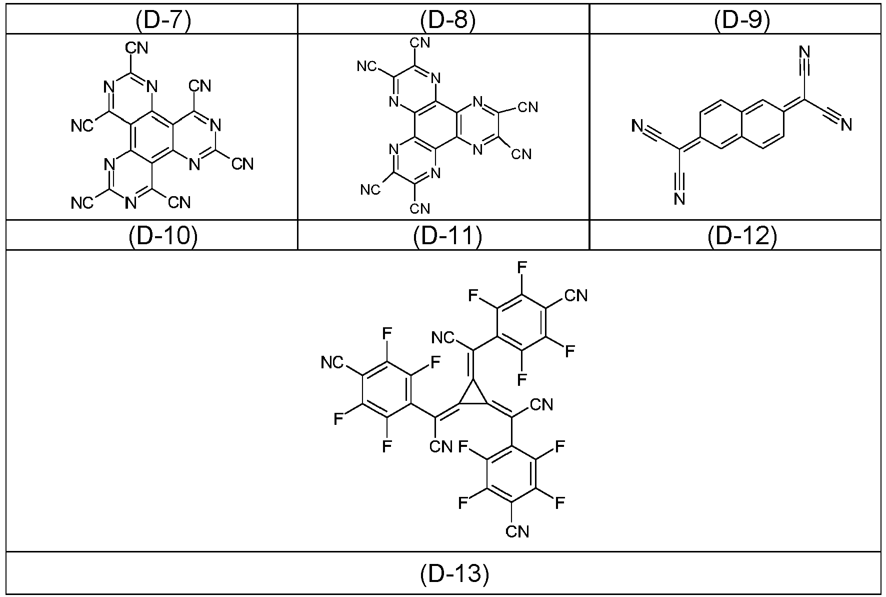

In a further preferred embodiment of the invention, the compound of formula (IA) or (IB) is used as hole transport material in combination with a hexaazatriphenylene derivative as described in US 2007/0092755. Particular preference is given here to using the hexaazatriphenylene derivative in a separate layer.

In a further embodiment of the present invention, the compound of the formula (IA) or (IB) is used in an emitting layer as matrix material in combination with one or more emitting compounds, preferably phosphorescent emitting compounds.

The proportion of the matrix material in the emitting layer in this case is between 50.0% and 99.9% by volume, preferably between 80.0% and 99.5% by volume, and more preferably between 92.0% and 99.5% by volume for fluorescent emitting layers and between 85.0% and 97.0% by volume for phosphorescent emitting layers.

Correspondingly, the proportion of the emitting compound is between 0.1% and 50.0% by volume, preferably between 0.5% and 20.0% by volume, and more preferably between 0.5% and 8.0% by volume for fluorescent emitting layers and between 3.0% and 15.0% by volume for phosphorescent emitting layers.

An emitting layer of an organic electroluminescent device may also comprise systems comprising a plurality of matrix materials (mixed matrix systems) and/or a plurality of emitting compounds. In this case too, the emitting compounds are generally those compounds having the smaller proportion in the system and the matrix materials are those compounds having the greater proportion in the system. In individual cases, however, the proportion of a single matrix material in the system may be less than the proportion of a single emitting compound.

It is preferable that the compounds of formula (IA) or (IB) are used as a component of mixed matrix systems. The mixed matrix systems preferably comprise two or three different matrix materials, more preferably two different matrix materials. Preferably, in this case, one of the two materials is a material having hole-transporting properties and the other material is a material having electron-transporting properties. The compound of the formula (IA) or (IB) is preferably the matrix material having hole-transporting properties. The desired electron-transporting and hole-transporting properties of the mixed matrix components may, however, also be combined mainly or entirely in a single mixed matrix component, in which case the further mixed matrix component(s) fulfill(s) other functions. The two different matrix materials may be present in a ratio of 1:50 to 1:1, preferably 1:20 to 1:1, more preferably 1:10 to 1:1 and most preferably 1:4 to 1:1. Preference is given to using mixed matrix systems in phosphorescent organic electroluminescent devices. One source of more detailed information about mixed matrix systems is the application WO 2010/108579.

The mixed matrix systems may comprise one or more emitting compounds, preferably one or more phosphorescent emitting compounds. In general, mixed matrix systems are preferably used in phosphorescent organic electroluminescent devices.

Particularly suitable matrix materials which can be used in combination with the compounds of the invention as matrix components of a mixed matrix system are selected from the preferred matrix materials specified below for phosphorescent emitting compounds or the preferred matrix materials for fluorescent emitting compounds, according to what type of emitting compound is used in the mixed matrix system.

Preferred phosphorescent emitting compounds for use in mixed matrix systems are the same as detailed further up as generally preferred phosphorescent emitter materials.

Preferred embodiments of the different functional materials in the electronic device are listed hereinafter.

Preferred phosphorescent emitting compounds are the following ones:

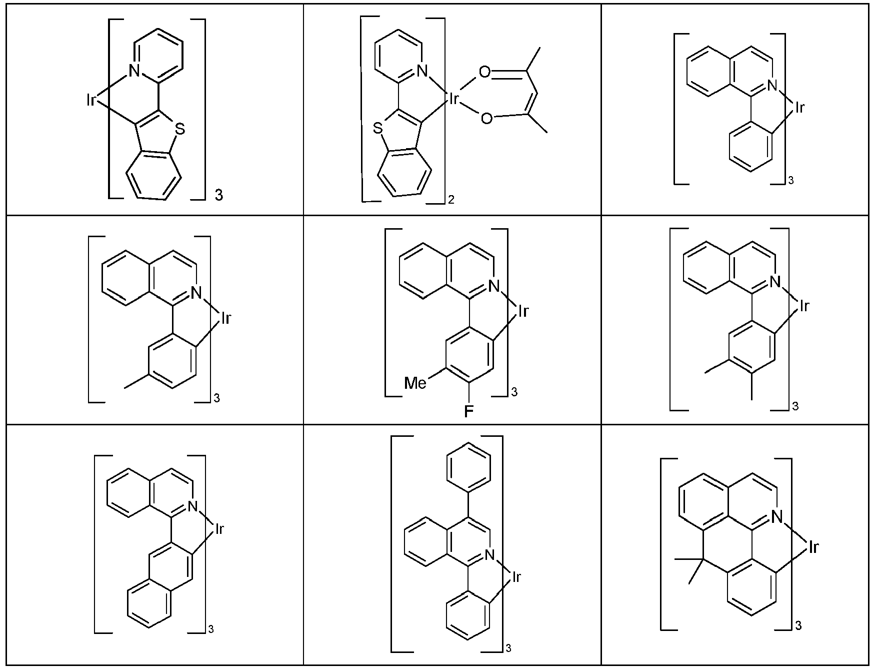

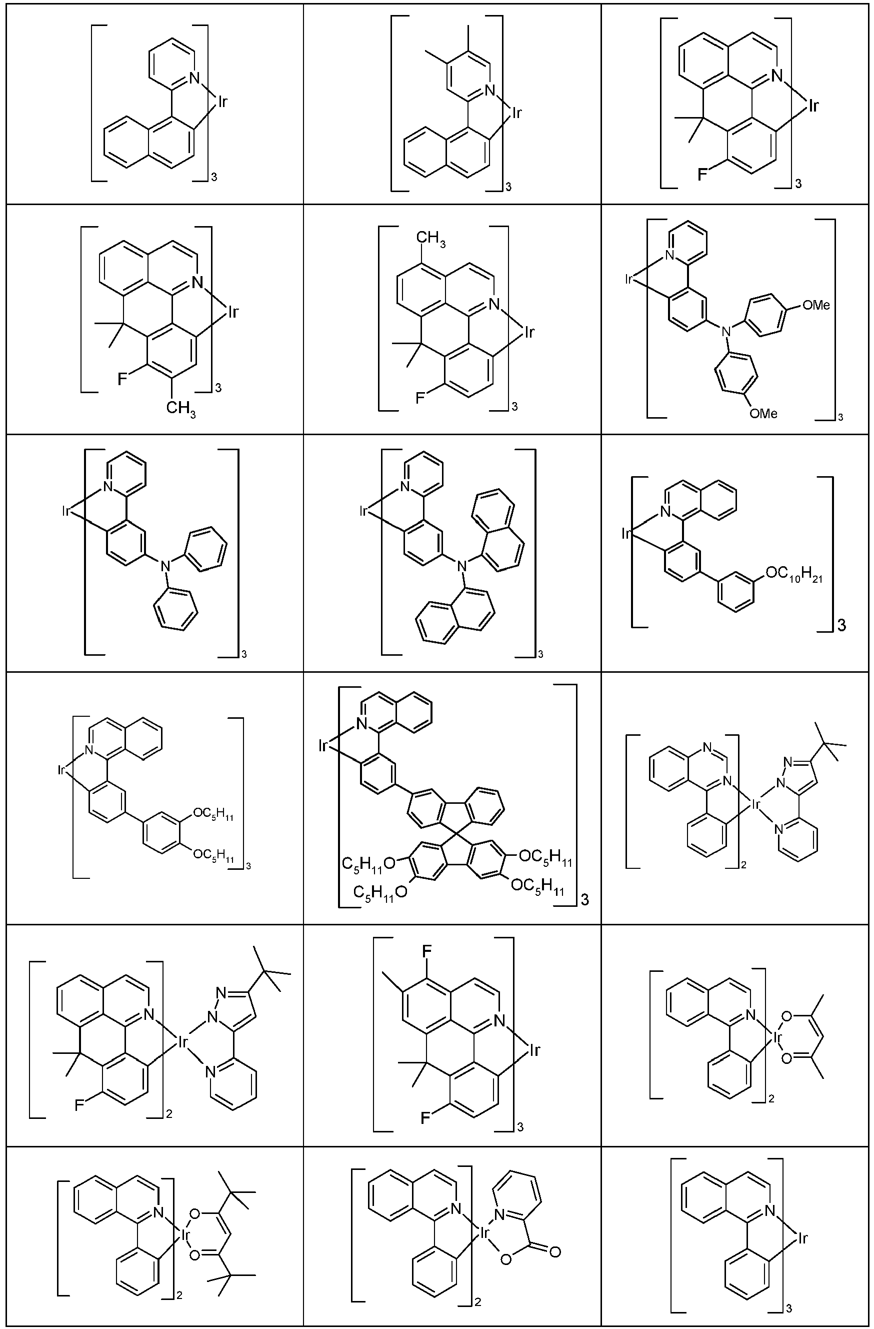

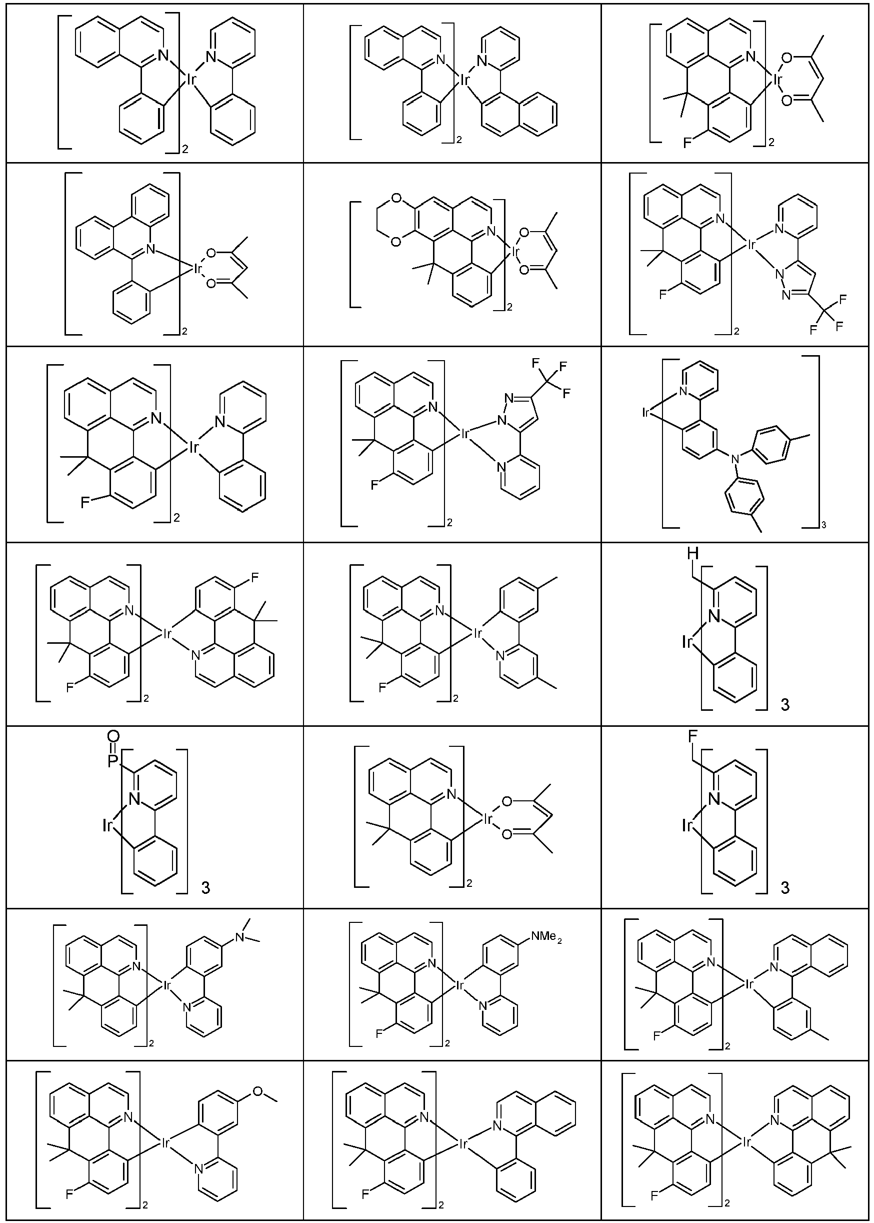

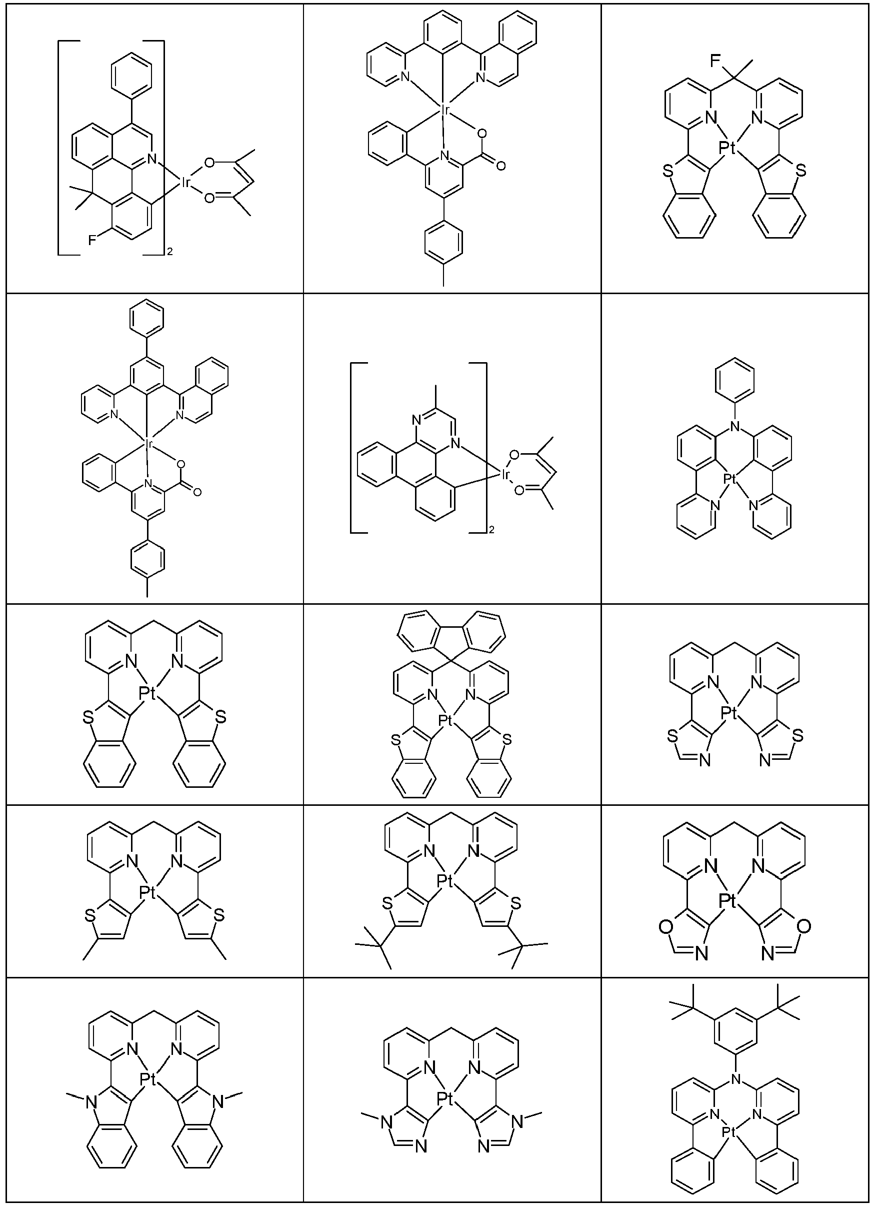

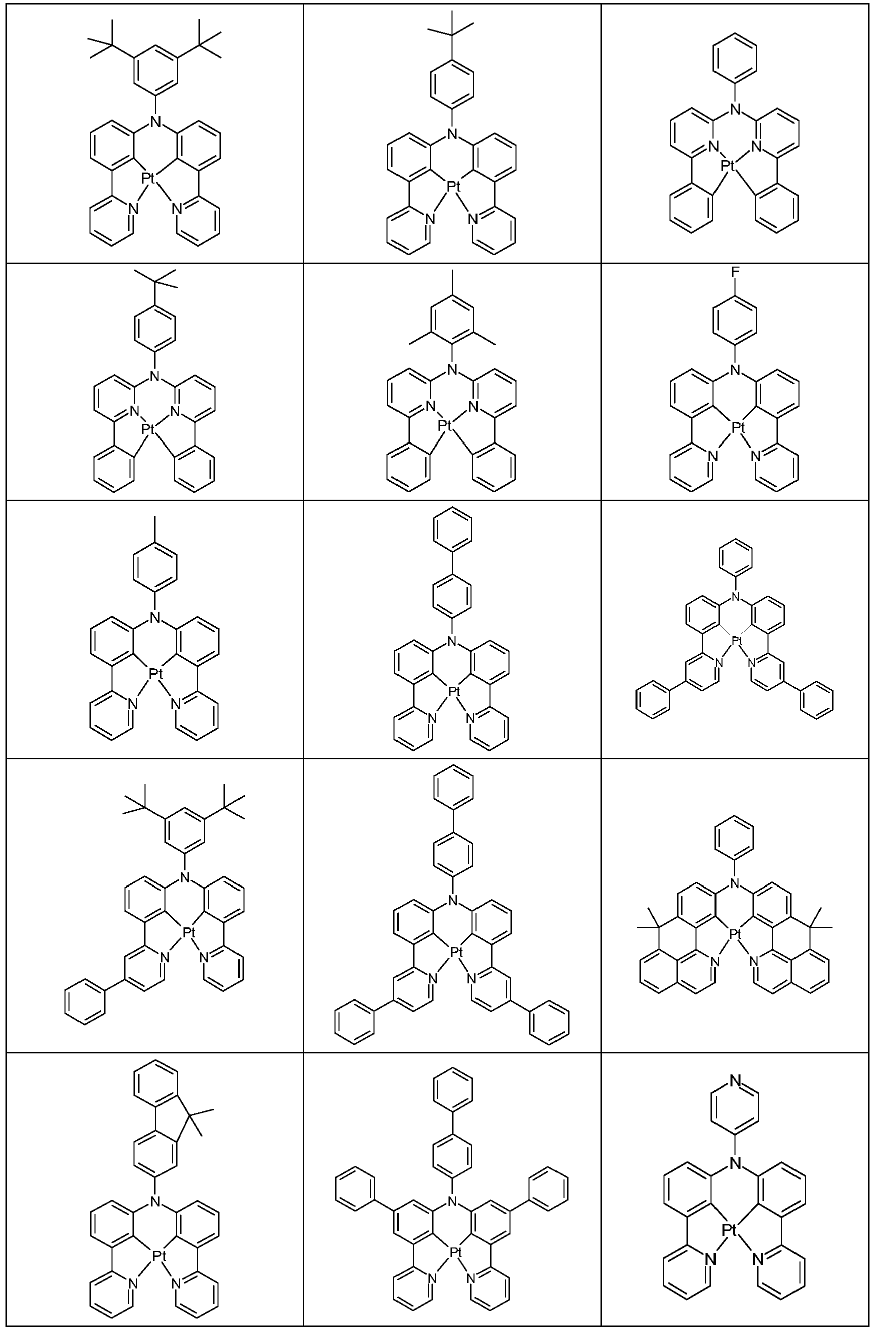

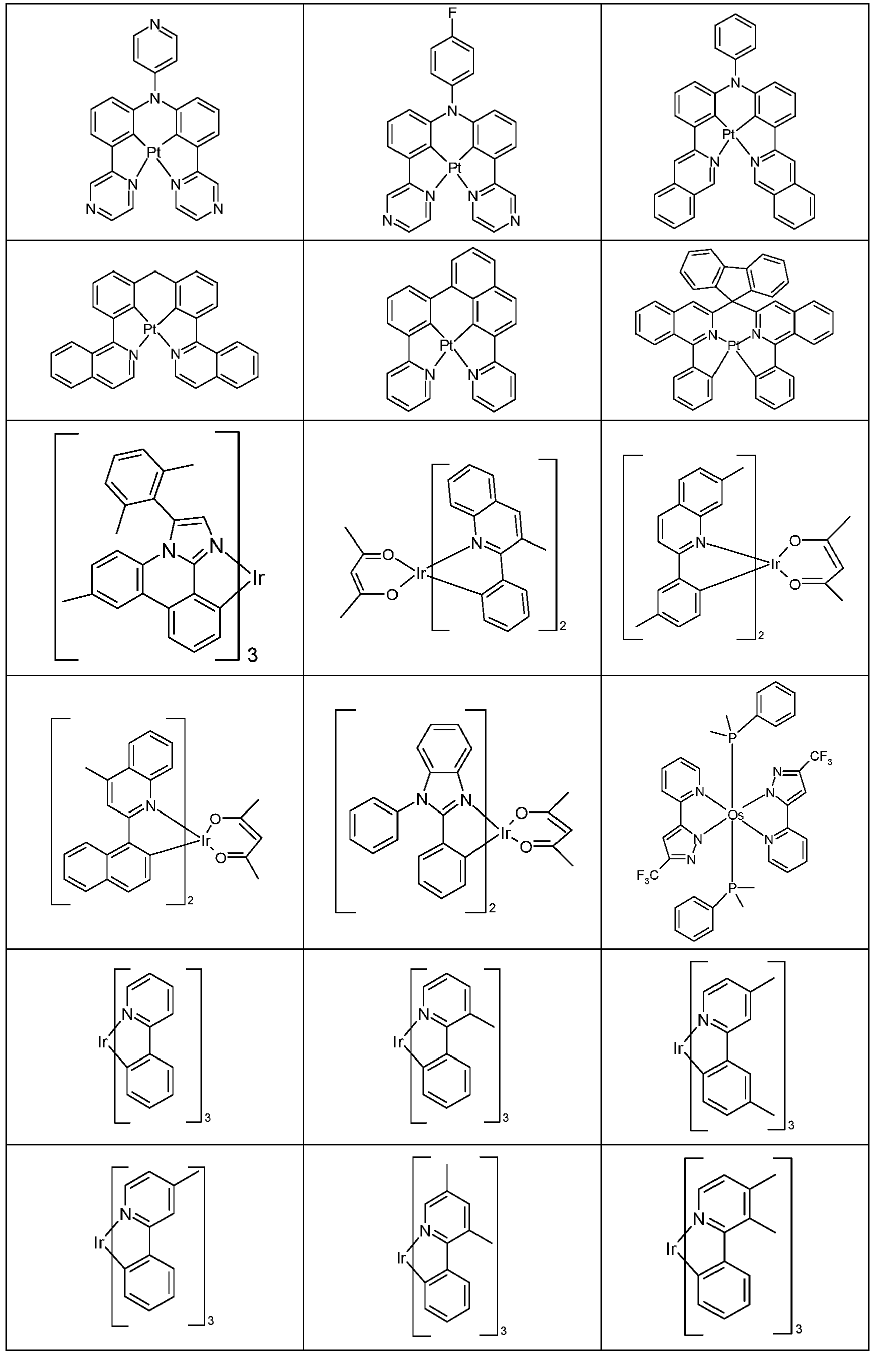

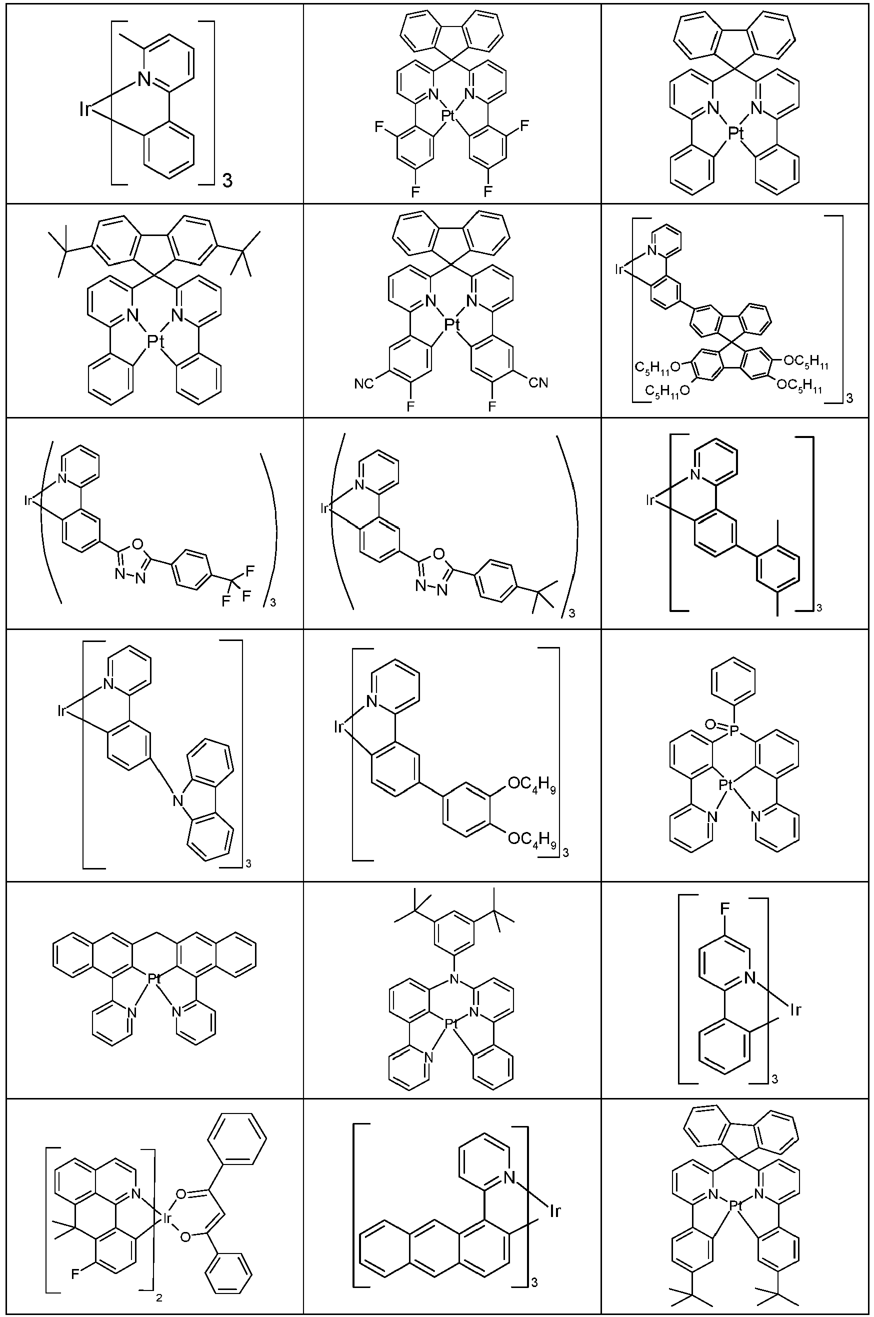

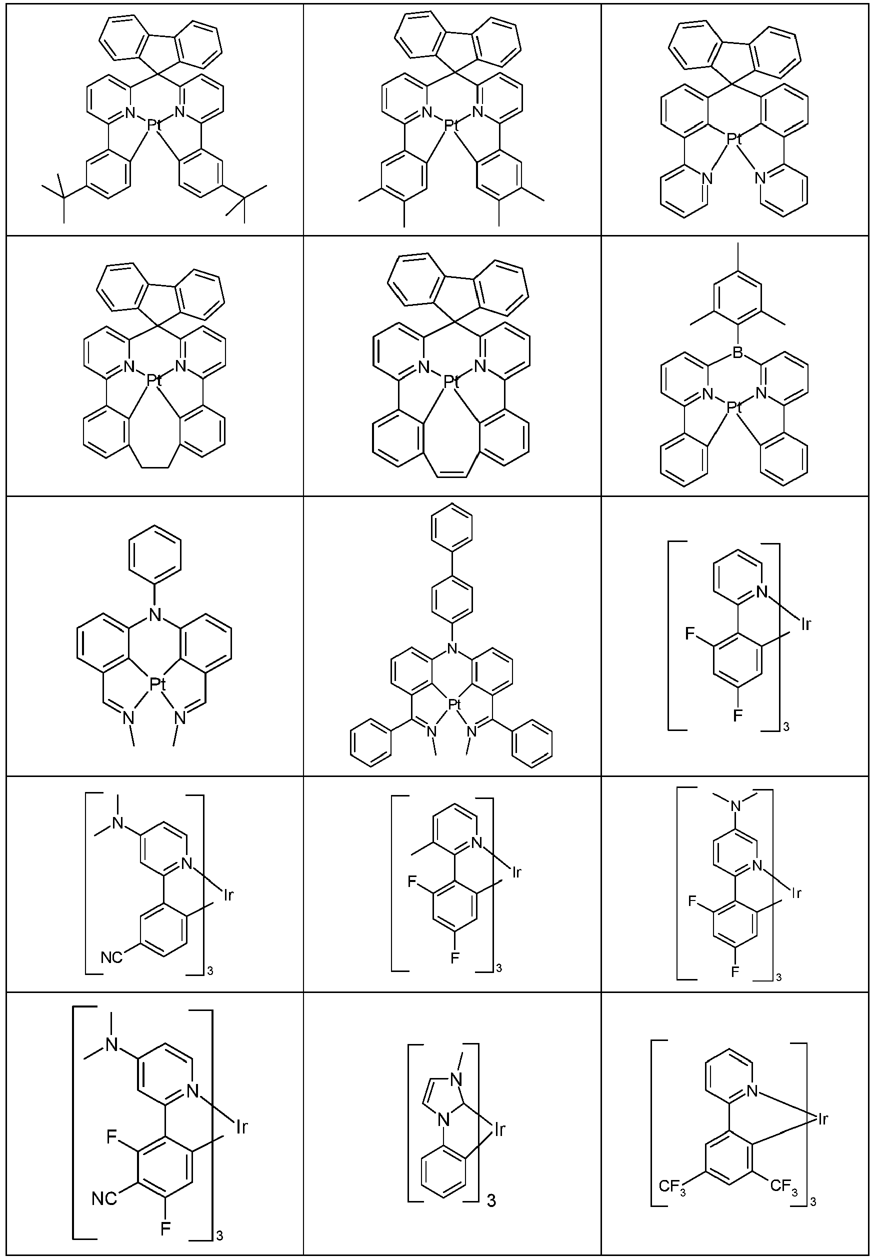

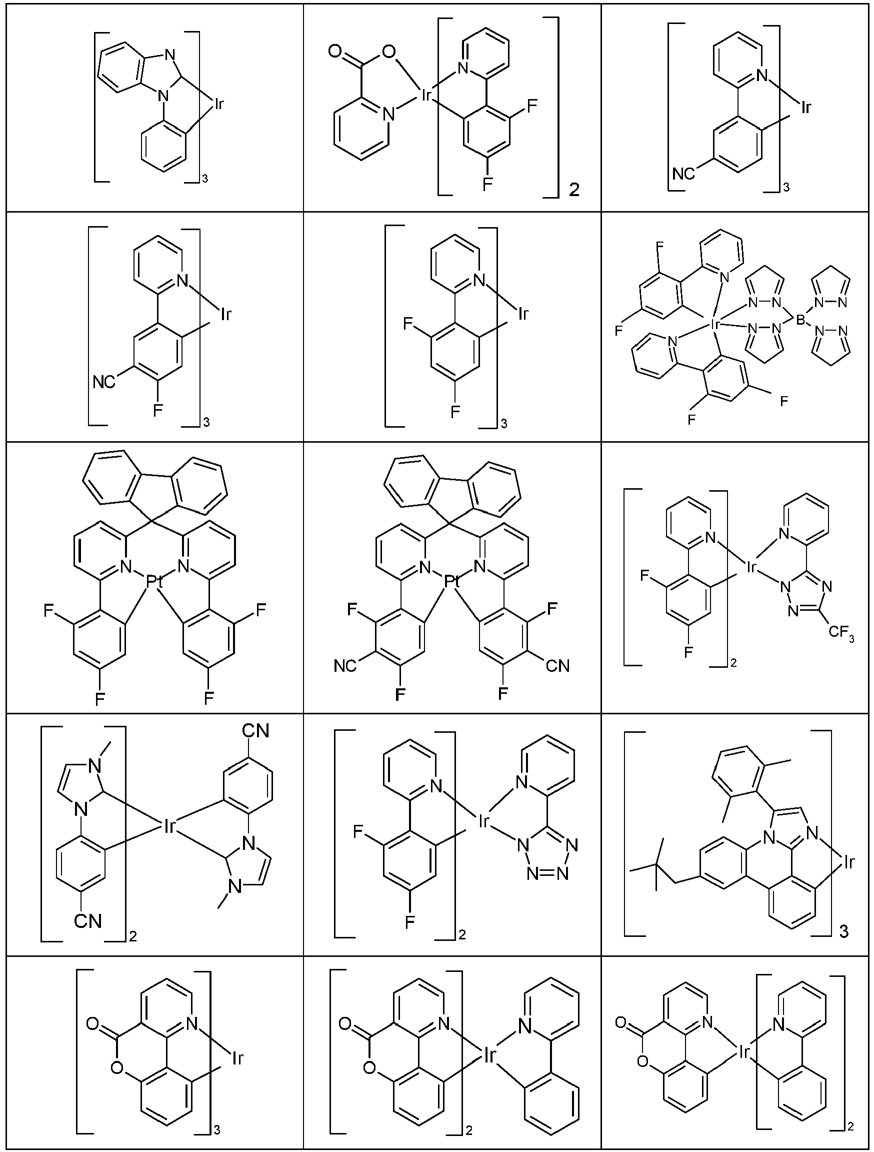

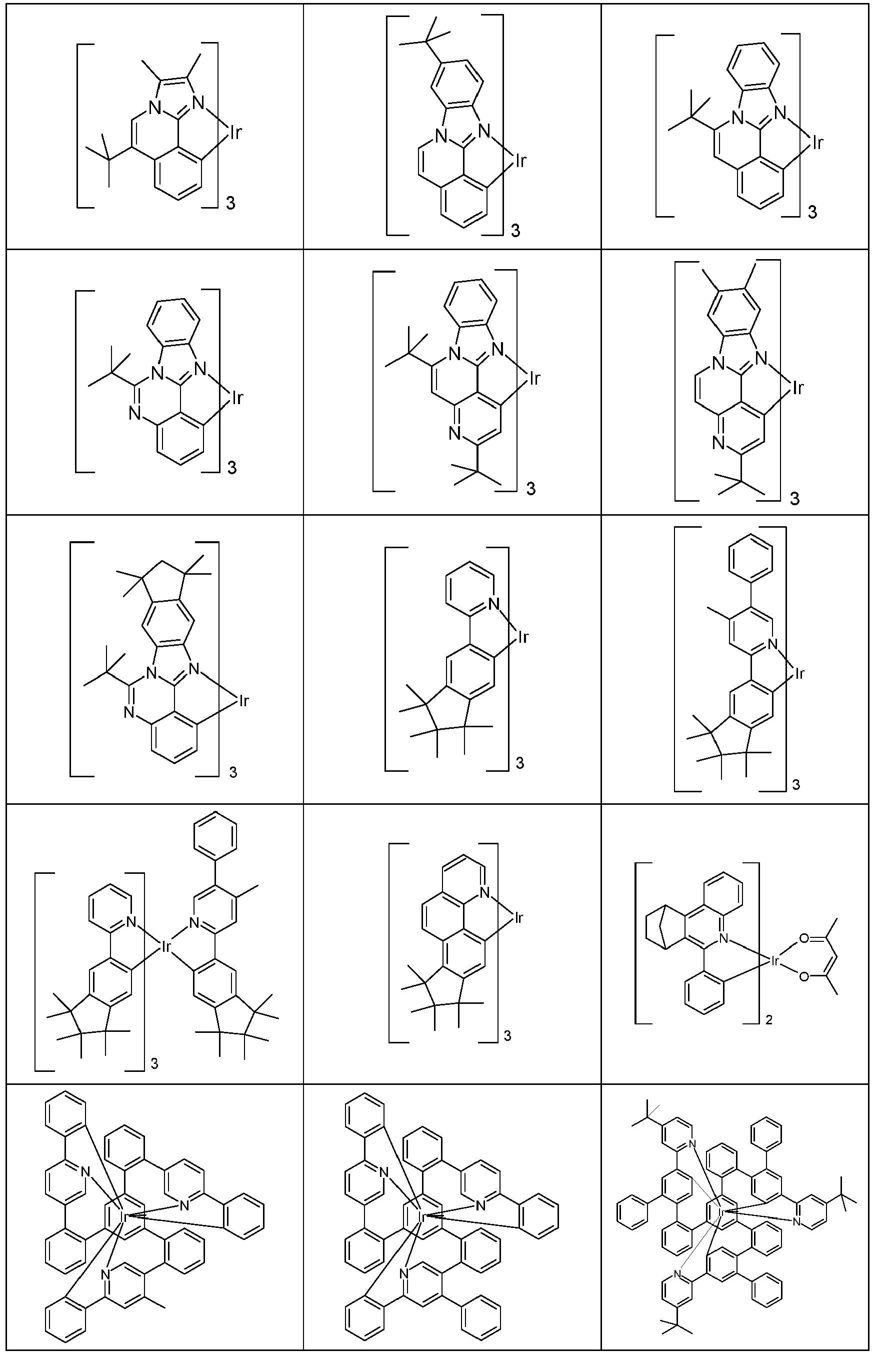

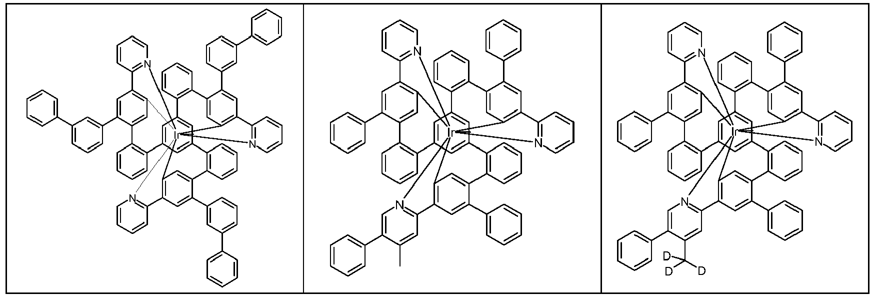

Preferred fluorescent emitting compounds are selected from the class of the arylamines. An arylamine or an aromatic amine in the context of this invention is understood to mean a compound containing three substituted or unsubstituted aromatic or heteroaromatic ring systems bonded directly to the nitrogen. Preferably, at least one of these aromatic or heteroaromatic ring systems is a fused ring system, more preferably having at least 14 aromatic ring atoms. Preferred examples of these are aromatic anthracenamines, aromatic anthracenediamines, aromatic pyrenamines, aromatic pyrenediamines, aromatic chrysenamines or aromatic chrysenediamines. An aromatic anthracenamine is understood to mean a compound in which a diarylamino group is bonded directly to an anthracene group, preferably in the 9 position. An aromatic anthracenediamine is understood to mean a compound in which two diarylamino groups are bonded directly to an anthracene group, preferably in the 9,10 positions. Aromatic pyrenamines, pyrenediamines, chrysenamines and chrysenediamines are defined analogously, where the diarylamino groups are bonded to the pyrene preferably in the 1 position or 1,6 positions. Further preferred emitting compounds are indenofluorenamines or -fluorenediamines, for example according to WO 2006/108497 or WO 2006/122630, benzoindenofluorenamines or - fluorenediamines, for example according to WO 2008/006449, and dibenzoindenofluoreneamines or -diamines, for example according to WO 2007/140847, and the indenofluorene derivatives having fused aryl groups disclosed in WO 2010/012328. Likewise preferred are the pyrenearylamines disclosed in WO 2012/048780 and in WO 2013/185871. Likewise preferred are the benzoindenofluorenamines disclosed in WO 2014/037077, the benzofluorenamines disclosed in WO 2014/106522, the extended benzoindenofluorenes disclosed in WO 2014/111269 and in WO 2017/036574, the phenoxazines disclosed in WO 2017/028940 and in WO 2017/028941, and the fluorene derivatives bonded to furan units or to thiophene units that are disclosed in WO 2016/150544.

Useful matrix materials, preferably for fluorescent emitting compounds, include materials of various substance classes. Preferred matrix materials are selected from the classes of the oligoarylenes (e.g. 2,2',7,7'-tetraphenylspirobifluorene according to EP 676461 or dinaphthylanthracene), especially of the oligoarylenes containing fused aromatic groups, the oligoarylenevinylenes (e.g. DPVBi or spiro-DPVBi according to EP 676461), the polypodal metal complexes (for example according to WO 2004/081017), the hole-conducting compounds (for example according to WO 2004/058911), the electron-conducting compounds, especially ketones, phosphine oxides, sulphoxides, etc. (for example according to WO 2005/084081 and WO 2005/084082), the atropisomers (for example according to WO 2006/048268), the boronic acid derivatives (for example according to WO 2006/117052) or the benzanthracenes (for example according to WO 2008/145239). Particularly preferred matrix materials are selected from the classes of the oligoarylenes comprising naphthalene, anthracene, benzanthracene and/or pyrene or atropisomers of these compounds, the oligoarylenevinylenes, the ketones, the phosphine oxides and the sulphoxides. Very particularly preferred matrix materials are selected from the classes of the oligoarylenes comprising anthracene, benzanthracene, benzophenanthrene and/or pyrene or atropisomers of these compounds. An oligoarylene in the context of this invention shall be understood to mean a compound in which at least three aryl or arylene groups are bonded to one another. Preference is further given to the anthracene derivatives disclosed in WO 2006/097208, WO 2006/131192, WO 2007/065550, WO 2007/110129, WO 2007/065678, WO 2008/145239, WO 2009/100925, WO 2011/054442 and EP 1553154, the pyrene compounds disclosed in EP 1749809, EP 1905754 and US 2012/0187826, the benzanthracenylanthracene compounds disclosed in WO 2015/158409, the indenobenzofurans disclosed in WO 2017/025165, and the phenanthrylanthracenes disclosed in WO 2017/036573.

Preferred matrix materials for phosphorescent emitting compounds are, as well as the compounds of the formula (IA) or (IB), aromatic ketones, aromatic phosphine oxides or aromatic sulphoxides or sulphones, for example according to WO 2004/013080, WO 2004/093207, WO 2006/005627 or WO 2010/006680, triarylamines, carbazole derivatives, e.g. CBP (N,N-biscarbazolylbiphenyl) or the carbazole derivatives disclosed in WO 2005/039246, US 2005/0069729, JP 2004/288381, EP 1205527 or WO 2008/086851, indolocarbazole derivatives, for example according to WO 2007/063754 or WO 2008/056746, indenocarbazole derivatives, for example according to WO 2010/136109, WO 2011/000455 or WO 2013/041176, azacarbazole derivatives, for example according to EP 1617710, EP 1617711, EP 1731584, JP 2005/347160, bipolar matrix materials, for example according to WO 2007/137725, silanes, for example according to WO 2005/111172, azaboroles or boronic esters, for example according to WO 2006/117052, triazine derivatives, for example according to WO 2010/015306, WO 2007/063754 or WO 2008/056746, zinc complexes, for example according to EP 652273 or WO 2009/062578, diazasilole or tetraazasilole derivatives, for example according to WO 2010/054729, diazaphosphole derivatives, for example according to WO 2010/054730, bridged carbazole derivatives, for example according to US 2009/0136779, WO 2010/050778, WO 2011/042107, WO 2011/088877 or WO 2012/143080, triphenylene derivatives, for example according to WO 2012/048781, or lactams, for example according to WO 2011/116865 or WO 2011/137951.

Suitable charge transport materials as usable in the hole injection or hole transport layer or electron blocking layer or in the electron transport layer of the electronic device of the invention are, as well as the compounds of the formula (IA) or (IB), for example, the compounds disclosed in Y. Shirota et al., Chem. Rev. 2007, 107(4), 953-1010, or other materials as used in these layers according to the prior art.

Preferably, the inventive OLED comprises two or more different hole-transporting layers. The compound of the formula (IA) or (IB) may be used here in one or more of or in all the hole-transporting layers. In a preferred embodiment, the compound of the formula (IA) or (IB) is used in exactly one or exactly two hole-transporting layers, and other compounds, preferably aromatic amine compounds, are used in the further hole-transporting layers present. Further compounds which are used alongside the compounds of the formula (IA) or (IB), preferably in hole-transporting layers of the OLEDs of the invention, are especially indenofluorenamine derivatives (for example according to WO 06/122630 or WO 06/100896), the amine derivatives disclosed in EP 1661888, hexaazatriphenylene derivatives (for example according to WO 01/049806), amine derivatives with fused aromatics (for example according to US 5,061,569), the amine derivatives disclosed in WO 95/09147, monobenzoindenofluorenamines (for example according to WO 08/006449), dibenzoindenofluorenamines (for example according to WO 07/140847), spirobifluorenamines (for example according to WO 2012/034627 or WO 2013/120577), fluorenamines (for example according to WO 2014/015937, WO 2014/015938, WO 2014/015935 and WO 2015/082056), spirodibenzopyranamines (for example according to WO 2013/083216), dihydroacridine derivatives (for example according to WO 2012/150001), spirodibenzofurans and spirodibenzothiophenes, for example according to WO 2015/022051, WO 2016/102048 and WO 2016/131521, phenanthrenediarylamines, for example according to WO 2015/131976, spirotribenzotropolones, for example according to WO 2016/087017, spirobifluorenes with meta-phenyldiamine groups, for example according to WO 2016/078738, spirobisacridines, for example according to WO 2015/158411, xanthenediarylamines, for example according to WO 2014/072017, and 9,10-dihydroanthracene spiro compounds with diarylamino groups according to WO 2015/086108.

Very particular preference is given to the use of spirobifluorenes substituted by diarylamino groups in the 4 position as hole-transporting compounds, especially to the use of those compounds that are claimed and disclosed in WO 2013/120577, and to the use of spirobifluorenes substituted by diarylamino groups in the 2 position as hole-transporting compounds, especially to the use of those compounds that are claimed and disclosed in WO 2012/034627.

Materials used for the electron transport layer may be any materials as used according to the prior art as electron transport materials in the electron transport layer. Especially suitable are aluminum complexes, for example Alq₃, zirconium complexes, for example Zrq₄, lithium complexes, for example Liq, benzimidazole derivatives, triazine derivatives, pyrimidine derivatives, pyridine derivatives, pyrazine derivatives, quinoxaline derivatives, quinoline derivatives, oxadiazole derivatives, aromatic ketones, lactams, boranes, diazaphosphole derivatives and phosphine oxide derivatives. Further suitable materials are derivatives of the abovementioned compounds as disclosed in JP 2000/053957, WO 2003/060956, WO 2004/028217, WO 2004/080975 and WO 2010/072300.

Preferred cathodes of the electronic device are metals having a low work function, metal alloys or multilayer structures composed of various metals, for example alkaline earth metals, alkali metals, main group metals or lanthanoids (e.g. Ca, Ba, Mg, Al, In, Mg, Yb, Sm, etc.). Additionally suitable are alloys composed of an alkali metal or alkaline earth metal and silver, for example an alloy composed of magnesium and silver. In the case of multilayer structures, in addition to the metals mentioned, it is also possible to use further metals having a relatively high work function, for example Ag or Al, in which case combinations of the metals such as Ca/Ag, Mg/Ag or Ba/Ag, for example, are generally used. It may also be preferable to introduce a thin interlayer of a material having a high dielectric constant between a metallic cathode and the organic semiconductor. Examples of useful materials for this purpose are alkali metal or alkaline earth metal fluorides, but also the corresponding oxides or carbonates (e.g. LiF, Li₂O, BaF₂, MgO, NaF, CsF, Cs₂CO₃, etc.). It is also possible to use lithium quinolinate (LiQ) for this purpose. The layer thickness of this layer is preferably between 0.5 and 5 nm.

Preferred anodes are materials having a high work function. Preferably, the anode has a work function of greater than 4.5 eV versus vacuum. Firstly, metals having a high redox potential are suitable for this purpose, for example Ag, Pt or Au. Secondly, metal/metal oxide electrodes (e.g. Al/Ni/NiOₓ, Al/PtOₓ) may also be preferred. For some applications, at least one of the electrodes has to be transparent or partly transparent in order to enable the irradiation of the organic material (organic solar cell) or the emission of light (OLED, O-laser). Preferred anode materials here are conductive mixed metal oxides. Particular preference is given to indium tin oxide (ITO) or indium zinc oxide (IZO). Preference is further given to conductive doped organic materials, especially conductive doped polymers. In addition, the anode may also consist of two or more layers, for example of an inner layer of ITO and an outer layer of a metal oxide, preferably tungsten oxide, molybdenum oxide or vanadium oxide.

The device is structured appropriately (according to the application), contact-connected and finally sealed, in order to rule out damaging effects by water and air.

In a preferred embodiment, the electronic device is characterized in that one or more layers are coated by a sublimation process. In this case, the materials are applied by vapour deposition in vacuum sublimation systems at an initial pressure of less than 10⁻⁵ mbar, preferably less than 10⁻⁶ mbar. In this case, however, it is also possible that the initial pressure is even lower, for example less than 10⁻⁷ mbar.

Preference is likewise given to an electronic device, characterized in that one or more layers are coated by the OVPD (organic vapour phase deposition) method or with the aid of a carrier gas sublimation. In this case, the materials are applied at a pressure between 10⁻⁵ mbar and 1 bar. A special case of this method is the OVJP (organic vapour jet printing) method, in which the materials are applied directly by a nozzle and thus structured (for example M. S. Arnold et al., Appl. Phys. Lett. 2008, 92, 053301).

Preference is additionally given to an electronic device, characterized in that one or more layers are produced from solution, for example by spin-coating, or by any printing method, for example screen printing, flexographic printing, nozzle printing or offset printing, but more preferably LITI (light-induced thermal imaging, thermal transfer printing) or inkjet printing. For this purpose, soluble compounds of formula (IA) or (IB) are needed. High solubility can be achieved by suitable substitution of the compounds.

It is further preferable that an electronic device of the invention is produced by applying one or more layers from solution and one or more layers by a sublimation method.

According to the invention, the electronic devices comprising one or more compounds of formula (IA) or (IB) can be used in displays, as light sources in lighting applications and as light sources in medical and/or cosmetic applications (e.g. light therapy).

### Examples

### A) Synthesis examples

### A-1) Route 1

### Synthesis of 2',7'-di-tert-Butyl-1-bromospiro-9,9'-bifluorene 1a

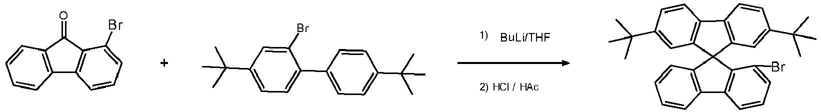

A solution of 4, 4'-di-t-Butyl-2,Br-biphenyl (250 g, 725 mmol) in THF (1900ml) is treated with 318 mL of n-BuLi (2,5 M in hexane, 785 mmol) under argon at -78 °C. The mixture is stirred for 30 minutes. A solution of 1-Br-fluoren-9-one (144 g, 556 mmol) in 1000 mL THF is added dropwise. The reaction proceeds at -78 °C for 30 minutes and then is stirred at room temperature overnight. The reaction is quenched with water and the solid is filtered. Without further purification, a mixture of the alcohol (262 g, 90%) , acetic acid (2200 mL) and concentrated HCl (100 mL) is refluxed for 2 hours. After cooling, the mixture is filtered and washed with water and dried under vacuum. The product is isolated in the form of a white solid (240 g, 95% of theory).

The synthesis of further brominated spirobifluorene derivatives is carried out analogously:

| Ex. | Bromo-biphenyl | Bromo-fluorenone | Product: |
|---|---|---|---|
| | | | Bromo-Spirobifluorene |
| 1b | 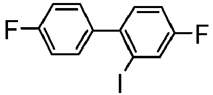 | 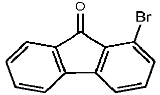 | 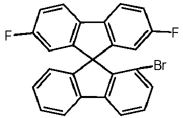 |
| 1c | 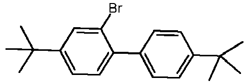 70728-89-1 | 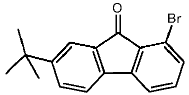 2082789-21-5 | 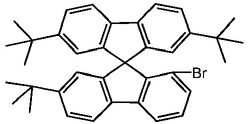 |
| 1d | 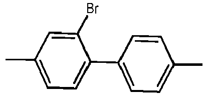 132462-55-6 | 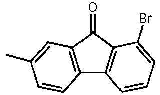 2082789-19-1 | 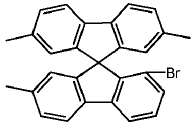 |
| 1e | 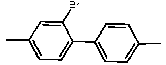 132462-55-6 | 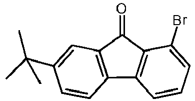 2082789-21-5 | 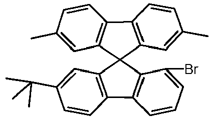 |
| 1f | 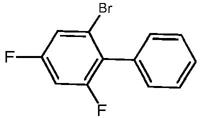 1428234-76-7 | 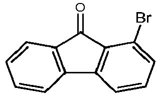 | 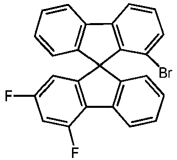 |
| 1g | 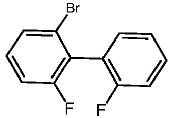 2060059-24-5 | 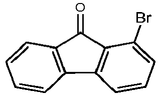 | 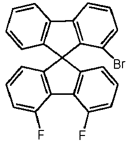 |
| 1h | 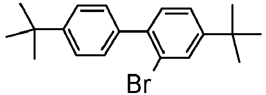 [70728-89-1] | 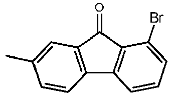 | 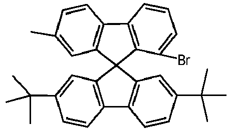 |
| 1i | 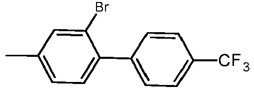 | 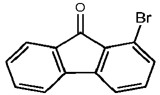 | 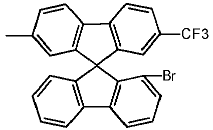 |
| 1j | 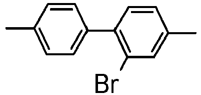 [132462-55-6] | 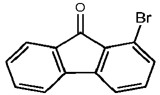 | 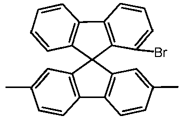 |
| 1k | 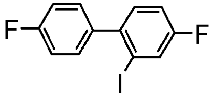 [1214351-66-2] | 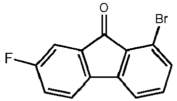 2082789-15-7 | 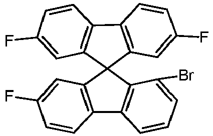 |
| 1l | 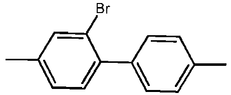 132462-55-6 | 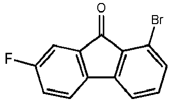 2082789-15-7 | 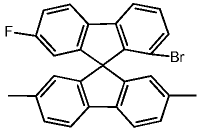 |
| 1m | 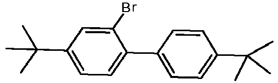 70728-89-1 | 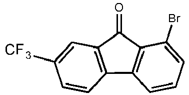 2082789-18-0 | 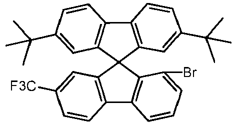 |

### Synthesis of 2',7'-di-tert-Butyl-4-biphenyl-2-(9,9'-dimethylfluorenyl)-1 -spiro-9,9'-bifluorenylamine 2a

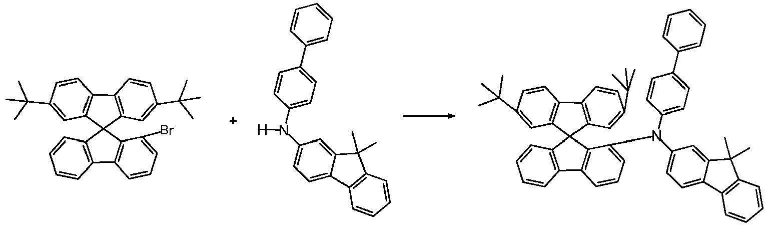

Tri-*tert*-butylphosphine (4.4 ml of a 1.0 M solution in toluene, 4.4 mmol), palladium acetate (248 mg, 1.1 mmol) and sodium *tert*-butoxide (16.0 g, 166 mmol) are added to a solution of biphenyl-2-yl-(9,9-dimethyl-9*H*-fluo-ren-2-yl)amine (40.0 g, 111 mmol) and 2',7'-di-tertButyl-1-bromospiro-9,9'-bifluorene (56.9 g, 108 mmol) in degassed toluene (500 ml), and the mixture is heated under reflux for 2 h. The reaction mixture is cooled to room temperature, extended with toluene and filtered through Celite. The filtrate is evaporated in vacuo, and the residue is crystallised from ethyl acetate/heptane. The crude product is extracted in a Soxhlet extractor (toluene) and purified by zone sublimation in vacuo twice (p = 3 × 10⁻⁴ mbar, T = 298°C). The product is isolated in the form of a pale-yellow solid (20.4 g, 24% of theory, purity > 99.99% according to HPLC).

The following compounds are obtained analogously:

| Ex. | Br-spiro | Amine | Product |
|---|---|---|---|
| 2b | 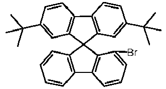 | 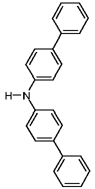 [102113-98-4] | 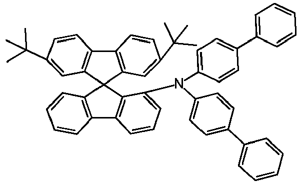 |
| 2c | 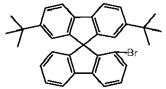 | 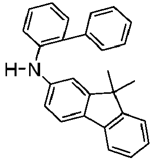 [1198395-24-2] | 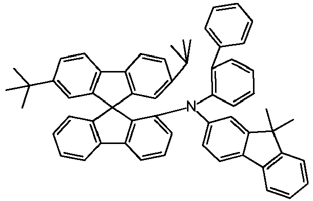 |
| 2d | 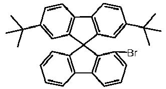 | 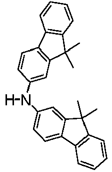 [500717-23-7] | 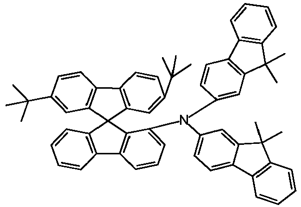 |
| #2e | 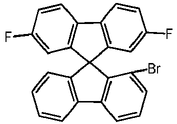 | 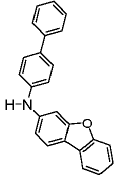 [1290039-85-8] | 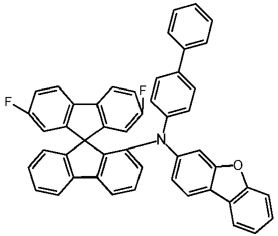 |
| 2f | 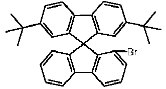 | 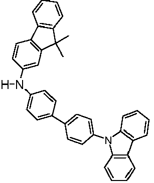 [1267248-54-3] | 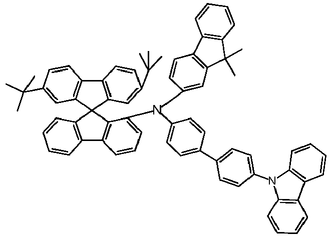 |
| 2g | 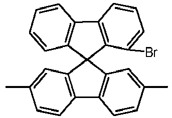 | 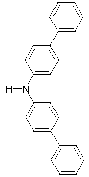 [102113-98-4] | 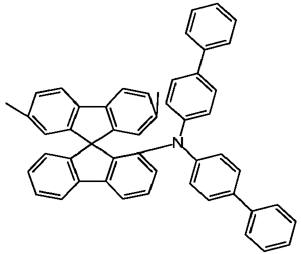 |
| 2h | 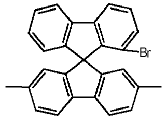 | 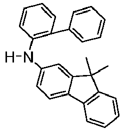 [1198395-24-2] | 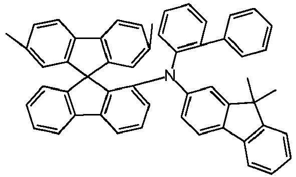 |
| 2i | 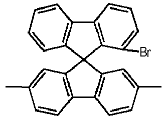 | 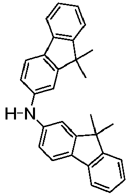 [500717-23-7] | 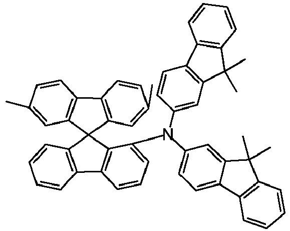 |
| #2j | 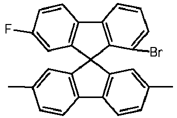 | 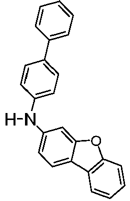 [1290039-85-8] | 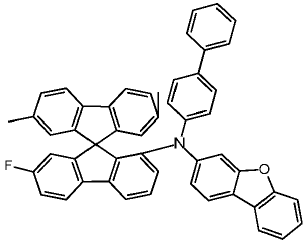 |
| 2k | 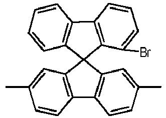 | 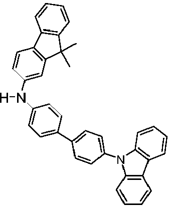 [1267248-54-3] | 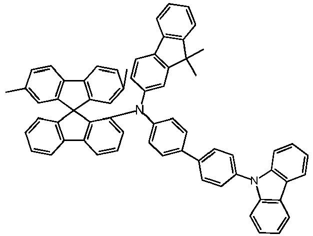 |
| 2l | 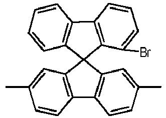 | 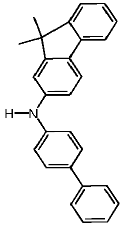 | 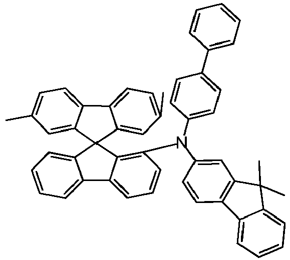 |

### A-2) Route 2:Synthesis of 2',1'-di-tert-Butyl-4-biphenyl-2-(9,9'-dimethyl-fluorenyl)-1 -spiro-9,9'-bifluorenylamine 2a

### Synthesis of 1-(1-biphen-4-yl)-(9,9'-dimethylfluoren-2-yl)amine-9H-Fluoren-9-one 3a

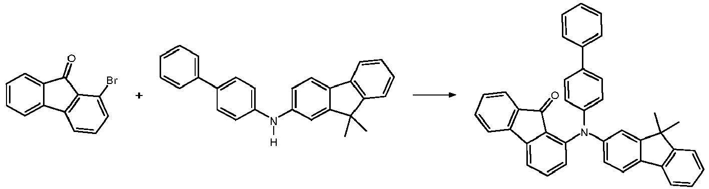

Tri-tert-butylphosphine (4.5 ml of a 1.0 M solution in toluene, 1,9 mmol), palladium acetate (217 mg, 0.97 mmol) and sodium *tert*-butoxide (13.9 g, 145 mmol) are added to a solution of 1-biphenyl-yl-(9,9-dimethyl-9*H*-fluoren-2-yl)-amine (40.0 g, 111 mmol), 1-bromo-fluoren-9-one, (25 g, 96 mmol) in degassed toluene (200 ml), and the mixture is heated under reflux overnight. The reaction mixture is cooled to room temperature, extended with toluene and filtered through Celite. The filtrate is evaporated in vacuo, and the residue is crystallised from toluene/heptane The product is isolated in the form of a pale-yellow solid (43 g, 82% of theory).

The following compounds are obtained analogously:

| Ex. | Bromofluorenone | Amine | Product: |
|---|---|---|---|
| | | | 1-Amine-fluorenone |
| 3b | 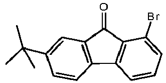 2082789-21-5 | 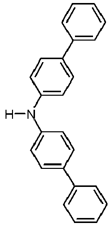 [102113-98-4] | 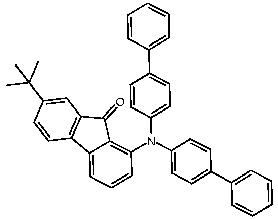 |
| 3c | 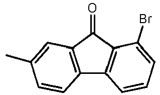 2082789-19-1 | 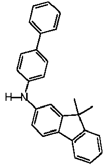 | 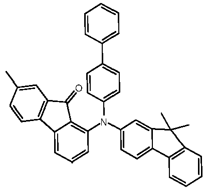 |
| 3d | 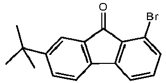 2082789-21-5 | 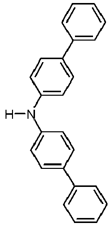 | 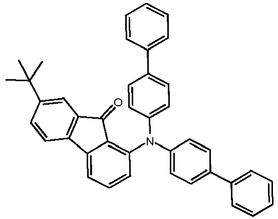 |
| 3e | 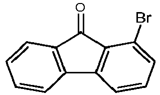 | 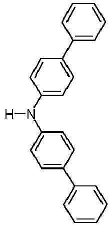 | 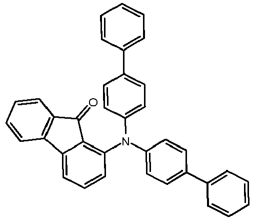 |
| 3f | 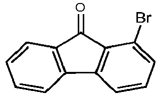 | 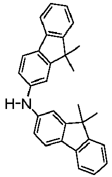 | 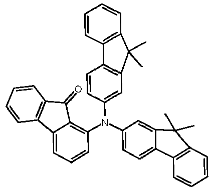 |
| 3g | 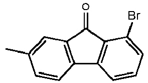 | 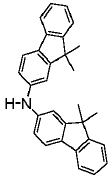 | 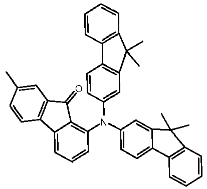 |
| 3h | 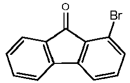 | 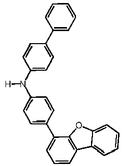 | 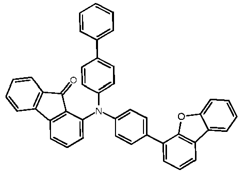 |
| 3i | 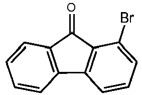 | 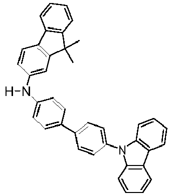 | 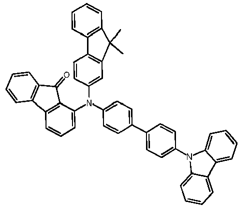 |
| 3j | 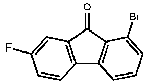 2082789-15-7 | 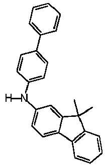 | 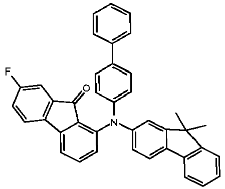 |
| 3k | 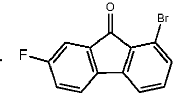 2082789-15-7 | 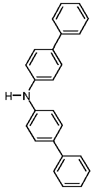 | 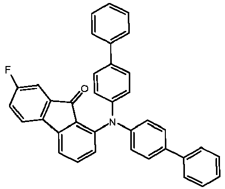 |
| 3l | 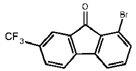 2082789-18-0 | 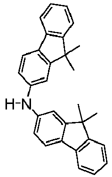 | 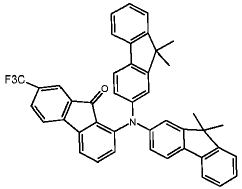 |
| 3m | 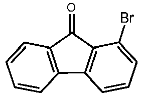 | 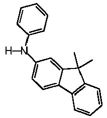 | 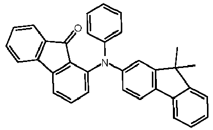 |

### Synthesis of 2',7'-di-tert-Butyl-4-biphenyl-2-(9,9'-dimethylfluorenyl)-1 -spiro-9,9'-bifluorenylamine 4a

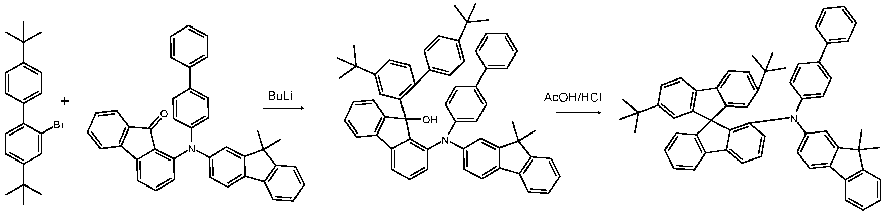

A solution of 4, 4'-di-t-butyl-2-Br-biphenyl (17 g, 49 mmol) in THF (90ml) is treated with 25 mL of *n*-BuLi (2,1 M in hexane, 50 mmol) under argon at -78 °C. The mixture is stirred for 30 minutes. A solution of of 1-(1-biphen-4-yl)-(9,9'-dimethylfluoren-2-yl)amine-9H-fluoren-9-one (27 g, 50 mmol) in 90 mL THF is added dropwise. The reaction proceeds at -78 °C for 30 minutes and then is stirred at room temperature overnight. The reaction is quenched with water and extracted with ethyl acetate. The intermediate alcohol is obtained after the solvent is removed (31 g, 76%). Without further purification, a mixture of the alcohol, acetic acid (700 mL) and concentrated HCl (62 mL) is refluxed for 2 hours. After cooling, the mixture is filtered and washed with water. The residue is crystallised from toluene. The crude product is extracted in a Soxhlet extractor (toluene) and purified by zone sublimation in vacuo. The product is isolated in the form of a pale-yellow solid (13 g, 42% of theory, purity > 99.99% according to HPLC).

The following compounds are obtained analogously:

| Ex. | 1-Amine-fluorenone | Br-Biphenyl | Product: |
|---|---|---|---|
| 4b | 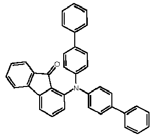 | 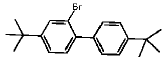 70728-89-1 | 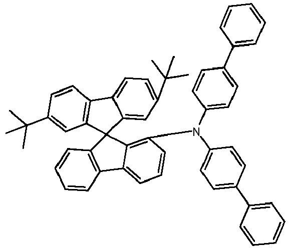 |
| 4c | 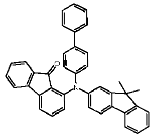 | 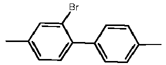 132462-55-6 | 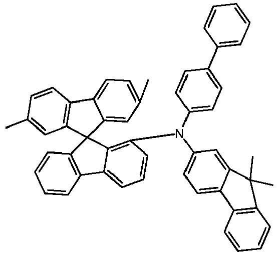 |
| #4d | 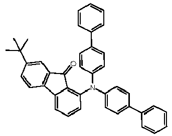 | 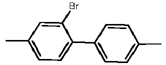 132462-55-6 | 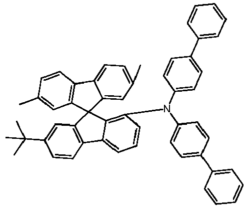 |
| 4e | 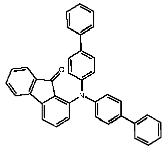 | 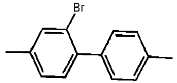 | 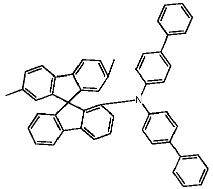 |
| #4f | 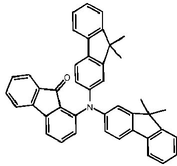 | 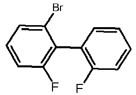 2060059-24-5 | 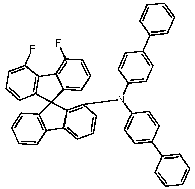 |
| 4g | 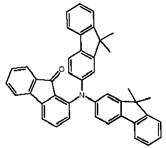 | 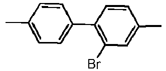 | 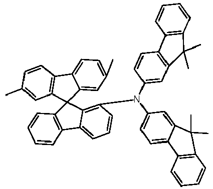 |
| 4h | 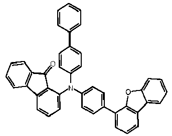 | 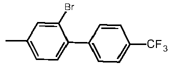 | 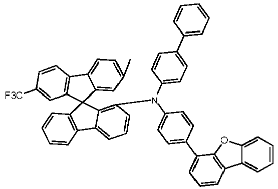 |
| 4i | 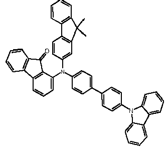 | 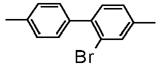 [132462-55-6] | 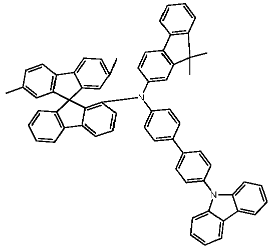 |
| #4j | 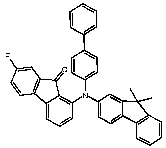 | 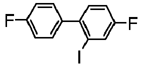 [1214351-66-2] | 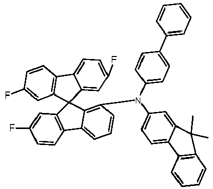 |
| #4k | 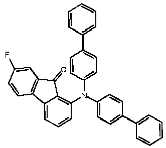 | 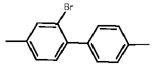 132462-55-6 | 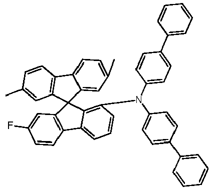 |
| 4l | 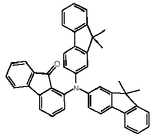 | 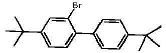 70728-89-1 | 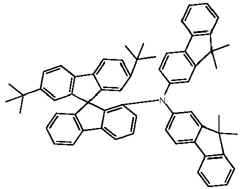 |
| 4m | 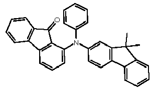 | 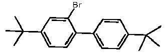 | 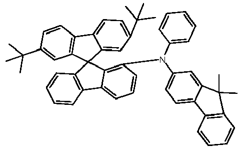 |

### A-3) Route 3: Synthesis of 2',7'-di-tert-Butyl-biphenyl-4-yl-(9,9-dimethyl-9H-fluoren-2-yl)-[1-(9,9'-spiro-bifluoren-4-yl)-phenyl]-amine

### Synthesis of Biphenyl-4-yl-(9,9-dimethyl-9H-fluoren-2-yl (4,4,5,5tetramethyl-[1,3,2]dioxaborolan-2-yl)-phenyl]-amine

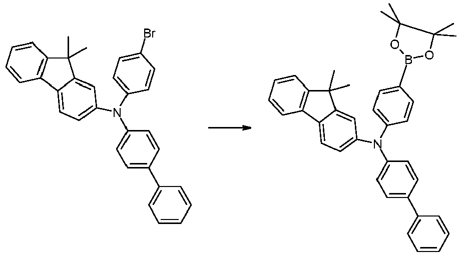

102 g (198 mmol) of Biphenyl-4-yl-(4-bromo-phenyl)-(9,9-dimethyl-9H-fluoren-2-yl)-amine, 4,8 g (5,9 mmol) of Pd(dppf)Cl₂, 61,6 g ( 238 mmol) of bis(pinacolato)diboron and 58,3 g (594 mmol) of potassium acetate are dissolved in 1300 mL of 1,4-dioxane. The reaction mixture is refluxed and agitated under an argon atmosphere for 12 hours and after cooling to room temperature, the mixture is filtered through Celite. The filtrate is evaporated in vacuo, and the residue is crystallised from heptane. The product is isolated in the form of a pale-yellow solid (87 g, 78% of theory).

### Synthesis of 2',7'-di-tert-Butyl-biphenyl-4-yl-(9,9-dimethyl-9H-fluoren-2-yl)-[1-(9,9'-spiro-bifluoren-4-yl)-phenyl]-amine 5a

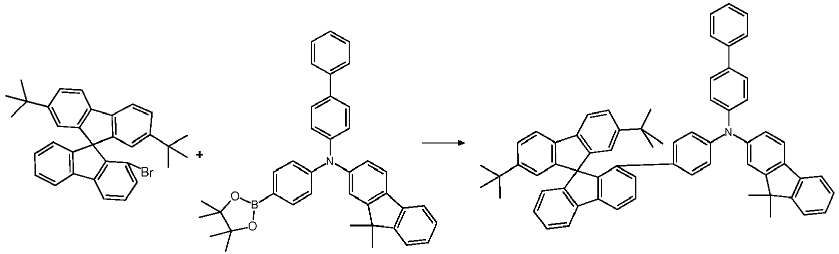

28 g (49,4 mmol) of Biphenyl-4-yl-(9,9-dimethyl-9H-fluoren-2-yl (4,4,5,5-tetramethyl-[1,3,2]dioxaborolan-2-yl)-phenyl]-amine, 20 g (39 mmol) of 2',7'-di-tert-butyl-1-bromospiro-9,9'-bifluorene, 1,8 g (2,5 mmol) of PdCl₂(Cy)₃, 15 g ( 99 mmol) of cesium fluoride are dissolved in 500 mL of toluene. The reaction mixture is refluxed and agitated under an argon atmosphere for 12 hours and after cooling to room temperature, the mixture is filtered through Celite. The filtrate is evaporated in vacuo, and the residue is crystallised from heptane. The crude product is extracted in a Soxhlet extractor (toluene) and purified by zone sublimation in vacuo twice.

The product is isolated in the form of a pale-yellow solid (9 g, 25% of theory, purity > 99.99% according to HPLC).

The following compounds are synthesized analogously:

| Ex. | Br-Spiro | Amine | Product |
|---|---|---|---|
| 5b | 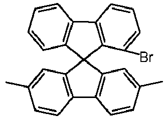 | 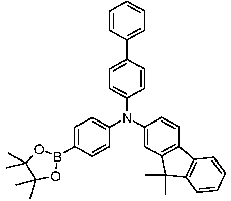 | 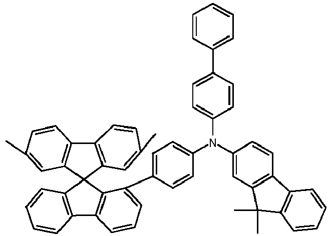 |
| 5c | 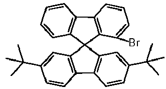 | 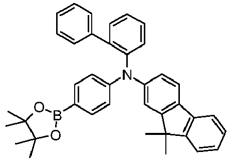 | 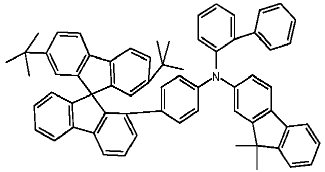 |
| #5d | 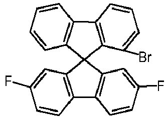 | 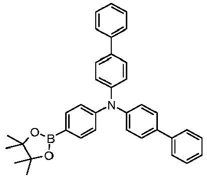 | 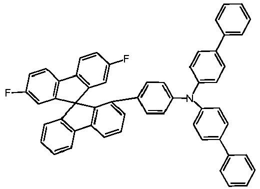 |
| 5e | 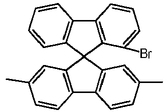 | 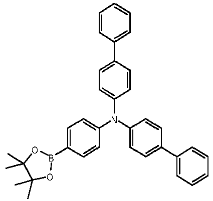 | 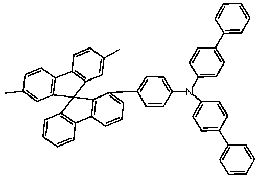 |

### A-4) Route 4: Synthesis of 2',7'-di-tert-Butyl-9-Spiro-1 -yl-3,6-diphenyl-9H-carbazol 6a

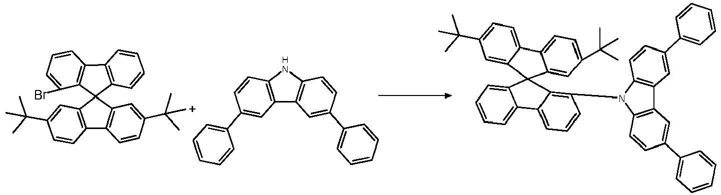

19.2 g (38 mmol) 2',1'-di-tert-Butyl-1-bromospiro-9,9'-bifluorene, 15 g (47 mmol) 3,6-Diphenyl-9-H-carbazole and 29.2 g Rb₂CO₃ are suspended in 250 mL p-Xylol. To the suspension are given 0.95 g (4,2 mmol) Pd(OAc)₂ and 12.6 ml of a 1M solution of Tri-tert-butylphosphine. The mixture is stirred 24 h under reflux. After cooling the organic phase is separated, washed three times with 150 mL water and is subsequently concentrated to dryness in vacuo. The residue is hot extracted with toluene, recrystallized three times from toluene and subsequently sublimated at high vacuum. Yield is 19.6 g (26.2 mmol) corresponding to 68 % of theory. Purity is according to HPLC 99.9 %.

The following compounds are obtained analogously:

| | Starting material 1 | Starting material 2 | Product |
|---|---|---|---|
| 6b | 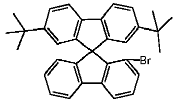 | 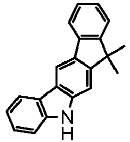 1257220-47-5 | 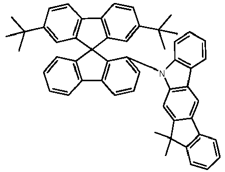 |
| 6c | 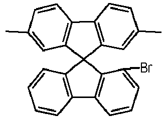 | 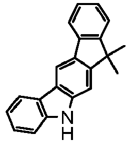 1257220-47-5 | 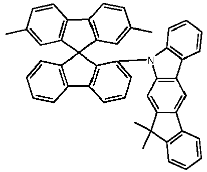 |
| 6d | 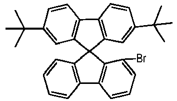 | 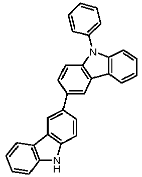 1060735-14-9 | 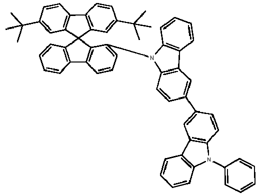 |
| 6e | 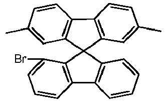 | 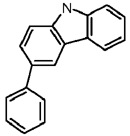 103012-26-6 | 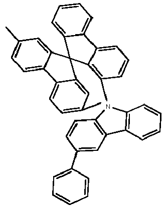 |

### A-5) Route 5: Synthesis of compound 7a

### Synthesis of 1-(4-Chloro-phenyl)-fluoren-9-one 7a

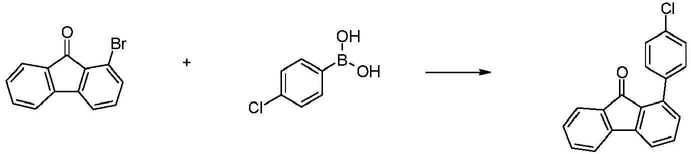

76 g (486 mmol) of 4-chlorophenylboronic acid, 120 g (463 mmol) of 1-Brom-fluoren-9-one and 16 g (14 mmol) of Pd(Ph₃P)₄ are suspended in 1900 ml of THF. 463 ml of 2 M potassium carbonate solution are slowly added to this suspension, and the reaction mixture is heated under reflux for 16 h. After cooling, the organic phase is separated off, filtered through silica gel, washed three times with 500 ml of water and subsequently evaporated to dryness. The residue is purified by crystallitation with MeOH. Yield: 125 g (420 mmol), 90 % of theory, purity according to HPLC >98%.

| | **1-Br-Fluorenone** | **Boron acid** | **Product** |
|---|---|---|---|
| 7b | 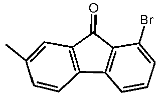 | 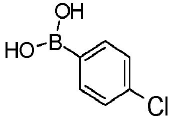 | 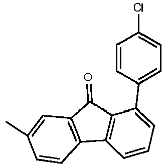 |
| 7c | 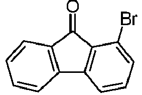 | 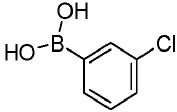 (63503-60-6) | 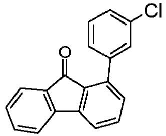 |
| 7d | 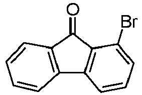 | 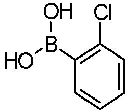 (3900-89-8) | 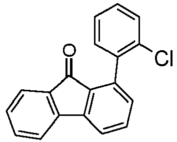 |
| 7e | 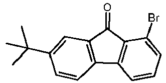 | 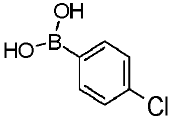 | 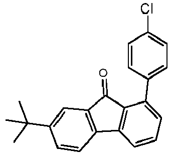 |
| 7f | 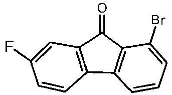 | 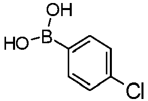 | 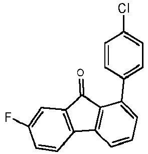 |

### Synthesis of 1-(4-Brom-phenyl)-fluoren-9-one 1-1 (8a)

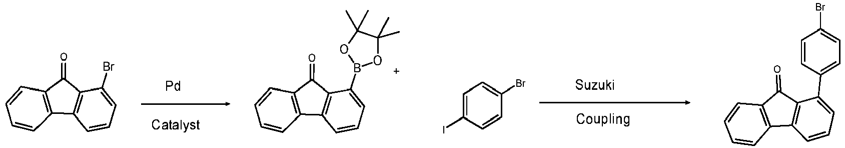

### Synthesis of boronester

10 g (39 mmol) of 1-bromofluorenone, 14.7 g (58 mmol) of bis(pinacolato)diborane and 12.5 g (127 mmol) of potassium acetate are suspended in 300 ml of dioxane. 1.6 g (1.9 mmol) of 1,1-bis(diphenyl-phosphino)ferrocenepalladium(II) dichloride complex with DCM are added to this suspension. The reaction mixture is heated under reflux for 16 h. After cooling, the organic phase is separated off, washed three times with 400 ml of water and subsequently evaporated to dryness. The residue is recrystallised from toluene (6 g, 51% yield).

### Synthesis of 8a

20 g (69 mmol) of 1-Bromo-4-iodo-benzene, 21.1 g (69 mmol) of 1-pinacolboron ester -fluoren-9-one and 2.4 g (2.1 mmol) of Pd(Ph₃P)₄ are suspended in 300 ml of THF. 283 ml of 2 M potassium carbonate solution are slowly added to this suspension, and the reaction mixture is heated under reflux for 16 h. After cooling, the organic phase is separated off, filtered through silica gel, washed three times with 300 ml of water and subsequently evaporated to dryness. The residue is purified by crystallisation with MeOH. Yield: 19 g (54 mmol), 78 % of theory, purity according to HPLC >98%.

The following compounds are prepared analogously:

| | | | | |
|---|---|---|---|---|
| 8b | 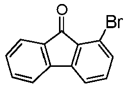 | 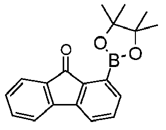 | 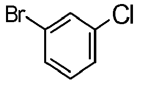 | 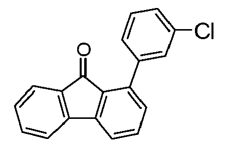 |
| 8c | 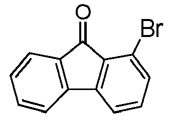 | 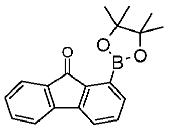 | 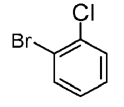 | 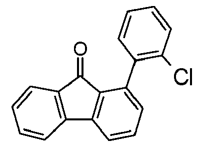 |
| 8d | 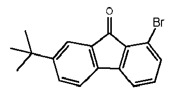 | 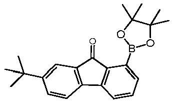 | 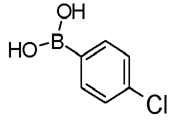 | 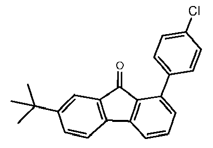 |
| 8e | 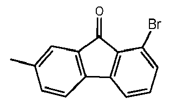 | 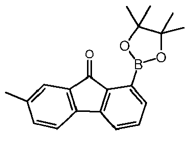 | 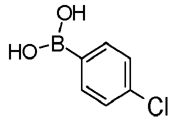 | 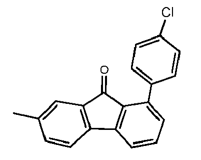 |
| 8f | 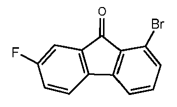 | 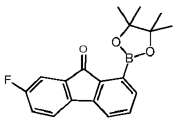 | 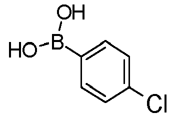 | 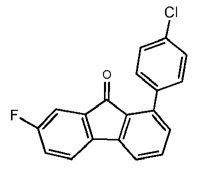 |

### Synthesis of 1 -(4-((1,1 '-biphenyl-4-yl)-(9,9-dimethyl-9H-fluoren-2-yl)amino)phenyl)-9H-Fluoren-9-one 9a

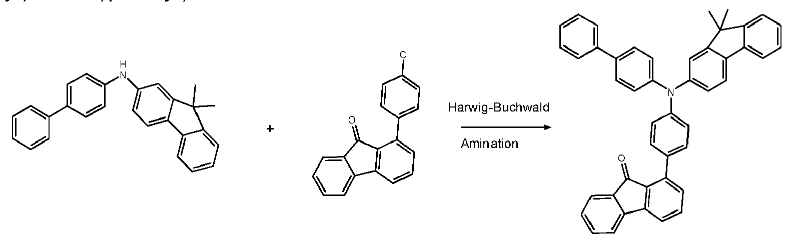

Tri-tert-butylphosphine (4.5 ml of a 1.0 M solution in toluene, 1,9 mmol) and 0,98 g (1 mmol) of Pd₂(dba)₃ and sodium *tert*-butoxide (5,1 g, 50 mmol) are added to a solution of 1-biphenyl-yl-(9,9-dimethyl-9*H*-fluoren-2-yl)amine (32 g, 90 mmol), 1-1-(4-chlor-phenyl)-fluoren-9-one, (25 g, 86 mmol) in degassed toluene (200 ml), and the mixture is heated under reflux overnight. The reaction mixture is cooled to room temperature, extended with toluene and filtered through Celite. The filtrate is evaporated in vacuo, and the residue is crystallised from toluene/heptane The product is isolated in the form of a pale-yellow solid (43 g, 81 % of theory).

| Ex. | Fluorenone | Amine | Product: |
|---|---|---|---|
| | | | 1-Amine-fluorenone |
| 9b | 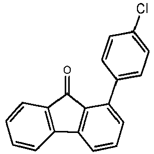 | 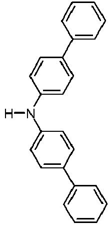 | 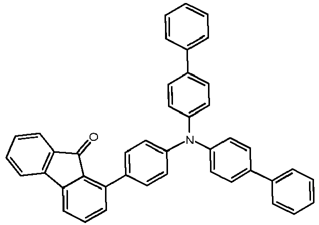 |
| 9c | 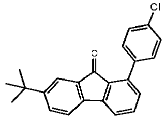 | 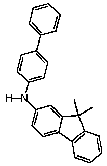 | 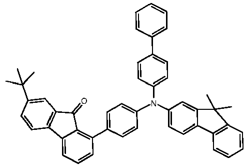 |
| 9d | 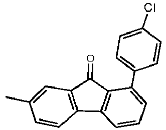 | 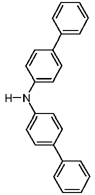 | 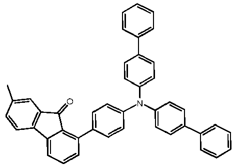 |
| 9e | 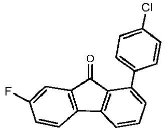 | 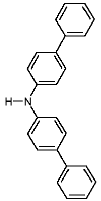 | 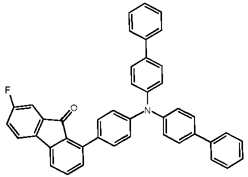 |
| 9f | 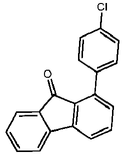 | 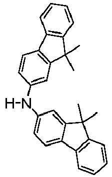 | 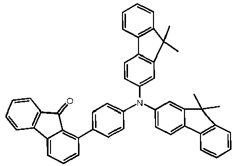 |
| 9g | 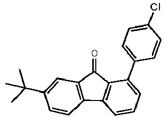 | 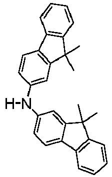 | 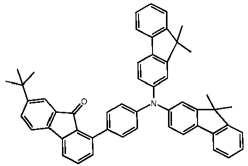 |
| 9h | 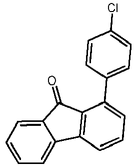 | 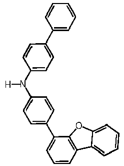 | 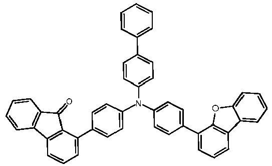 |
| 9i | 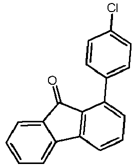 | 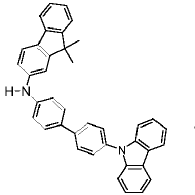 | 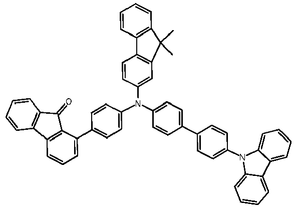 |
| 9j | 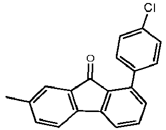 | 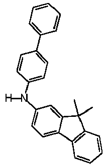 | 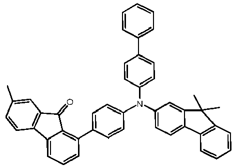 |
| 9k | 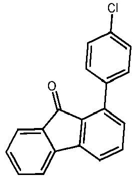 | 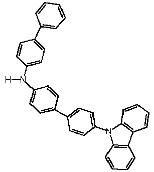 | 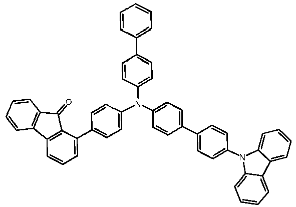 |
| 9l | 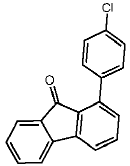 | 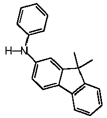 | 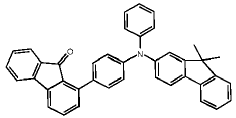 |

### Synthesis of N-((1,1 '-biphenyl)-4-yl)N-(4-(2',7'-di-tert-butyl-9,9'-spirobi(fluorene)-1-yl)phenyl)-9,9-dimethylfluoren-2-amine 10a

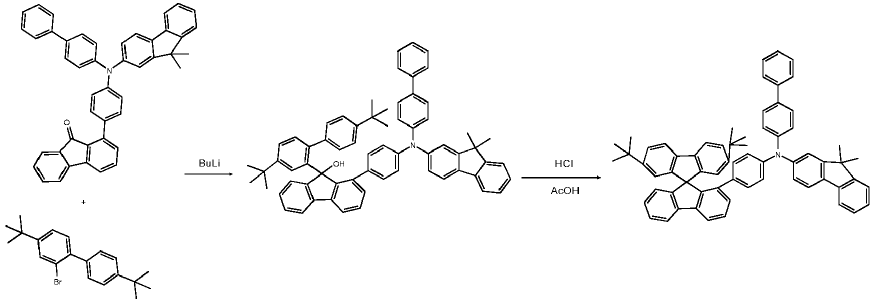

A solution of 4, 4'-di-t-Butyl-2,Br-biphenyl (17 g, 49 mmol) in THF (90ml) is treated with 25 mL of *n*-BuLi (2,1 M in hexane, 50 mmol) under argon at - 78 °C. The mixture is stirred for 30 minutes. A solution of 1-(4-((1,1'-biphenyl-4-yl)-(9,9-dimethyl-9H-fluoren-2-yl)amino)phenyl)-9H-Fluoren-9-one (27 g, 44 mmol) in 90 mL THF is added dropwise. The reaction proceeds at -78 °C for 30 minutes and then is stirred at room temperature overnight. The reaction is quenched with water and extracted with ethyl acetate. The intermediate alcohol is obtained after the solvent is removed (31 g, 76%). Without further purification, a mixture of the alcohol, acetic acid (700 mL) and concentrated HCl (62 mL) is refluxed for 2 hours. After cooling, the mixture is filtered and washed with water. The residue is crystallised from toluene. The crude product is extracted in a Soxhlet extractor (toluene) and purified by zone sublimation in vacuo. The product is isolated in the form of a pale-yellow solid (13 g, 34% of theory, purity > 99.99% according to HPLC).

The following compounds are prepared analogously:

| Ex. | Product: | Br-Biphenyl | Product: |
|---|---|---|---|
| | 1-Amine-fluorenone | | |
| 10b | 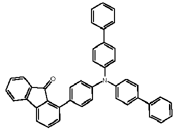 | 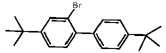 70728-89-1 | 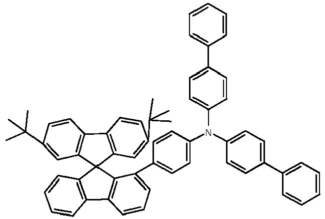 |
| 10c | 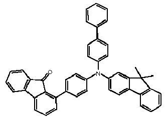 | 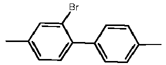 132462-55-6 | 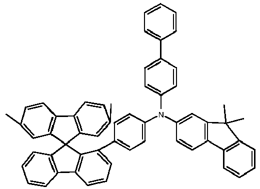 |
| 10d | 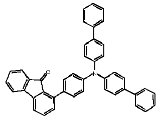 | 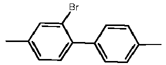 132462-55-6 | 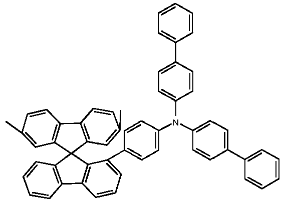 |
| #10e | 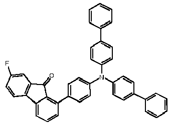 | 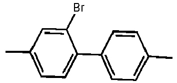 | 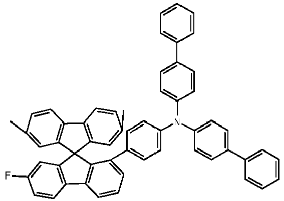 |
| 10f | 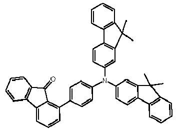 | 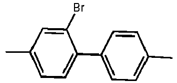 | 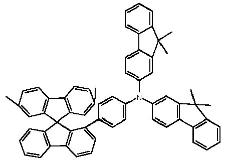 |
| #10g | 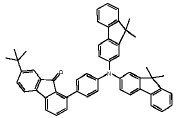 | 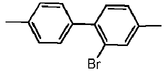 | 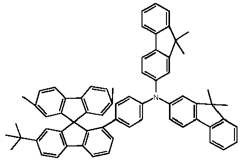 |
| 10h | 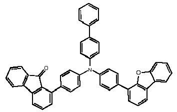 | 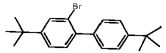 | 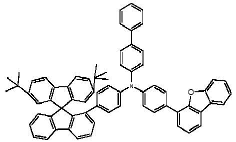 |
| 10i | 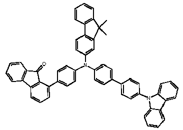 | 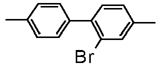 [132462-55-6] | 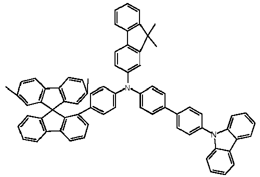 |
| #10j | 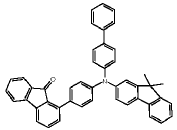 | 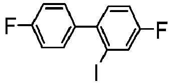 [1214351-66-2] | 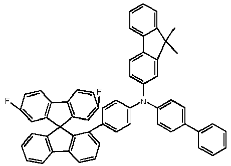 |
| 10k | 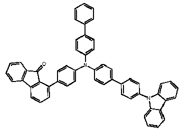 | 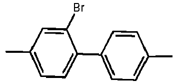 132462-55-6 | 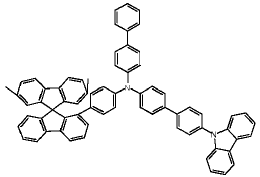 |
| 10l | 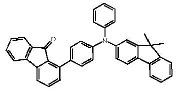 | 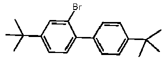 70728-89-1 | 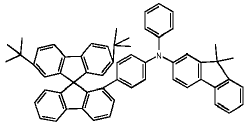 |
| 10m | 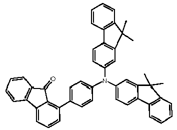 | 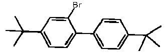 | 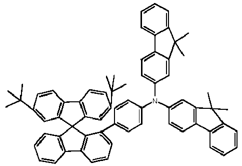 |

### A-6) Route 6

### Synthesis of 2,7-di-tert-butyl-8'-(4-chlorophenyl)-9,9'-spirobifluorene 11a

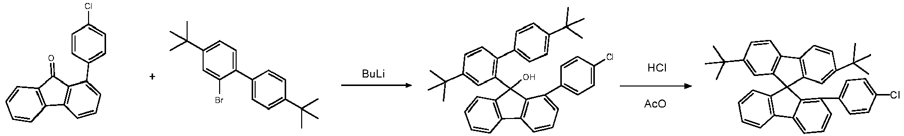

20 g (58 mmol) of 2-Br -4, 4'-di-tert-Butyl-1,1' -biphenyl are initially introduced in 400 ml of THF at -78°C. 30 ml of BuLi (2 M in hexane) are added dropwise at this temperature. After 1 hour, 16.9 g (58 mmol) of 1-(4-chloro-phenyl)-fluoren-9-one in 200 ml of THF are added dropwise. The batch is left to stir overnight at room temperature, added to ice-water and extracted with dichloromethane. The combined organic phases are washed with water and dried over sodium sulfate. The solvent is removed in vacuo, and the residue is, without further purification, heated under reflux at 100°C overnight with 30 ml of HCl and 300 ml of AcOH. After cooling, the precipitated solid is filtered off with suction, washed once with 100 ml of water, three times with 100 ml of ethanol each time and subsequently recrystallised from heptane. Yield: 17 g (56 mmol), 53%; purity approx. 98% according to ¹H-NMR.

The following compounds are synthesized analogously:

| **Example** | **Reagent 1** | **Reagent 2** | **Product** |
|---|---|---|---|
| 11b | 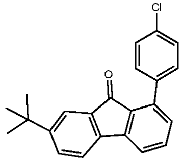 | 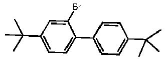 70728-89-1 | 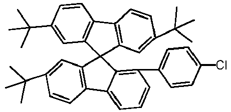 |
| 11c | 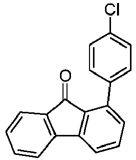 | 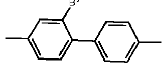 132462-55-6 | 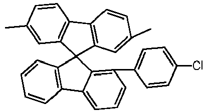 |
| 11d | 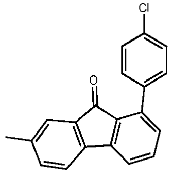 | 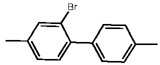 132462-55-6 | 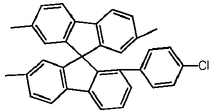 |
| 11e | 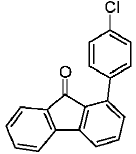 | 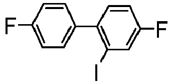 [1214351-66-2] | 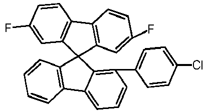 |
| 11f | 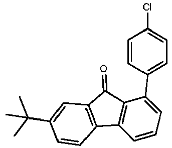 | 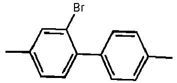 | 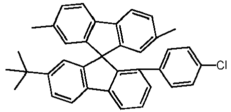 |
| 11g | 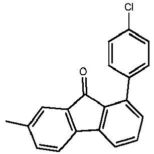 | 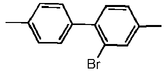 | 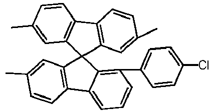 |
| 11h | 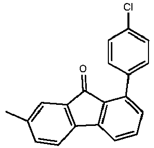 | 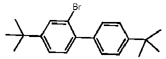 | 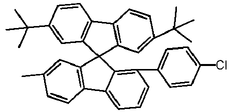 |

### Synthesis of 2,7-di-tert-butyl-8'-(4-chlorophenyl)-9,9'-spirobifluorene 12a

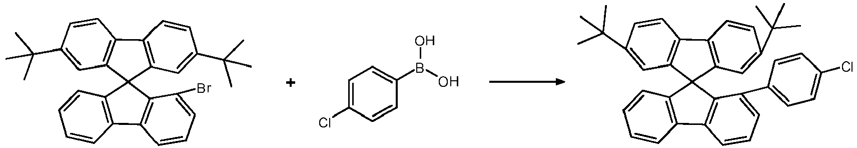

10.7 g (69 mmol) of 4-chlorophenylboronic acid, 35 g (69 mmol) of 2',7'-di-tert-butyl-1-brom-spirofluorene and 5,4 g (5 mmol) of Pd(Ph₃P)₄ are suspended in 600 ml of THF. 155 ml of 2 M potassium carbonate solution are slowly added to this suspension, and the reaction mixture is heated under reflux for 16 h. After cooling, the organic phase is separated off, filtered through silica gel, washed three times with 500 ml of water and subsequently evaporated to dryness. The residue is purified by crystallisation with MeOH. Yield: 29 g (65 mmol), 94 % of theory, purity according to HPLC >98%.

| **Example** | **Reagent 1** | **Reagent 2** | **Product** |
|---|---|---|---|
| 12b | 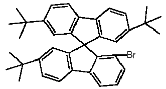 | 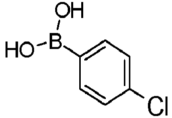 | 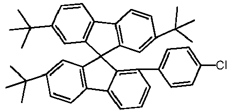 |
| 12c | 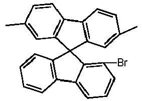 | 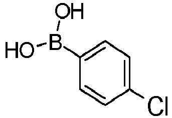 | 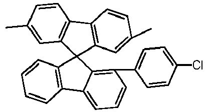 |
| 12d | 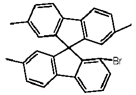 | 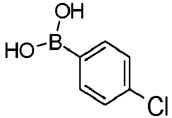 | 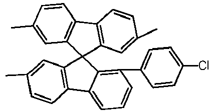 |
| 12e | 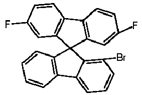 | 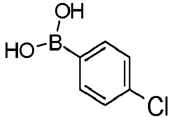 | 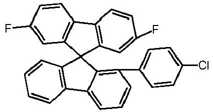 |
| 12f | 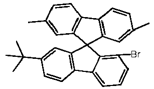 | 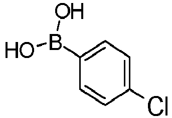 | 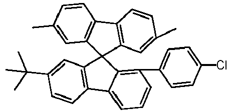 |
| 12g | 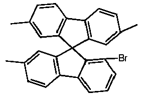 | 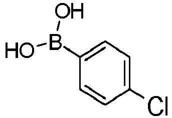 | 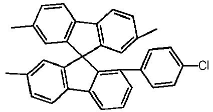 |
| 12h | 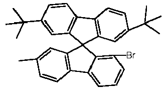 | 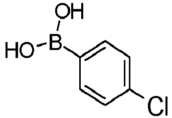 | 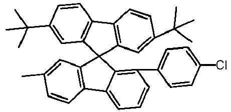 |

### Synthesis of 2,7-di-tert-butyl-8'-(4-chlorophenyl)-9,9'-spirobifluorene

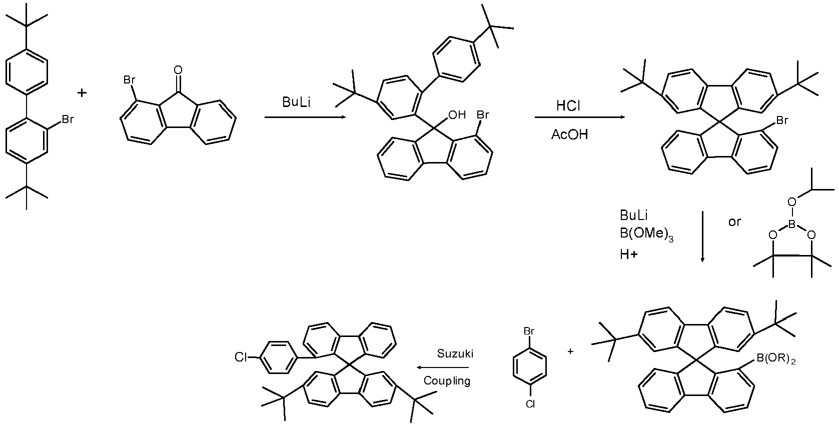

### Synthesis of 2-{2',7'-di-tert-butyl-9,9'-spirobi[fluorene]-8-yl}-4,4,5,5-tetramethyl-1,3,2-dioxaborolane ester 13a

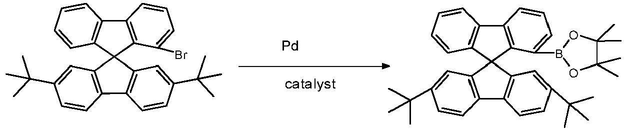

50 g (99 mmol) of 2',7'-di-tert-butyl-1-brom-spirofluorene, 32 g (123 mmol) of bis(pinacolato)diborane and 30 g (309 mmol) of potassium acetate are suspended in 800 ml of dioxane. 2.5 g (3.09 mmol) of 1,1-bis(diphenyl-phosphino)ferrocenepalladium(II) dichloride complex with DCM are added to this suspension. The reaction mixture is heated under reflux for 16 h. After cooling, the organic phase is separated off, washed three times with 400 ml of water and subsequently evaporated to dryness. The residue is recrystallised from toluene (52 g, 95% yield).

The following compounds are prepared analogously:

| **Example** | **Reagent 1** | **Product** |
|---|---|---|
| 13b | 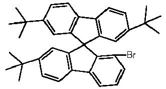 | 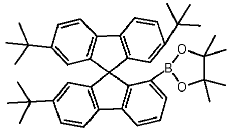 |
| 13c | 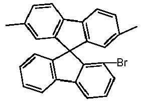 | 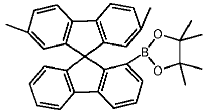 |
| 13d | 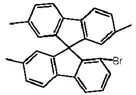 | 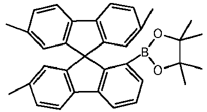 |
| 13e | 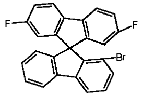 | 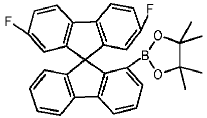 |

### Synthesis of 2-{2',7'-di-tert-butyl-9,9'-spirobi[fluorene]-8-yl}-4,4,5,5-tetramethyl-1,3,2-dioxaborolane ester 14a

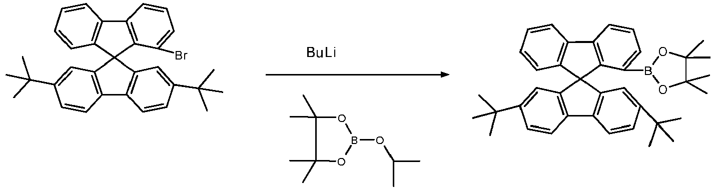

50 g (93 mmol) of 2',7'-di-tert-butyl-1-brom-spirofluorene are initially introduced in 50 ml of THF at -20°C. 56 ml of BuLi (2 M in hexane) are added dropwise at this temperature. After 4 hours, 18.6 g (100 mmol) of isopropoxytetramethyldioxaborolane are added dropwise. The batch is left to stir overnight at room temperature. When the reaction is complete, water and ethyl acetate are added, and the organic phase is separated off, dried and evaporated. The residue is purified by chromatography on silica gel. Yield: 44 g (79 mmol), 85% of theory, purity according to HPLC >98%.

The following compounds are prepared analogously:

| **Example** | **Reagent 1** | **Reagent 2** | **Product** |
|---|---|---|---|
| 14b | 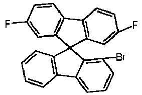 | 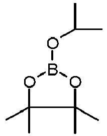 | 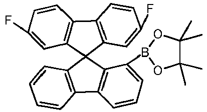 |
| 14c | 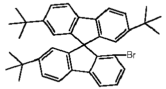 | 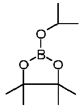 | 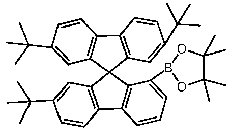 |
| 14d | 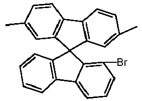 | 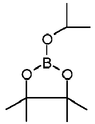 | 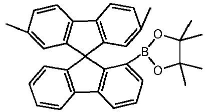 |
| 14e | 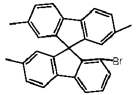 | 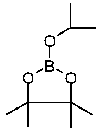 | 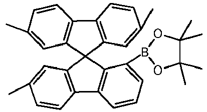 |

### Synthesis of 2,7-di-tert-butyl-8'-(4-chlorophenyl)-9,9'-spirobifluorene 15a

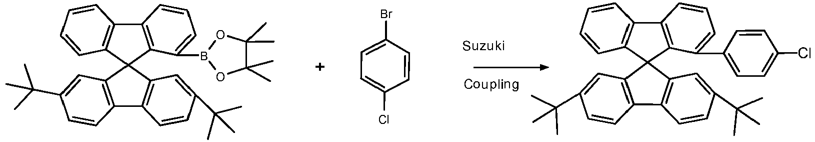

20.3 g (37 mmol) of 2-{2',7'-di-tert-butyl-9,9'-spirobi[fluorene]-8-yl}-4,4,5,5-tetramethyl-1 ,3,2-dioxaborolane ester and 8.8 g (46.3 mmol) of chlorine derivative are suspended in 300 ml of dioxane and 14.1 g of caesium fluoride (92.6 mmol). 4.1 g (5.56 mmol) of bis-(tricyclohexylphosphine)palladium dichloride are added to this suspension, and the reaction mixture is heated under reflux for 24 h. After cooling, the organic phase is separated off, filtered through silica gel, washed three times with 100 ml of water and subsequently evaporated to dryness. The crude product is recrystallised from heptane/toluene The yield is 15.8 g (78% of theory).

The following compounds are prepared analogously:

| **Example** | **Reagent 1** | **Reagent 2** | **Product** |
|---|---|---|---|
| 15b | 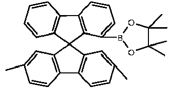 | 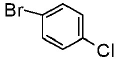 | 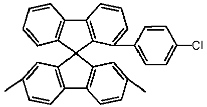 |
| 15c | 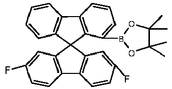 | 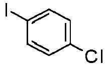 | 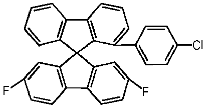 |
| 15d | 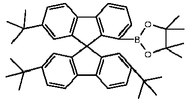 | 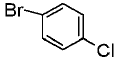 | 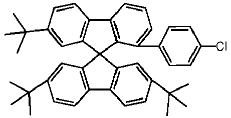 |
| 15e | 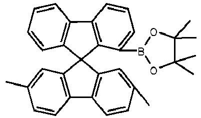 | 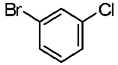 | 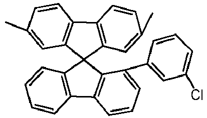 |
| 15f | 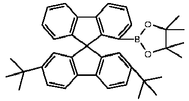 | 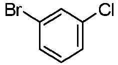 | 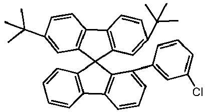 |

### Synthesis of N-((1,1'-biphenyl)-4-yl)N-(4-(2',7'-di-tert-butyl-9,9'-spirobi(fluorene)-1-yl)phenyl)-9,9-dimethylfluoren-2-amine 16a

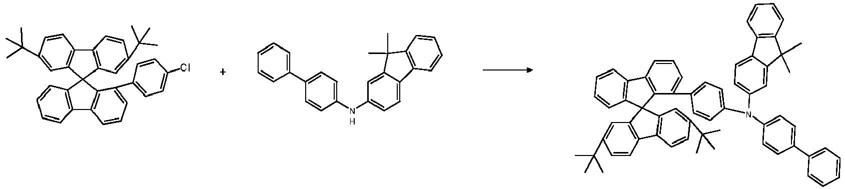

10.1 g (28 mmol) of biphenyl-4-yl-(9,9-dimethyl-9H-fluoren-2-yl)amine and 14.5 g (27 mol) of the 2,7-di-tert-butyl-8'-(4-chlorophenyl)-9,9'-spirobifluorene are dissolved in 225 ml of toluene. The solution is degassed and saturated with N₂. 2.1 ml (2.1 mmol) of a 10% tri-tert-butylphosphine solution and 0,98 g (1 mmol) of Pd₂(dba)₃ are then added, and 5.1 g of sodium tert-butoxide (53 mmol) are subsequently added. The reaction mixture is heated at the boil under a protective atmosphere for 5 h. The mixture is subsequently partitioned between toluene and water, the organic phase is washed three times with water and dried over Na₂SO₄ and evaporated in a rotary evaporator. After filtration of the crude product through silica gel with toluene, the residue which remains is recrystallised from heptane/toluene and finally sublimed in a high vacuum. The purity is 99.9% (HPLC). The yield of compound is 11.5 g (48% of theory).

The following compounds are also prepared analogously to the synthesis of compound 1.

| Ex. | Starting material 1 | Starting material 2 | Product |
|---|---|---|---|
| 16b | 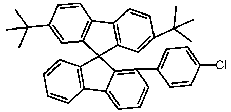 | 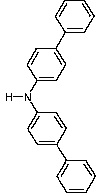 | 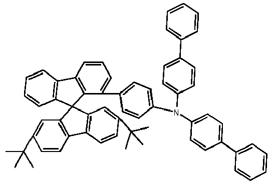 |
| 16c | 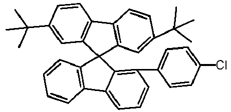 | 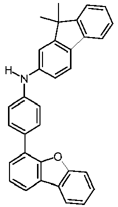 | 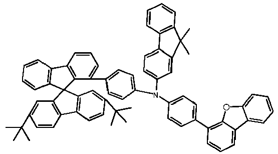 |
| 16d | 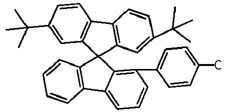 | 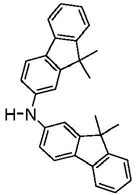 | 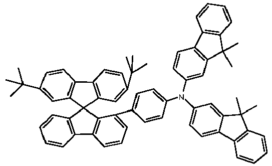 |
| 16e | 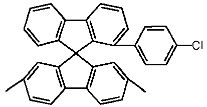 | 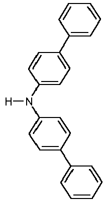 | 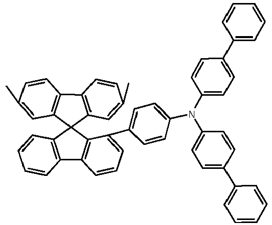 |
| 16f | 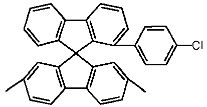 | 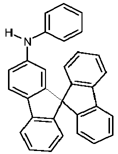 | 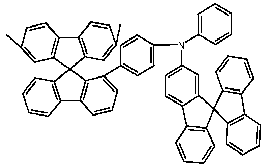 |
| 16g | 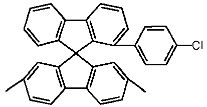 | 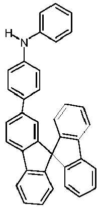 | 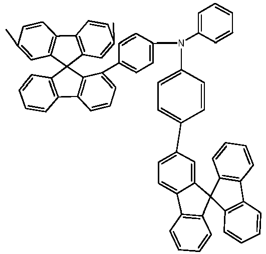 |
| #16h | 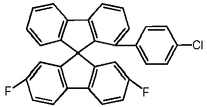 | 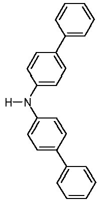 | 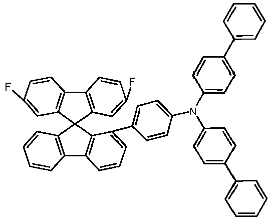 |
| 16i | 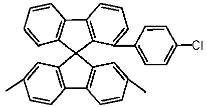 | 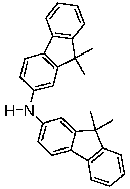 | 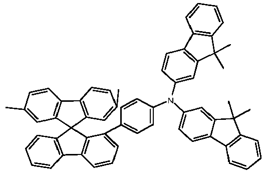 |
| 16j | 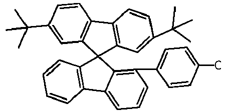 | 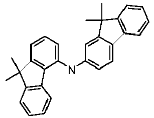 | 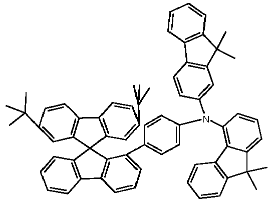 |
| 16k | 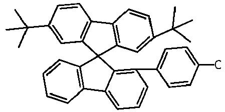 | 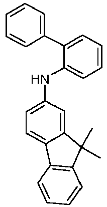 | 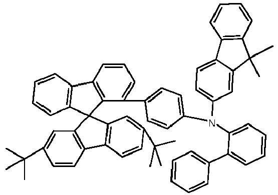 |
| 16l | 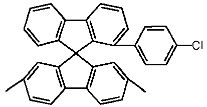 | 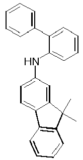 | 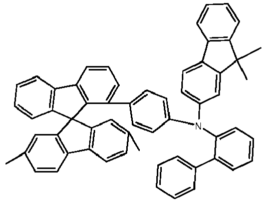 |

### B) Use examples

### 1) EBL use of compounds

A fluorescent blue emitting OLED comprising the compound HTM according to the present application in the EBL is prepared. The OLED has the following stack structure:
Anode / HIM:F4TCNQ (5%) (20nm) / HIM (180nm) / HTM (10nm) / H:SEB (5%) (20nm) / ETM:LiQ (50%) (30nm) / LiQ (1 nm) / cathode.

In the above stack, the anode consists of a glass plate coated with a 50 nm layer of structured ITO. The cathode is made of a 100 nm thick layer of Al. The structures of the materials which are present in the different layers are given in Table 1. The materials are deposited by thermal vapor deposition in a vacuum chamber. If two materials are present in a layer, the percentage given above is the proportion of the second material in percent by volume.

The OLED is electrically driven, and is characterized by establishing the following parameters: 1) external quantum efficiency (EQE, measured in per cent) is determined as a function of luminance, calculated from current-voltage-luminance characteristics (IUL characteristics) assuming Lambertian radiation characteristics, at a current density of 10 mA/cm²; 2) lifetime LD80 @ 5000cd/m², which is the time until the OLED has dropped from its starting brightness of 5000 cd/m² to 80% of its starting brightness; 3) operating voltage at 10mA/cm², and 4) LD80 @ 60 mA/cm², which is the time until the OLED has dropped from its starting brightness at 60 mA/cm² to 80% of its starting brightness.

For the OLED, the following values are measured: EQE @ 10 mA/cm²: 7.6 %, lifetime LD80 @ 5000cd/m²: 320 h, operating voltage at 10 mA/cm²: 4.0 V.

Compounds 2a-2c, 2e-2l, 4a-4m, 5a-5e, 6a-6e, 10a-10m and 16a-16l of the synthesis examples give results which are similar to the ones obtained with compound HTM.

### 2) HTL use of compounds

A fluorescent blue emitting OLED comprising the compound HTM-1 according to the present application in the HIL and the HTL is prepared. The OLED has the following stack structure:
Anode / HTM-1:F4TCNQ (5%) (20nm) / HTM-1 (180nm) / EBM (10nm) / H:SEB (5%) (20nm) / ETM:LiQ (50%) (30nm) / LiQ (1 nm) / cathode.

The preparation of the OLED and of the electrode layers, and the characterization is the same as described above in 1).

For the OLED, the following values are measured: EQE @ 10 mA/cm²: 8.2 %, lifetime LD80 @ 60 mA/cm²: 340 h, operating voltage at 10 mA/cm²: 4.2 V.

Compounds 2a-2l, 4a-4f, 4h-4m, 5a-5e, 6a-6e, 10a-10m and 16a-16l of the synthesis examples give results which are similar to the ones obtained with compound HTM-1.

### 3) Comparison of compound EBM-1 according to the application with compound EBM-2

OLEDs are prepared which have the following stack structure:

### Example according to the invention:

Anode / HIM:F4TCNQ (5%) (20nm) / HIM (180nm) / EBM-1 (10nm) / H:SEB (5%) (20nm) / ETM:LiQ (50%) (30nm) / LiQ (1 nm) / cathode.

### Comparative example:

As above, only EBM-1 is replaced by EBM-2.

The preparation of the OLEDs and of the electrode layers, and the characterization is the same as described above in 1).

For the OLED comprising the compound EBM-1, the following values are measured: EQE @ 10 mA/cm²: 8.2 %, lifetime LD80 @ 60 mA/cm²: 103 h, operating voltage at 10 mA/cm²: 4.3 V.

For the OLED comprising the compound EBM-2 (comparative example), the following values are measured: EQE @ 10 mA/cm²: 8.1 %, lifetime LD80 @ 60 mA/cm²: 81 h, operating voltage at 10 mA/cm²: 4.1 V.

This shows in a direct comparison of performance, that an OLED comprising the compound EBM-1 according to the present application, shows strongly improved lifetime, compared to an OLED comprising the compound EBM-2 (comparative example). The other parameters efficiency and operating voltage remain similar.

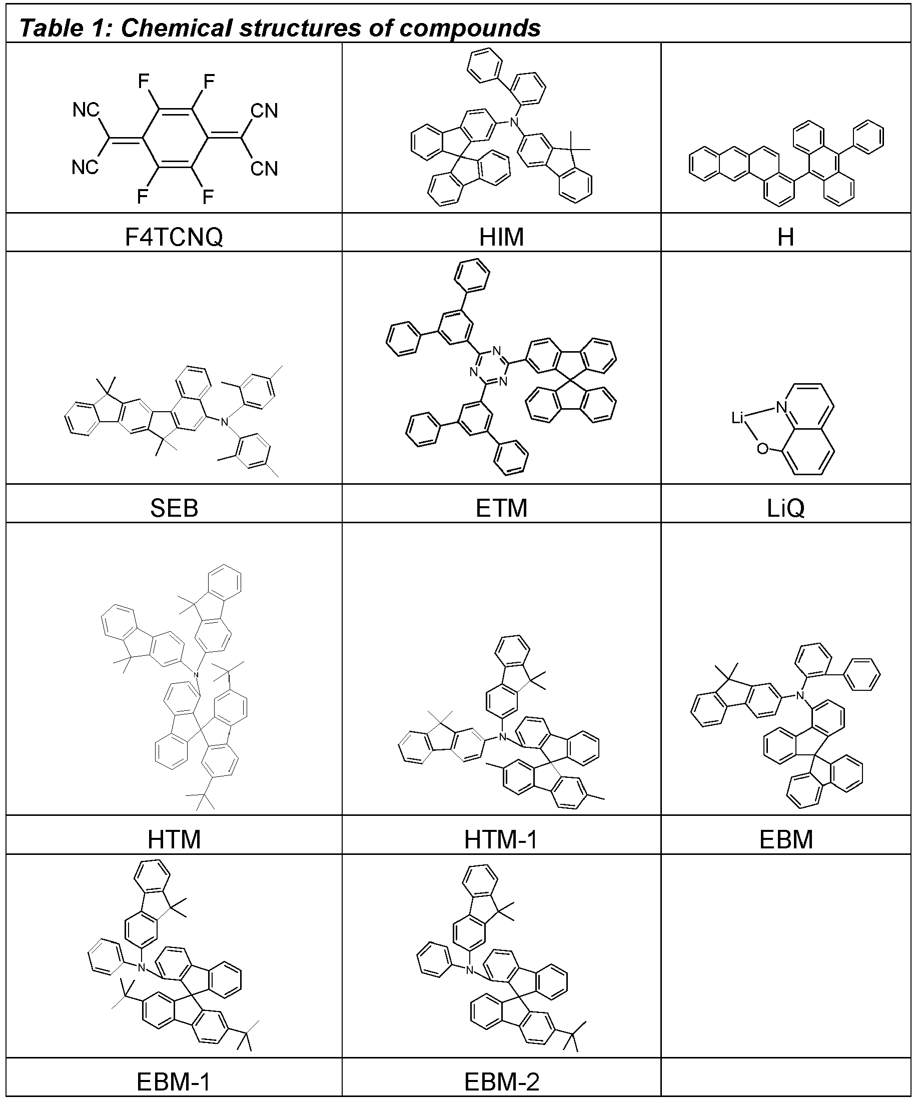

## Claims

1. Compound of a formula (IA) or (IB) 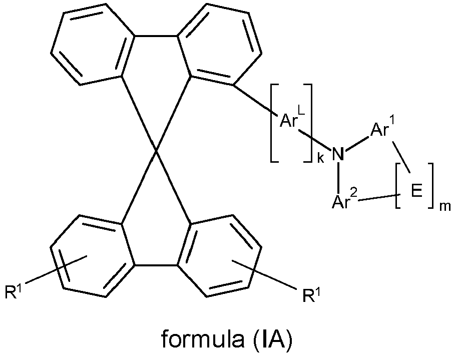 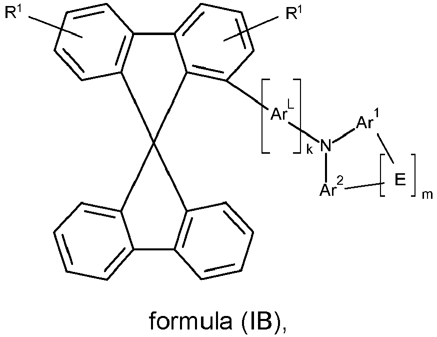 where the following applies to the variables:
Ar^{L} is selected from aromatic ring systems having 6 to 30 aromatic ring atoms, which may be substituted by one or more radicals R³, and
heteroaromatic ring systems having 5 to 30 aromatic ring atoms, which may be substituted by one or more radicals R³;
Ar¹ and Ar² are, identically or differently, selected from aromatic ring systems having 6 to 30 aromatic ring atoms, which may be substituted by one or more radicals R³, and heteroaromatic ring systems having 5 to 30 aromatic ring atoms, which may be substituted by one or more radicals R³;
E is a single bond or is a divalent group selected from C(R³)₂, N(R³), O, and S;
R¹ is, identically or differently on each occurrence, selected from straight-chain alkyl, alkoxy or thioalkyl groups having 1 to 20 C atoms, which may optionally be substituted by one or more groups F, and from branched or cyclic alkyl, alkoxy or thioalkyl groups having 3 to 20 C atoms, which may optionally be substituted by one or more groups F;
R³ is, identically or differently at each occurrence, selected from H, D, F, C(=O)R⁴, CN, Si(R⁴)₃, N(R⁴)₂, P(=O)(R⁴)₂, OR⁴, S(=O)R⁴, S(=O)₂R⁴, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R³ may be connected to each other to form a ring; where the said alkyl, alkoxy, alkenyl and alkynyl groups and the said aromatic and heteroaromatic ring systems may in each case be substituted by one or more radicals R⁴, and where one or more CH₂ groups in the said alkyl, alkoxy, alkenyl and alkynyl groups may in each case be replaced by -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO or SO₂;
R⁴ is, identically or differently at each occurrence, selected from H, D, F, C(=O)R⁵, CN, Si(R⁵)₃, N(R⁵)₂, P(=O)(R⁵)₂, OR⁵, S(=O)R⁵, S(=O)₂R⁵, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R⁴ may be connected to each other to form a ring; where the said alkyl, alkoxy, alkenyl and alkynyl groups and the said aromatic and heteroaromatic ring systems may in each case be substituted by one or more radicals R⁵, and where one or more CH₂ groups in the said alkyl, alkoxy, alkenyl and alkynyl groups may in each case be replaced by -R⁵C=CR⁵-, -C=C-, Si(R⁵)₂, C=O, C=NR⁵, -C(=O)O-, -C(=O)NR⁵-, NR⁵, P(=O)(R⁵), -O-, -S-, SO or SO₂;
R⁵ is selected, identically or differently at each occurrence, from H, D, F, CN, alkyl groups having 1 to 20 C atoms, aromatic ring systems having 6 to 40 C atoms, or heteroaromatic ring systems having 5 to 40 aromatic ring atoms; where two or more radicals R⁵ may be connected to each other to form a ring; and where the said alkyl groups, aromatic ring systems and heteroaromatic ring systems may be substituted by F and CN;
k is 0 or 1; where in the case of k=0, the group Ar^{L} is not present and the nitrogen atom and the spirobifluorene group are directly connected;
m is 0 or 1, where in the case of m=0, the group E is not present and the groups Ar¹ and Ar² are not connected.

2. Compound according to claim 1, **characterized in that** index k is 0, so that the group Ar^{L} is not present, and the spirobifluorene and the nitrogen atom of the amine are directly connected with each other.

3. Compound according to claim 1 or 2, **characterized in that** groups Ar¹ and Ar² are, identically or differently, selected from radicals derived from a group selected from phenyl, biphenyl, terphenyl, quaterphenyl, naphthyl, fluorenyl, benzofluorenyl, spirobifluorenyl, indenofluorenyl, dibenzofuranyl, dibenzothiophenyl, carbazolyl, benzofuranyl, benzothiophenyl, indolyl, quinolinyl, pyridyl, pyrimidyl, pyrazinyl, pyridazinyl and triazinyl, where the groups may each be substituted by one or more radicals R³, or from combinations of 2 or 3 radicals derived from those groups, where the groups may each be substituted by one or more radicals R³.

4. Compound according to one or more of claims 1 to 3, **characterized in that** the groups R¹ conform to one of the following formulae
| | | |
|---|---|---|
| -CH₃ | -C(CH₃)₃ | -CH₂CH₃ |
| R¹-1 | R¹-2 | R¹-3 |
| -CH₂CH(CH₃)₂ | -CF₃ | -CF₂CF₃ |
| R¹-4 | R¹-5 | R¹-6 |
| -OCF₃ | -SCF₃ | -SF₅ |
| R¹-7 | R¹-8 | R¹-9 |
| -OCF₂CF₃ | -SCF₂CF₃ | 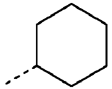 |
| R¹-10 | R¹-11 | R¹-12 |
| 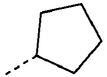 | 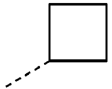 | 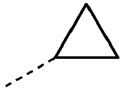 |
| R¹-13 | R¹-14 | R¹-15 |
| -OCH₃ | -SCH₃ | |
| R¹-22 | R¹-23 | |

5. Compound according to one or more of claims 1 to 4, **characterized in that** it conforms to one of formulae (I-A-1) to (I-A-5) and (I-B-1) to (I-B-5) 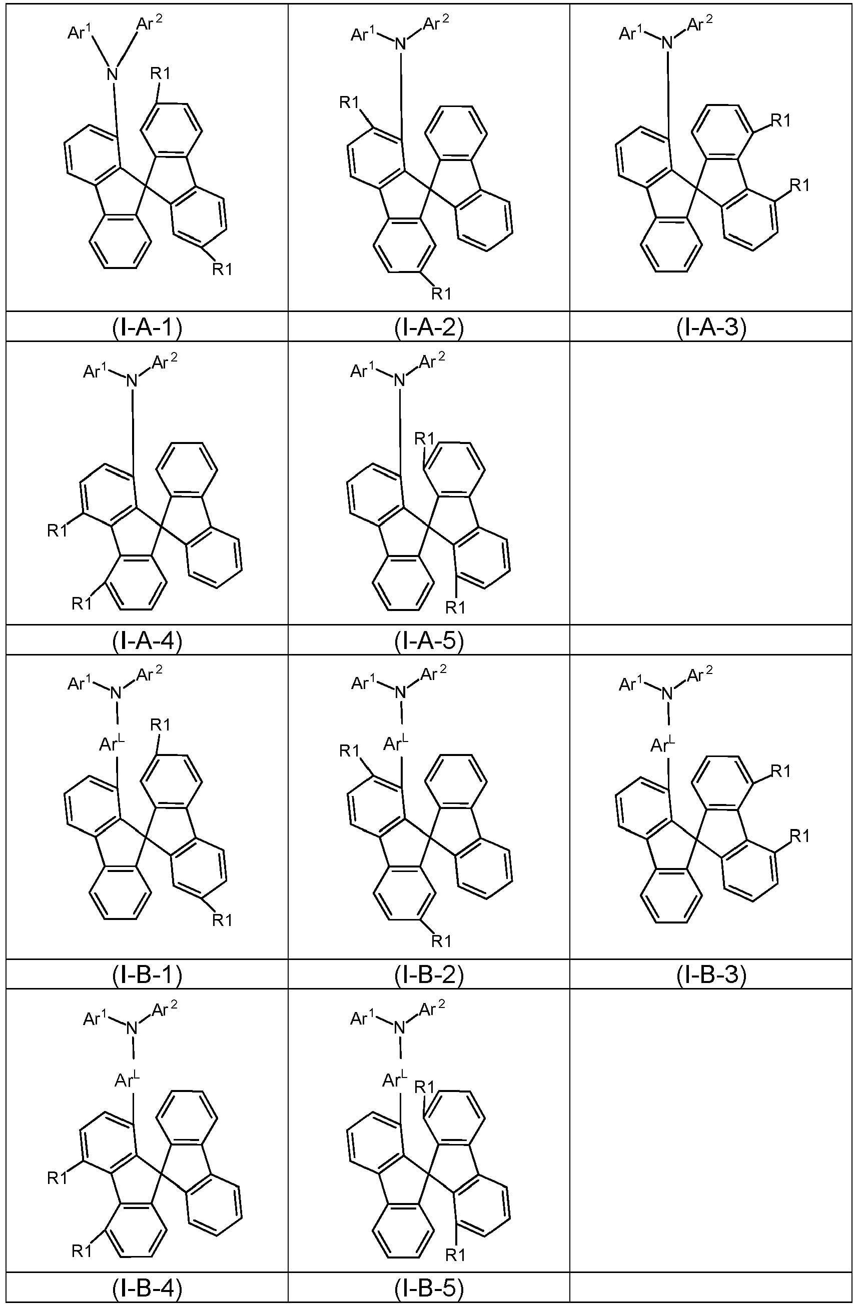 where the variables are defined in one or more of claims 1 to 4.

6. Process for preparation of a compound according to one or more of claims 1 to 5, **characterized in that** it comprises the reactions steps
1) metallation of a biphenyl derivative which has a reactive group in a position which is ortho to the phenyl-phenyl bond;
2) addition of the metallated biphenyl derivative to a fluorenone derivative which has a group A in its 1-position; where the group A is selected from i) X, or ii) -Ar-X, or iii) -NAr₂, or iv) -Ar-NAr₂, where Ar is aromatic or heteroaromatic group, and where X is a reactive group; and
3) cyclisation of the resulting addition product to a spirobifluorene derivative under acidic conditions or with a Lewis acid.

7. Oligomer, polymer or dendrimer, comprising one or more compounds of formula (IA) or (IB) according to one or more of claims 1 to 5, where the bond(s) to the polymer, oligomer or dendrimer may be localised at any desired positions in formula (IA) or (IB) substituted by R¹ or R³.

8. Formulation, comprising at least one compound of formula (IA) or (IB) according to one or more of claims 1 to 5 or at least one polymer, oligomer or dendrimer according to claim 7, and at least one solvent.

9. Electronic device, comprising at least one compound according to one or more of claims 1 to 5, or at least one polymer, oligomer or dendrimer according to claim 7.

10. Electronic device according to claim 9, **characterized in that** it is an organic electroluminescent device, comprising anode, cathode and at least one emitting layer, where at least one organic layer of the device, which is an emitting layer, a hole transport layer, an electron blocking layer or a hole injection layer, comprises the at least one compound.

11. Use of a compound according to one or more of claims 1 to 5, or of a polymer, oligomer or dendrimer according to claim 7, in an electronic device.

## Patentansprüche

1. Verbindung einer Formel (IA) oder (IB) 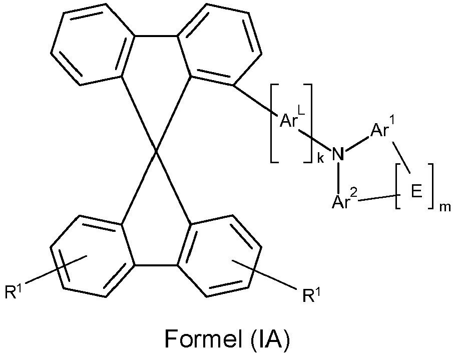 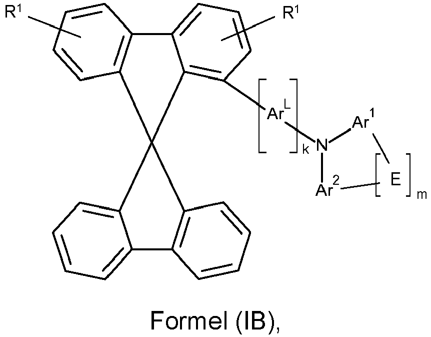 wobei für die Variablen Folgendes gilt:
Ar^{L} ist ausgewählt aus aromatischen Ringsystemen mit 6 bis 30 aromatischen Ringatomen, die durch einen oder mehrere Reste R³ substituiert sein können, und heteroaromatischen Ringsystemen mit 5 bis 30 aromatischen Ringatomen, die durch einen oder mehrere Reste R³ substituiert sein können;
Ar¹ und Ar² sind gleich oder verschieden ausgewählt aus aromatischen Ringsystemen mit 6 bis 30 aromatischen Ringatomen, die durch einen oder mehrere Reste R³ substituiert sein können, und heteroaromatischen Ringsystemen mit 5 bis 30 aromatischen Ringatomen, die durch einen oder mehrere Reste R³ substituiert sein können;
E ist eine Einfachbindung oder ist eine zweiwertige Gruppe, die ausgewählt ist aus C(R³)₂, N(R³), O und S;
R¹ ist bei jedem Auftreten gleich oder verschieden ausgewählt aus geradkettigen Alkyl-, Alkoxy- oder Thioalkylgruppen mit 1 bis 20 C-Atomen, die gegebenenfalls durch eine oder mehrere Gruppen F substituiert sein können, und aus verzweigten oder cyclischen Alkyl-, Alkoxy- oder Thioalkylgruppen mit 3 bis 20 C-Atomen, die gegebenenfalls durch eine oder mehrere Gruppen F substituiert sein können;
R³ ist bei jedem Auftreten gleich oder verschieden ausgewählt aus H, D, F, C(=O)R⁴, CN, Si(R⁴)₃, N(R⁴)₂, P(=O)(R⁴)₂, OR⁴, S(=O)R⁴, S(=O)₂R⁴, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R³ miteinander zu einem Ring verbunden sein können; wobei die genannten Alkyl-, Alkoxy-, Alkenyl- und Alkinylgruppen und die genannten aromatischen und heteroaromatischen Ringsysteme jeweils durch einen oder mehrere Reste R⁴ substituiert sein können und wobei eine oder mehrere CH₂-Gruppen in den genannten Alkyl-, Alkoxy-, Alkenyl- und Alkinylgruppen jeweils durch -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können;
R⁴ ist bei jedem Auftreten gleich oder verschieden ausgewählt aus H, D, F, C(=O)R⁵, CN, Si(R⁵)₃, N(R⁵)₂, P(=O)(R⁵)₂, OR⁵, S(=O)R⁵, S(=O)₂R⁵, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁴ miteinander zu einem Ring verbunden sein können; wobei die genannten Alkyl-, Alkoxy-, Alkenyl- und Alkinylgruppen und die genannten aromatischen und heteroaromatischen Ringsysteme jeweils durch einen oder mehrere Reste R⁵ substituiert sein können und wobei eine oder mehrere CH₂-Gruppen in den genannten Alkyl-, Alkoxy-, Alkenyl- und Alkinylgruppen jeweils durch -R⁵C=CR⁵-, -C=C-, Si(R⁵)₂, C=O, C=NR⁵, -C(=O)O-, -C(=O)NR⁵-, NR⁵, P(=O)(R⁵), -O-, -S-, SO oder SO₂ ersetzt sein können;
R⁵ ist bei jedem Auftreten gleich oder verschieden ausgewählt aus H, D, F, CN, Alkylgruppen mit 1 bis 20 C-Atomen, aromatischen Ringsystemen mit 6 bis 40 C-Atomen oder heteroaromatischen Ringsystemen mit 5 bis 40 aromatischen Ringatomen; wobei zwei oder mehr Reste R⁵ miteinander zu einem Ring verbunden sein können; und wobei die genannten Alkylgruppen, aromatischen Ringsysteme und heteroaromatischen Ringsysteme durch F und CN substituiert sein können;
k ist 0 oder 1; wobei für k=0 die Gruppe Ar^{L} nicht vorhanden ist und das Stickstoffatom und die Spirobifluorengruppe direkt verbunden sind;
m ist 0 oder 1; wobei für m=0 die Gruppe E nicht vorhanden ist und die Gruppen Ar¹ und Ar² nicht verbunden sind.

2. Verbindung nach Anspruch 1, **dadurch gekennzeichnet, dass** der Index k 0 ist, so dass die Gruppe Ar^{L} nicht vorhanden ist und das Spirobifluoren und das Stickstoffatom des Amins direkt miteinander verbunden sind.

3. Verbindung nach Anspruch 1 oder 2, **dadurch gekennzeichnet, dass** die Gruppen Ar¹ und Ar² gleich oder verschieden ausgewählt sind aus Resten, die sich von einer Gruppe ableiten, die ausgewählt ist aus Phenyl, Biphenyl, Terphenyl, Quaterphenyl, Naphthyl, Fluorenyl, Benzofluorenyl, Spirobifluorenyl, Indenofluorenyl, Dibenzofuranyl, Dibenzothiophenyl, Carbazolyl, Benzofuranyl, Benzothiophenyl, Indolyl, Chinolinyl, Pyridyl, Pyrimidyl, Pyrazinyl, Pyridazinyl und Triazinyl, wobei die Gruppen jeweils durch einen oder mehrere Reste R³ substituiert sein können, oder aus Kombinationen von 2 oder 3 Resten, die sich von diesen Gruppen ableiten, wobei die Gruppen jeweils durch einen oder mehrere Reste R³ substituiert sein können.

4. Verbindung nach einem oder mehreren der Ansprüche 1 bis 3, **dadurch gekennzeichnet, dass** die Gruppen R¹ einer der folgenden Formeln
| | | |
|---|---|---|
| -CH₃ | -C(CH₃)₃ | -CH₂CH₃ |
| R¹-1 | R¹-2 | R¹-3 |
| -CH₂CH(CH₃)₂ | -CF₃ | -CF₂CF₃ |
| R¹-4 | R¹-5 | R¹-6 |
| -OCF₃ | -SCF₃ | -SF₅ |
| R¹-7 | R¹-8 | R¹-9 |
| -OCF₂CF₃ | -SCF₂CF₃ | 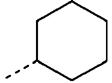 |
| R¹-10 | R¹-11 | R¹-12 |
| 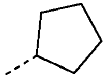 | 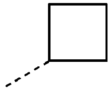 | 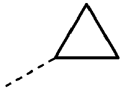 |
| R¹-13 | R¹-14 | R¹-15 |
| -OCH₃ | -SCH₃ | |
| R¹-22 | R¹-23 | |
entsprechen.

5. Verbindung nach einem oder mehreren der Ansprüche 1 bis 4, **dadurch gekennzeichnet, dass** sie einer der Formeln (I-A-1) bis (I-A-5) und (I-B-1) bis (I-B-5) 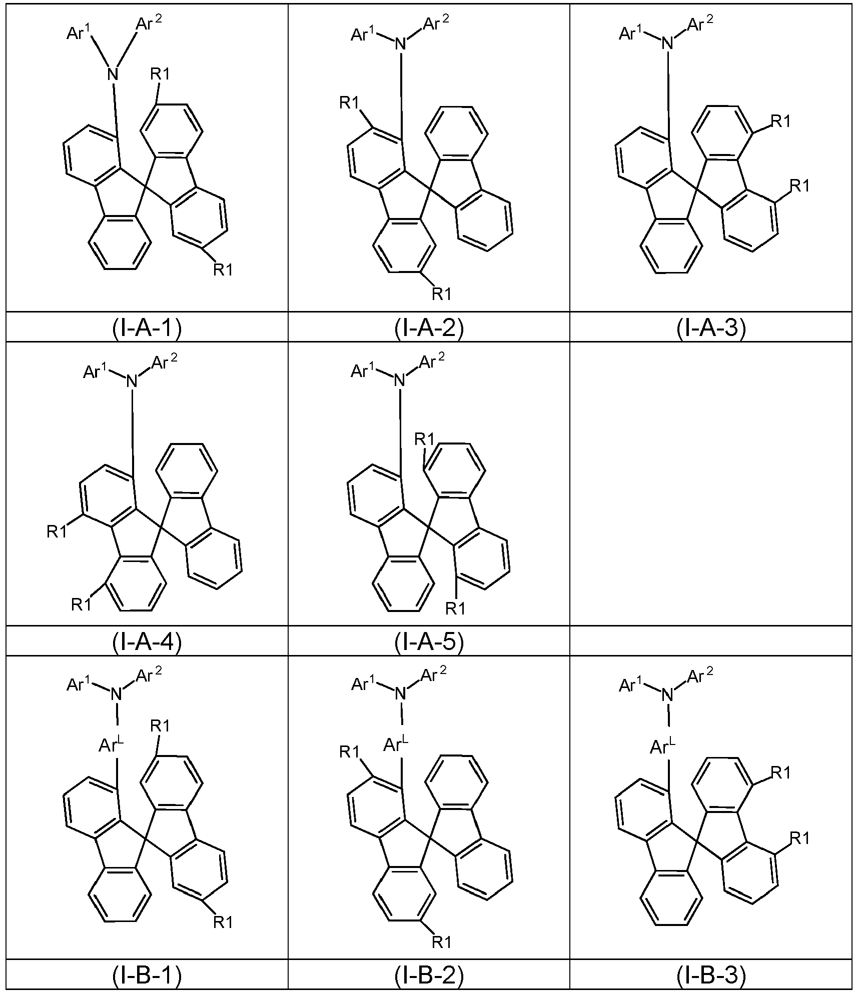 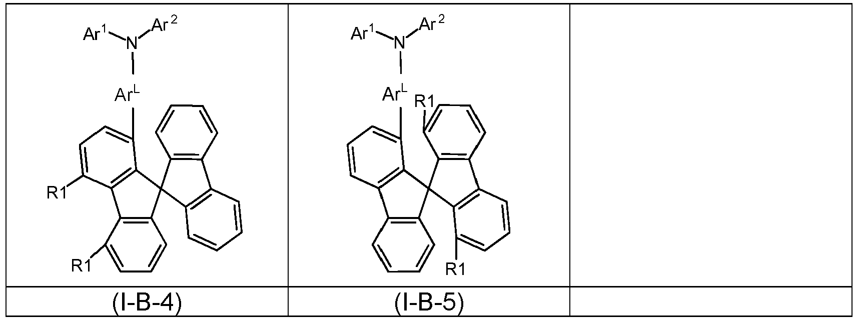 entspricht, wobei die Variablen wie in einem oder mehreren der Ansprüche 1 bis 4 definiert sind.

6. Verfahren zur Herstellung einer Verbindung nach einem oder mehreren der Ansprüche 1 bis 5, **dadurch gekennzeichnet, dass** es die folgenden Reaktionsschritte umfasst
1) Metallierung eines Biphenylderivats, das eine reaktive Gruppe in einer Position aufweist, die ortho zur Phenyl-Phenyl-Bindung ist;
2) Addition des metallierten Biphenylderivats an ein Fluorenonderivat, das in seiner 1-Position eine Gruppe A aufweist; wobei die Gruppe A ausgewählt ist aus i) X, oder ii) -Ar-X, oder iii) -NAr₂, oder iv) -Ar-NAr₂, wobei Ar eine aromatische oder heteroaromatische Gruppe ist und wobei X eine reaktive Gruppe ist; und
3) Cyclisierung des entstandenen Additionsprodukts zu einem Spirobifluorenderivat unter sauren Bedingungen oder mit einer Lewissäure.

7. Oligomer, Polymer oder Dendrimer enthaltend eine oder mehrere Verbindungen der Formel (IA) oder (IB) nach einem oder mehreren der Ansprüche 1 bis 5, wobei die Bindung(en) zu dem Polymer, Oligomer oder Dendrimer an beliebigen Positionen in Formel (IA) oder (IB) lokalisiert sein kann/können, die durch R¹ oder R³ substituiert sind.

8. Formulierung enthaltend mindestens eine Verbindung der Formel (IA) oder (IB) nach einem oder mehreren der Ansprüche 1 bis 5 oder mindestens ein Polymer, Oligomer oder Dendrimer nach Anspruch 7 und mindestens ein Lösungsmittel.

9. Elektronische Vorrichtung enthaltend mindestens eine Verbindung nach einem oder mehreren der Ansprüche 1 bis 5 oder mindestens ein Polymer, Oligomer oder Dendrimer nach Anspruch 7.

10. Elektronische Vorrichtung nach Anspruch 9, **dadurch gekennzeichnet, dass** es sich um eine organische Elektrolumineszenzvorrichtung handelt, die Anode, Kathode und mindestens eine Emitterschicht umfasst, wobei mindestens eine organische Schicht der Vorrichtung, bei der es sich um eine Emitterschicht, eine Lochtransportschicht, eine Elektronenblockierschicht oder eine Lochinjektionsschicht handelt, die mindestens eine Verbindung enthält.

11. Verwendung einer Verbindung nach einem oder mehreren der Ansprüche 1 bis 5 oder eines Polymers, Oligomers oder Dendrimers nach Anspruch 7 in einer elektronischen Vorrichtung.

## Revendications

1. Composé de formule (IA) ou (IB) 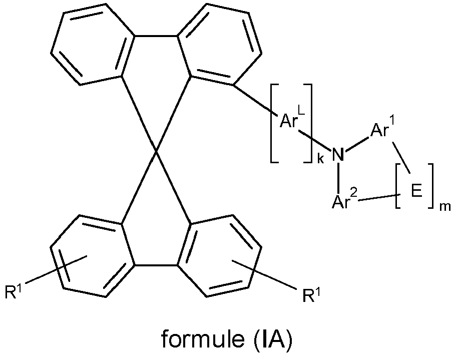 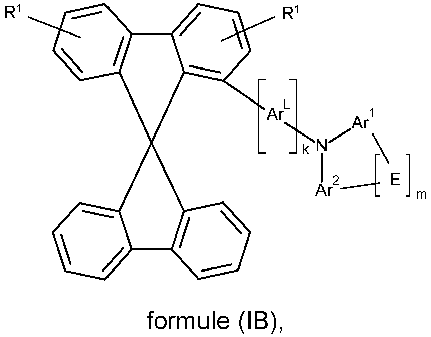 dans laquelle ce qui suit s'applique aux variables :
Ar^{L} est choisi parmi des noyaux aromatiques ayant de 6 à 30 atomes de cycle aromatique, qui peuvent être substitués par un ou plusieurs radicaux R³, et des noyaux hétéroaromatiques ayant de 5 à 30 atomes de cycle aromatique, qui peuvent être substitués par un ou plusieurs radicaux R³ ;
Ar¹ et Ar² sont choisis, de manière identique ou différente, parmi des noyaux aromatiques ayant de 6 à 30 atomes de cycle aromatique, qui peuvent être substitués par un ou plusieurs radicaux R³, et des noyaux hétéroaromatiques ayant de 5 à 30 atomes de cycle aromatique, qui peuvent être substitués par un ou plusieurs radicaux R³ ;
E est une liaison simple ou est un groupement divalent choisi parmi C(R³)₂, N(R³), O, et S ;
R¹ est, de manière identique ou différente à chaque occurrence, choisi parmi des groupements alkyle, alcoxy ou thioalkyle à chaîne linéaire ayant de 1 à 20 atomes de C, qui peuvent éventuellement être substitués par un ou plusieurs groupements F, et parmi des groupements alkyle, alcoxy ou thioalkyle ramifiés ou cycliques ayant de 3 à 20 atomes de C, qui peuvent éventuellement être substitués par un ou plusieurs groupements F ;
R³ est, de manière identique ou différente à chaque occurrence, choisi parmi H, D, F, C(=O)R⁴, CN, Si(R⁴)₃, N(R⁴)₂, P(=O)(R⁴)₂, OR⁴, S(=O)R⁴, S(=O)₂R⁴, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R³ peuvent être reliés les uns aux autres pour former un cycle ; où lesdits groupements alkyle, alcoxy, alcényle et alcynyle et lesdits noyaux aromatiques et hétéroaromatiques peuvent dans chaque cas être substitués par un ou plusieurs radicaux R⁴, et où un ou plusieurs groupements CH₂ dans lesdits groupements alkyle, alcoxy, alcényle et alcynyle peuvent dans chaque cas être remplacés par -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO ou SO₂ ;
R⁴ est, de manière identique ou différente à chaque occurrence, choisi parmi H, D, F, C(=O)R⁵, CN, Si(R⁵)₃, N(R⁵)₂, P(=O)(R⁵)₂, OR⁵, S(=O)R⁵, S(=O)₂R⁵, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R⁴ peuvent être reliés les uns aux autres pour former un cycle ; où lesdits groupements alkyle, alcoxy, alcényle et alcynyle et lesdits noyaux aromatiques et hétéroaromatiques peuvent dans chaque cas être substitués par un ou plusieurs radicaux R⁵, et où un ou plusieurs groupements CH₂ dans lesdits groupements alkyle, alcoxy, alcényle et alcynyle peuvent dans chaque cas être remplacés par -R⁵C=CR⁵-, -C=C-, Si(R⁵)₂, C=O, C=NR⁵, -C(=O)O-, -C(=O)NR⁵-, NR⁵, P(=O)(R⁵), -O-, -S-, SO ou SO₂ ;
R⁵ est choisi, de manière identique ou différente à chaque occurrence, parmi H, D, F, CN, des groupements alkyle ayant de 1 à 20 atomes de C, des noyaux aromatiques ayant de 6 à 40 atomes de C, ou des noyaux hétéroaromatiques ayant de 5 à 40 atomes de cycle aromatique ; où deux, ou plus, radicaux R⁵ peuvent être reliés les uns aux autres pour former un cycle ; et où lesdits groupements alkyle, noyaux aromatiques et noyaux hétéroaromatiques peuvent être substitués par F et CN ;
k vaut 0 ou 1 ; où, dans le cas où k=0, le groupement Ar^{L} n'est pas présent et l'atome d'azote et le groupement spirobifluorène sont directement reliés ;
m vaut 0 ou 1, où, dans le cas où m=0, le groupement E n'est pas présent et les groupements Ar¹ et Ar² ne sont pas reliés.

2. Composé selon la revendication 1, **caractérisé en ce que** l'indice k vaut 0, de sorte que le groupement Ar^{L} n'est pas présent, et le spirobifluorène et l'atome d'azote de l'amine sont directement reliés l'un à l'autre.

3. Composé selon la revendication 1 ou 2, **caractérisé en ce que** les groupements Ar¹ et Ar² sont choisis, de manière identique ou différente, parmi des radicaux dérivés d'un groupement choisi parmi phényle, biphényle, terphényle, quaterphényle, naphtyle, fluorényle, benzofluorényle, spirobi-fluorényle, indénofluorényle, dibenzofuranyle, dibenzothiophényle, carb-azolyle, benzofuranyle, benzothiophényle, indolyle, quinoléinyle, pyridyle, pyrimidyle, pyrazinyle, pyridazinyle et triazinyle, où les groupements peuvent chacun être substitués par un ou plusieurs radicaux R³, ou parmi des combinaisons de 2 ou 3 radicaux dérivés de ces groupements, où les groupements peuvent chacun être substitués par un ou plusieurs radicaux R³.

4. Composé selon l'une ou plusieurs parmi les revendications 1 à 3, **caractérisé en ce que** les groupements R¹ répondent à l'une des formules suivantes
| | | |
|---|---|---|
| -CH₃ | -C(CH₃)₃ | -CH₂CH₃ |
| R¹-1 | R¹-2 | R¹-3 |
| -CH₂CH(CH₃)₂ | -CF₃ | -CF₂CF₃ |
| R¹-4 | R¹-5 | R¹-6 |
| -OCF₃ | -SCF₃ | -SF₅ |
| R¹-7 | R¹-8 | R¹-9 |
| -OCF₂CF₃ | -SCF₂CF₃ | 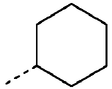 |
| R¹-10 | R¹-11 | R¹-12 |
| 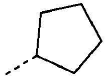 | 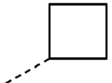 | 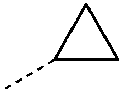 |
| R¹-13 | R¹-14 | R¹-15 |
| -OCH₃ | -SCH₃ | |
| R¹-22 | R¹-23 | |

5. Composé selon l'une ou plusieurs parmi les revendications 1 à 4, **caractérisé en ce qu'**il répond à l'une des formules (I-A-1) à (I-A-5) et (I-B-1) à (I-B-5) 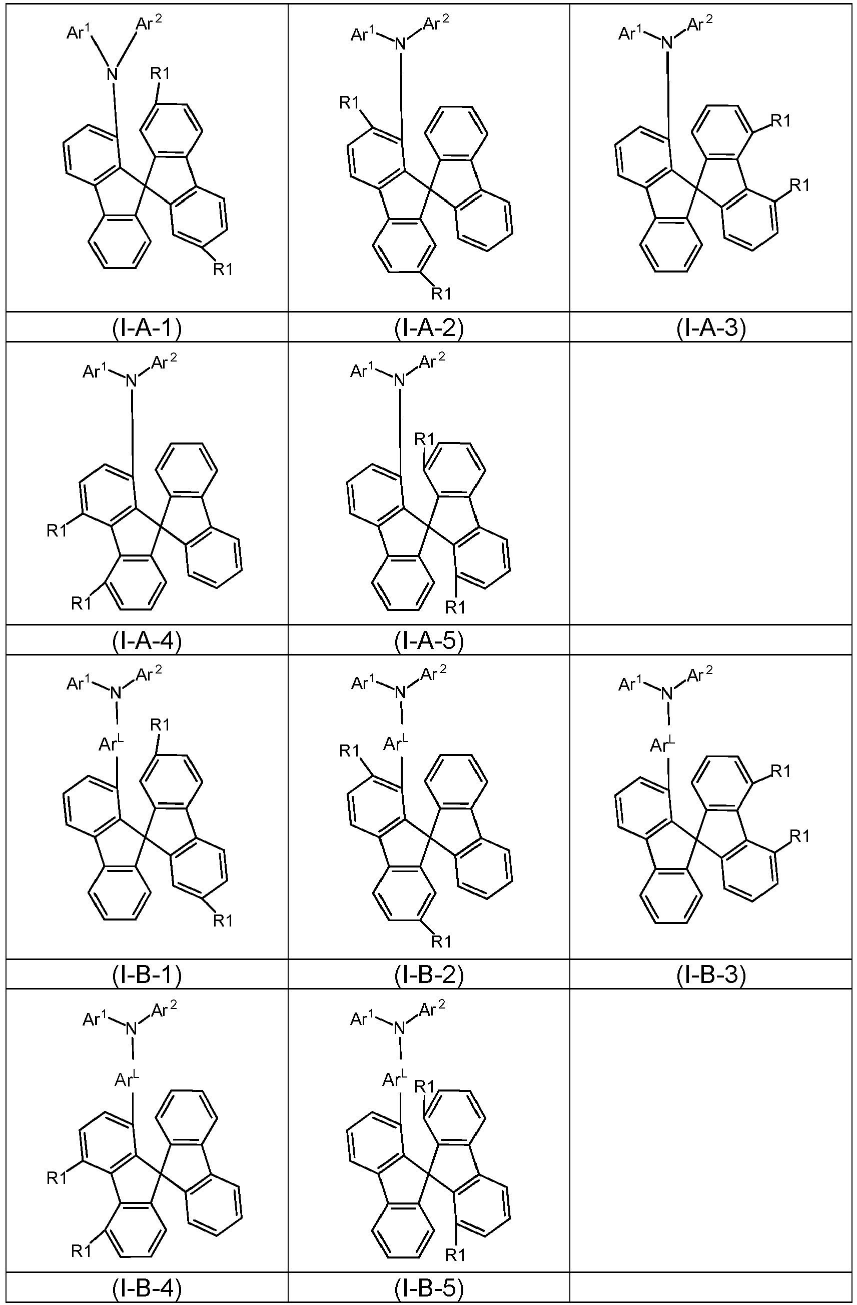 dans lesquelles les variables sont définies selon l'un ou plusieurs parmi les revendications 1 à 4.

6. Procédé de préparation d'un composé selon l'une ou plusieurs parmi les revendications 1 à 5, **caractérisé en ce qu'**il comprend les étapes de réaction
1) de métallation d'un dérivé de biphényle qui possède un groupement réactif dans une position en ortho par rapport à la liaison phényl-phényle ;
2) d'addition du dérivé de biphényle métallaté à un dérivé de fluorénone qui possède un groupement A au niveau de sa position 1 ; où le groupement A est choisi parmi i) X, ou ii) -Ar-X, ou iii) -NAr₂, ou iv) -Ar-NAr₂, où Ar est un groupement aromatique ou hétéroaromatique, et où X est un groupement réactif ; et
3) de cyclisation du produit d'addition résultant en dérivé de spirobifluorène dans des conditions acides ou avec un acide de Lewis.

7. Oligomère, polymère ou dendrimère comprenant un ou plusieurs composés de formule (IA) ou (IB) selon l'une ou plusieurs parmi les revendications 1 à 5, la ou les liaisons au polymère, à l'oligomère ou au dendrimère pouvant être situées au niveau de positions désirées quelconques dans la formule (IA) ou (IB) substituées par R¹ ou R³.

8. Formulation comprenant au moins un composé de formule (IA) ou (IB) selon l'une ou plusieurs parmi les revendications 1 à 5 ou au moins un polymère, oligomère ou dendrimère selon la revendication 7, et au moins un solvant.

9. Dispositif électronique comprenant au moins un composé selon l'une ou plusieurs parmi les revendications 1 à 5, ou au moins un polymère, oligomère ou dendrimère selon la revendication 7.

10. Dispositif électronique selon la revendication 9, **caractérisé en ce qu'**il s'agit d'un dispositif électroluminescent organique, comprenant une anode, une cathode et au moins une couche émettrice, où au moins une couche organique du dispositif, qui est une couche émettrice, une couche de transport de trous, une couche de blocage d'électrons ou une couche d'injection de trous, comprend le au moins un composé.

11. Utilisation d'un composé selon l'une ou plusieurs parmi les revendications 1 à 5, ou d'un polymère, oligomère ou dendrimère selon la revendication 7, dans un dispositif électronique.
